## Europäisches Patentamt
## European Patent Office
## Office européen des brevets

(19)

(11) Veröffentlichungsnummer: **0 261 403**
**A2**

(12) **EUROPÄISCHE PATENTANMELDUNG**

(21) Anmeldenummer: **87112104.2**

(22) Anmeldetag: **20.08.87**

(51) Int. Cl.4: **C12N 15/00** , **C12P 21/00** , **A61K 39/125**

(30) Priorität: **23.08.86 DE 3628658**
**17.01.87 DE 3701301**

(43) Veröffentlichungstag der Anmeldung:
**30.03.88 Patentblatt 88/13**

(84) Benannte Vertragsstaaten:
**AT BE CH DE ES FR GB GR IT LI LU NL SE**

(71) Anmelder: **Boehringer Ingelheim International G.m.b.H**

**D-6507 Ingelheim am Rhein(DE)**

(72) Erfinder: **Duechler, Markus**
**Runbensgasse 1/8**
**A-1040 Wien(AT)**
Erfinder: **Skern, Tim, Dr.**
**Rue de St. Diê**
**F-67100 Strasbourg-Neudorf(FR)**
Erfinder: **Sommergruber, Wolfgang, Dr.**
**Stösselgasse 6/5**
**A-1130 Wien(AT)**
Erfinder: **Neubauer, Christoph**
**Himmelstrasse 81**
**A-1190 Wien(AT)**
Erfinder: **Gründler, Peter, Dr.**
**Erlaaerstrasse 41**
**A-1232 Wien(AT)**
Erfinder: **Blaas, Dieter, Dr.**
**Liechtensteinstrasse 119**
**A-1090 Wien(AT)**
Erfinder: **Küchler, Ernst, Prof.**
**Görgengasse 23/6/26**
**A-1190 Wien(AT)**
Erfinder: **Frasel, Ljerka**
**Obere Donaustrasse 5**
**A-1020 Wien(AT)**
Erfinder: **Zorn, Manfred**
**Mauerbachstrasse 46 A-5**
**A-1140 Wien(AT)**

(54) **Polypeptide des Rhinovirusstammes HRV89 sowie die hierfür codierenden DNA-Moleküle.**

(57) Die vorliegende Erfindung betrifft Peptide, die ganz oder teilweise denen der viralen Proteine des humanen Rhinovirus Stamm 89 (HRV89) entsprechen, die für diese Peptide codierenden Deoxyribonukleinsäuremoleküle, sowie die Herstellung und Verwendung dieser Substanzen.

## Polypeptide des Rhinovirusstammes HRV89 sowie die hierfür codierenden DNA-Moleküle

Die vorliegende Erfindung betrifft Peptide, die ganz oder teilweise denen der viralen Proteine des humanen Rhinovirus Stamm 89 (HRV89) entsprechen, die für diese Peptide codierenden Deoxyribonukleinsäuremoleküle, sowie die Herstellung und Verwendung dieser Substanzen.

Rhinoviren sind RNA-Viren und stellen nach der herkömmlichen Einteiling der Viren eine Gattung innerhalb der Familie der Picornaviren dar (Cooper, P.D., Agol, H.L., Bachrach, H.L., Brown, F., Ghendon, Y., Gibbs, A.J., Gillespie, J.H., Lonberg-Holm, K. Mandel, B. Melnick, J.L., Mohanty, S.B. Povey, R. C., Rueckert, R.R., Schaffer, F.L. und Tyrrell, D.A.J., 1978, Intervirology, 10, 165 - 180; M.R. MacNaughton, 1982, Current Top. Microbiol. Immunol. 97, 1 - 26).

Sie sind weit verbreitet, befallen den oberen respiratorischen Trakt des Menschen und verursachen akute Infektionen, die zu Schnupfen, Husten, Heiserkeit etc. führen und allgemein als Erkältungen bezeichnet werden (Stott, E.J. und Killington, R.A., 1972, Ann. Rev. Microbiol. 26, 503 - 524). Infektionen durch Rhinoviren zählen zu den häufigsten Erkrankungen des Menschen. Obwohl der Verlauf dieser Erkrankungen meist harmloser Natur ist, führen Erkältungen doch zu einer vorübergehenden Schwächung des Organismus. Dadurch kann es zu Sekundärinfektionen durch andere Viren oder Bakterien kommen, die dann unter Umständen schwere Erkrankungen zur Folge haben. Außerdem ist der durch Rhinoviren verursachte volkswirtschaftliche Schaden beträchtlich. Es ist berechnet worden, daß in den U.S.A. durch Rhinovirusinfektionen jährlich mehr als 200 Millionen Arbeitstage bzw. Schultage verloren gehen (Davis, B.D., Dulbecco, R., Eisen, H.N. und Ginsberg H.S., 1980, Microbiology, Third Edition, Harper & Row, Publ., New York, S. 1114).

In den letzten Jahren ist zusätzlich eine beträchtliche Zunahme an Rhinovirusinfektionen in Ballungszentren festgestellt worden. Während es bei den meisten anderen Infektionskrankheiten zu einer langdauernden oder bleibenden Immunität gegen den betreffenden Krankheitserreger kommt, können Infektionen durch Rhinoviren immer wieder auftreten. Der Grund für das Fehlen einer bleibenden Immunität ist die große Vielfalt an Rhinovirusstämmen. Bis jetzt wurden über 100 Rhinovirusstämme isoliert, die untereinander keine oder nur wenig immunologische Kreuzreaktion zeigen (Fox, J.P. 1976, American J. Epidemiol. 103, 345 - 354; Melnick, J.L., 1980, Proc. Med. Virol. 26, 214 - 232). Nach erfolgter Infektion lassen sich zwar Antikörper gegen den betreffenden Virusstamm nachweisen, doch gewähren diese keinen Schutz gegen andere Rhinovirusstämme. Auf Grund der großen Anzahl von Stämmen, die in der Bevölkerung zirkulieren, sind wiederholte Infektionen durch Rhinoviren möglich.

Aufgabe der vorliegenden Erfindung war es daher, Mittel bereitzustellen, die einen Schutz gegen Infektionen durch Rhinoviren ermöglichen.

Unter Verwendung von Hyperimmunseren ist es möglich gewesen, 50 von insgesamt 90 Rhinovirus Serotypen in 16 Gruppen einzuteilen (Conney, M.K., Fox, J.P. und Kenny, G.E., 1982, Infect. Immun. 37, 642 - 647). Eine andere Form der Einteilung ergibt sich aufgrund der Bindung an zelluläre Rezeptoren. Trotz der ausgeprägten Heterogenität in den immunologischen Eigenschaften verhalten sich nämlich die Rhinoviren in Bezug auf die Bindung an Zelloberflächen-Rezeptoren untereinander ähnlich.

Mit Hilfe von Kompetitionsexperimenten konnte festgestellt werden, daß es für 88 untersuchte Rhinovirusstämme auf HeLa-Zellen (Klon R-19) nur zwei verschiedene Rezeptoren gibt ((Abraham, G. und Colonno, R.J., 1984, J. Virol. 51, 340 - 344; Colonno, R.J., Callahan, P.L. und Long, W.J., 1986, J. Virol. 57, 7 - 12). Einschränkend muß dazu allerdings gesagt werden, daß diese Ergebnisse mit HeLa-Zellen erhalten worden sind und nicht notwendigerweise auch für die Rezeptoren an den natürlichen Wirtszellen im oberen respiratorischen Trakt des Menschen Gültigkeit haben.

Eine weitere Aufgabe der Erfindung bestand darin, Oligopeptide bereitzustellen, die ganz oder teilweise den viralen Proteinen entsprechen, die entweder zur Anregung einer gegen intakte Viren gerichteten Immunanwort verwendet oder zur Bindung und Blockierung von zellulären Rezeptoren eingesetzt werden können.

Als typische Picornaviren enthalten Rhinoviren eine einzelsträngige RNA, die von einem Kapsid umgeben ist, das aus 4 Polypeptiden besteht, die als VP1 (P1D), VP2 (P1B), VP3 (P1C) und VP4 (P1A) bezeichnet werden (Medappa, K.C., McLean, C. und Rueckert, R.R., 1971, Virology 44, 259 - 270; die Ausdrücke in Klammer sind die neuen Bezeichnungen entsprechend dem Nomenklaturvorschlag von Rueckert, R.R. und Wimmer, E., 1984, J. Virol. 50, 957 - 959). Ein einzelnes Viruspartikel enthält 60 Kopien von jedem dieser Polypeptide. Die relativen Molekülmassen betragen bei verschiedenen Rhinoviren für VP1 34 - 36000, für VP2 27 - 30000, für VP3 24 - 28000 und für VP4 7 - 8000 (MacNaughton, M.R., 1982, loc.cit). Darüberhinaus ist für Rhinoviren charakteristisch, daß sie bei pH-Werten unter 5 rasch inaktiviert werden und empfindlich gegen Salzlösungen hoher Konzentration sind. Weiter zeigen die meisten Rhinoviren ein optimales Wachstum bei 33 - 34°C (Luria, S.E., Darnell, Jr., J.E., Baltimore, D. und Campbell, A., 1978, General Virology, Third Edition, John Wiley & Sons, New York, 308 ff.).

Die einzelsträngige RNA mit einer Länge von ca. 7100 Nukleotiden stellt das Genom des Virus dar und kann gleichzeitig auch als Matrize für die Synthese der viralen Proteine dienen. Dabei wird ein Großteil der Nukleotidsequenz zunächst in ein langes Polyprotein übersetzt, aus dem durch proteolytische Spaltung die fertigen viralen Proteine entstehen (Butterworth, B.E., 1973, Virology 56, 439 - 453; Mc Lean, C. und Rueckert, R.R., 1973, J. Virol. 11, 341 - 344, McLean, C., Matthews, T.J. und Rueckert, R.R., 1976, J. Virol. 19, 903 - 914). Als Produkte dieser Prozessierung werden neben den viralen Hüllenproteinen VP1, VP2, VP3 und VP4 noch eine Reihe von Proteinen gebildet, wie P2A, P2C, P3B, P3C und P3D. P3B (meist als VPg bezeichnet) ist kovalent an das 5'-terminale Nukleotid der viralen RNA gebunden. In Analogie zu Poliovirus kann angenommen werden, daß P2A und P3C Proteasen sind, die teilweise für die Prozessierung des viralen Polyproteins verantwortlich sind (MacNaughton, M.R., 1982, loc. cit.; Toyoda, H., Nicklin, M.J.H., Murray, M.G., Anderson, C.W., Dunn, J.J., Studier, F.W. und Wimmer, E. 1986, Cell 45, 761-770).

Dementsprechend ist P3D die Polymerase für die Replikation der viralen RNA. Über die Funktion des Proteins P2C ist bisher nur wenig bekannt.

Beim Prozessieren des viralen Polyproteins werden Teile der Aminosäuresequenz abgespalten und anschließend abgebaut (McLean, C., Matthew, T.J. und Rueckert, R.R., 1976, loc.cit.) Zur Zeit kann keine Aussage darüber gemacht werden, ob diesen Peptiden nicht eine bis jetzt noch unentdeckte Funktion zukommt.

Kenntnisse über Rhinoviren haben in der letzten Zeit dadurch eine starke Ausweitung erfahren, daß es gelungen ist, die Nukleotidsequenzen von zwei humanen Rhinovirus Serotypen zu bestimmen, und zwar HRV2 (deutsche Patentanmeldung P 35 05 148.5; Skern., T., Sommergruber, W. Blaas, D. Gruendler, P. Fraundorfer, F., Pieler, C., Fogy, I. und Kuechler, E., 1985, Nucleic Acids Res. 13, 2111 - 2126) und HRV14 (Stanway, G., Hughes, P.J., Mountford, R.C., Minor, P.D. und Almond, J.W., 1984, Nucleic Acids Res. 12, 7859 - 7875; Callahan, P.L., Mizutani, S. und Colonno, R.J., 1985, Proc. Natl. Acad. Sci. USA 82, 732 - 736; europäische Patentanmeldung No. 85 401 465.1). Weiter wurde die Struktur von HRV14 mit Hilfe der Röntgenstrukturanalyse mit einer Auflösung von 3 Angström aufgeklärt (Rossmann, M.G., Arnold, E., Erickson, J.W., Frankenberger, E.A., Griffith, J.P., Hecht, H.J. Johnson, J.E., Kramer, G., Luo, M., Mosser, A.G., Rueckert, R.R., Sherry, B. und Vriend, G., 1985, Nature (London) 317, 145 - 153) und die Position von 4 Epitopen in HRV14, die für die Neutralisation verantwortlich sind, mit Hilfe von HRV14 Mutanten, die gegen monoklonale Antikörper resistent sind, identifiziert (Sherry, B. Mosser, A.G., Colonno, R.J. and Rueckert, R.R., 1985, J. Virol. 57, 246 - 257). All diese Befunde weisen auf eine große Ähnlichkeit zu Poliovirus hin.

Dies betrifft sowohl die Sequenzen der Nukleinsäuren, bzw. der Proteine als auch die Virusstruktur, wie aus dem Vergleich mit der Röntgenstrukturanalyse von Poliovirus (Hogle, J.M., Chow, M. und Filman, D.J., 1985, Science 229, 1358 - 1365) ersichtlich ist. Dies läßt auf eine nahe Beziehung der Rhinoviren zu den Enteroviren schließen. Trotzdem hätte man erwartet, daß die beiden humanen Rhinoviren zueinander mehr Ähnlichkeit aufweisen als zu Poliovirus. Tatsache ist aber, daß die Homologie zwischen HRV2 und HRV14 in einigen Genen nicht größer ist, als die zu Poliovirus. Eine mögliche Erklärung für den vergleichsweise niedrigen Grad der Verwandtschaft zwischen den beiden Rhinoviren wäre die Tatsache, daß HRV2 und HRV14 sich an verschiedene zelluläre Rezeptoren binden und somit Prototypen für die jeweilige Gruppe darstellen. Demnach sollten andere Rhinoviren, die sich an den selben Rezeptor binden wie HRV14 mit diesem auch in der Sequenz stark homolog sein. Aus diesem Grund wurde für die Sequenzierungsarbeiten der humane Rhinovirusstamm 89 (HRV89) gewählt, der zur selben Rezeptorgruppe gehört wie HRV14 (Abrahem, G. und Colonno, R.J., 1984, loc. cit.).

Zur Gewinnung des viralen Ausgangsmaterials wurden HeLa-Zellen in einem geeigneten Infektionsmedium mit HRV89 infiziert und mehrere Stunden unter optimalen Wachstumsbedingungen inkubiert.

Virus konnte sowohl aus den Zellen als auch aus dem Medium gewonnen werden.

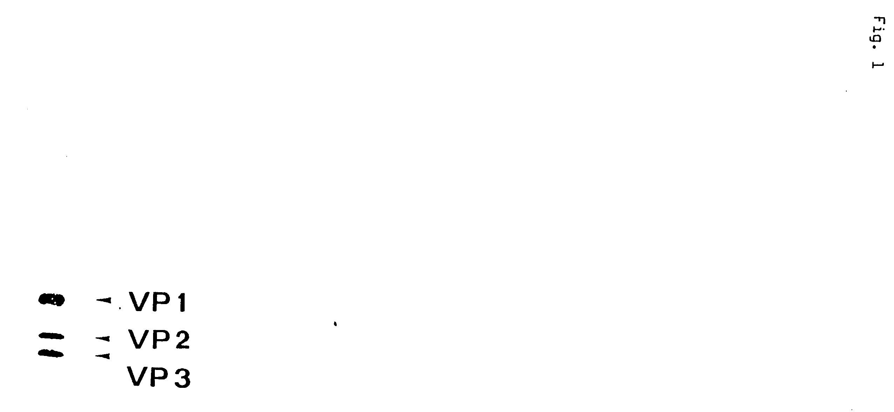

Verschiedene Reinigungsschritte erbrachten eine Viruspräparation, deren Proteinmuster sich typischerweise wie in Fig. 1 gezeigt, darstellt.

Die virale RNA wurde aus der Viruspräparation beispielsweise durch Phenolextraktion gewonnen und gereinigt; sie wurde anschließend mit Hilfe reverser Transkriptase und Oligo dT als Primer in cDNA transkribiert. Die erhaltenen RNA/cDNA-Hybride wurden homopolymer verlängert und in ein geeignetes Plasmid, beispielsweise das pBR322 eingebaut.

Die Verwendung von Methoden zur reversen Transkription von RNA, zur Integration von RNA-cDNA Hybriden in Plasmide und zur Transformation von Bakterien sind in der Literatur ausreichend beschrieben worden (Kitamura, N. und Wimmer, E., 1980, Proc. Natl. Acad. Sci. USA 77, 3196 - 3200; Nelson, T. und Brutlag, D., 1979, Methods in Enzymology 68, 41 - 50, Zain, S., Sambrook, J., Roberts, J.J., Keller, W., Fried, M. und Dunn, A., 1979, Cell 16, 851 - 861; Roychoudhury, R. und Wu, R., 1980, Methods in Enzymology 65, 42 - 62; Kitamura, N., Semler, B.L., Rothberg P.G., Larsen, G.R., Adler, C.J., Dorner, A.J., Emini, E.A., Hanecak, R., Lee, J.L., van der Werf, S., Anderson, C.W. und Wimmer, E. 1981, Nature (London) 291, 547 - 553;

van der Werf, S., Bregegere, F., Kopecka, H., Kitamura, N. Rothberg, P.G., Kourilsky, P., Wimmer, E. und Girard, M., 1981, Proc. Natl. Acad. Sci. USA 78, 5983 - 5987; Namoto, A., Omata, T., Toyoda, H., Kuge, S., Hosie, H., Kataoka, Y., Genba, A. Nakano, Y. und Imura, N. 1982, Proc. Natl. Acad. Sci USA 79, 5793 - 5797; Stanway, G., Cann, A.J., Hauptmann, R., Hughes, P., Clarke, L.D., Mountford, R.C., Minor, P.D., Schild, G.C. und Almond, J.W., 1983 Nucleic Acids Res. 11, 5629 - 5643; Skern, T., Sommergruber, W., Blaas, D., Gruendler, P., Fraundorfer, F., Pieler, C., Fogy, I. und Kuechler, E., 1985, loc cit.)

Nach erfolgter Transformation eines geeigneten Wirtsorganismus, beispielsweise E. coli HB 101 wurde die Plasmid-DNA nach der "Mini-Plasmid-Präparationstechnik" isoliert und die Größe der rekombinanten DNA ermittelt. Hierzu wurden die rekombinanten Plasmide mit Hilfe der Restriktionsendonuclease PstI geschnitten, die Bruchstücke elektrophoretisch aufgetrennt und mit bekannter Lambda-HindIII Marker DNA verglichen.

Zur Subklonierung der DNA wurden die durch PstI Verdau der rekombinanten pBR322 Klone erhaltenen und gereinigten DNA-Fragmente in einen geeigneten Vektor, beispielsweise in das Plasmid pUC9 eingebaut und die rekombinanten Vektoren in einen geeigneten Wirt, beispielsweise E. coli JM101 transformiert. Die Subklone, die mit rekombinanten Vektoren erfolgreich transformiert worden waren, wurden hochgezüchtet und deren Plasmid-DNA isoliert und gereinigt.

Aus drei Subklonen wurden drei verschieden lange Primer-Fragmente [54 Nukleotide (Fragment A), 122 Nukleotide (Fragment B) und 139 Nukleotide (Fragment C)] isoliert und gereinigt. Zusätzlich wurde noch ein 20 Nukleotide langer synthetischer primer (D) verwendet. Mit Hilfe dieser komplementären Einzelstrang DNA wurde ein mit $^{32}$P markiertes reverses Transkript von HRV89-RNA hergestellt.

Zum Nachweis von HRV89-Sequenzen in rekombinanter DNA wurde die Plasmid DNA mit PstI verdaut, elektrophoretisch analysiert und die DNA Fragmente vom Gel auf Nitrozellulosefilter transferiert und fixiert. Diese DNA-tragenden Filter wurden mit der radioaktiv markierten HRV89-cDNA hybridisiert und die Filter anschließend exponiert. Von den so ermittelten, der HRV89-RNA entsprechenden, rekombinanten DNA-Fragmenten wurden die Restriktionsstellen kartiert und die DNA sequenziert. Man erhielt Klone, die das Genom von HRV89 repräsentieren.

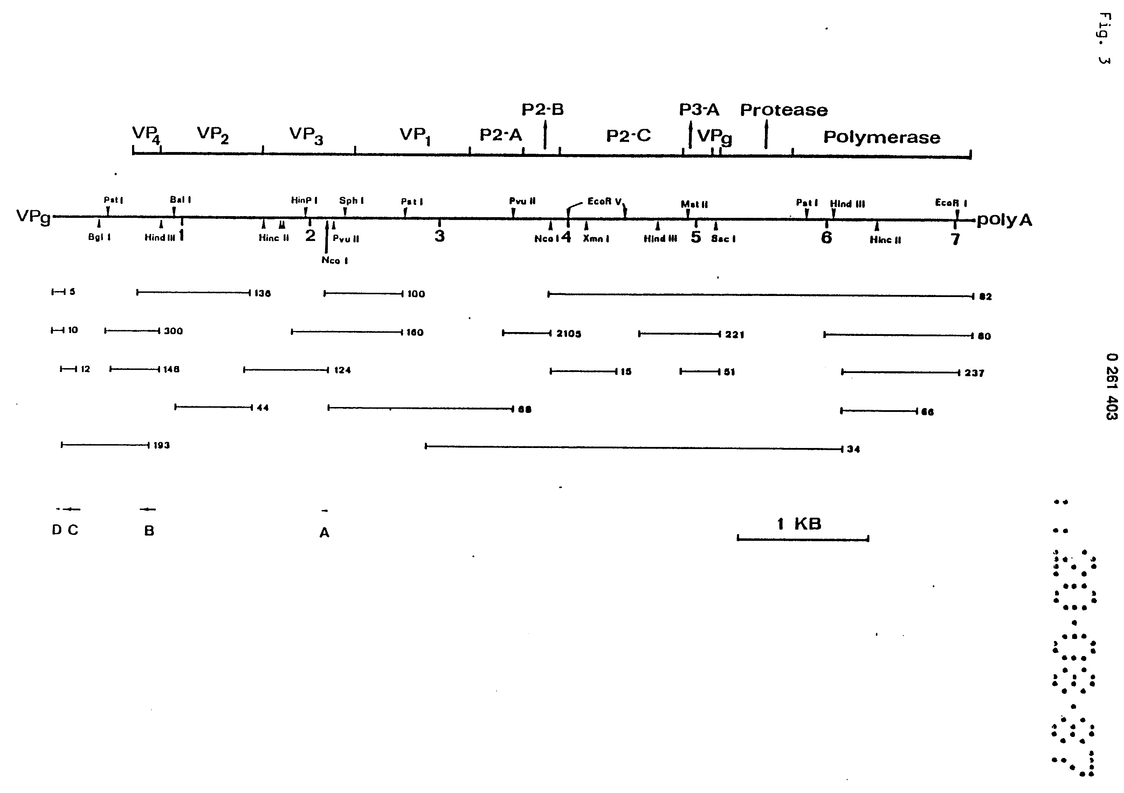

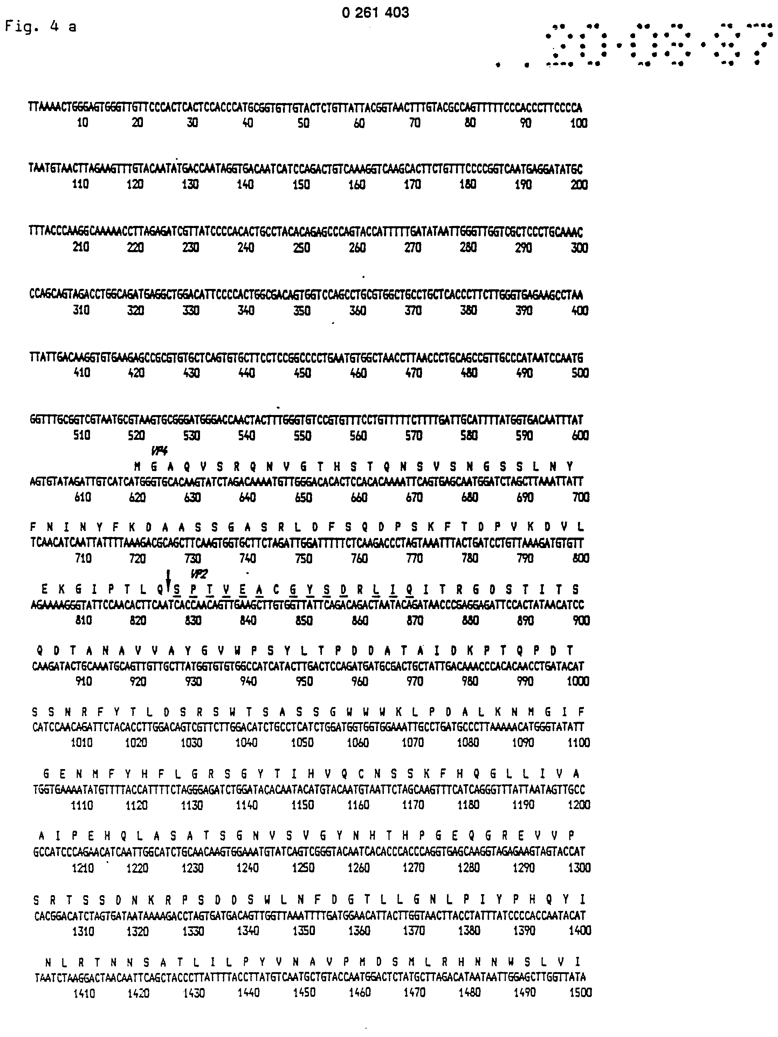

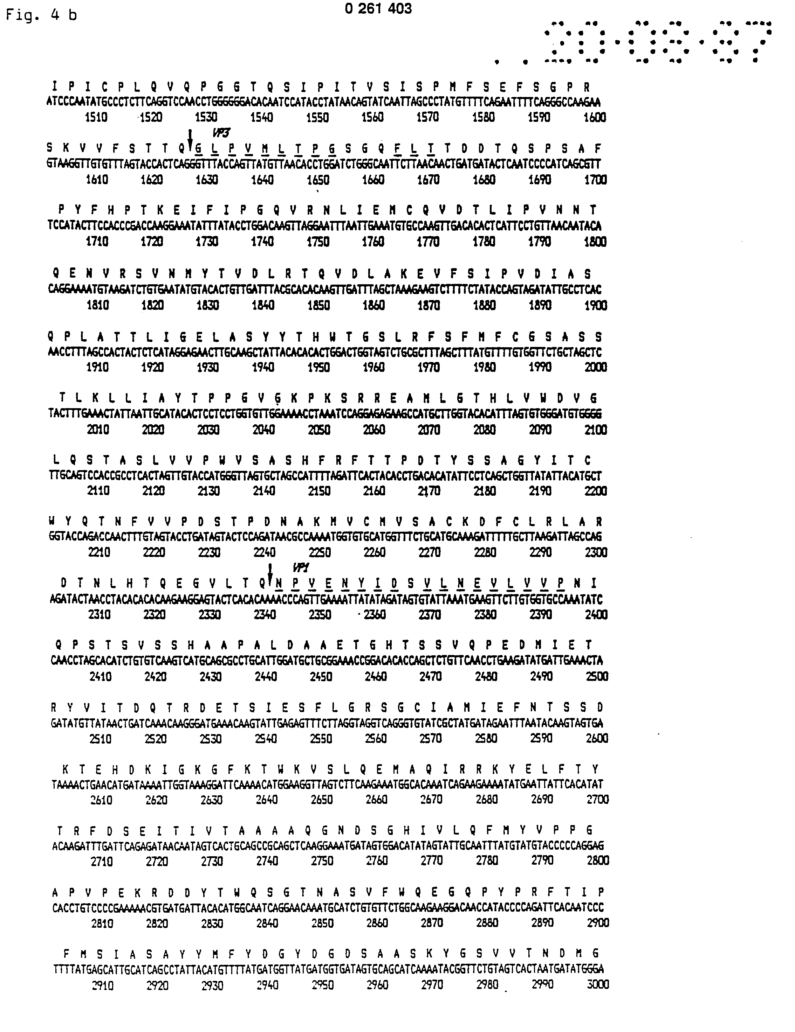

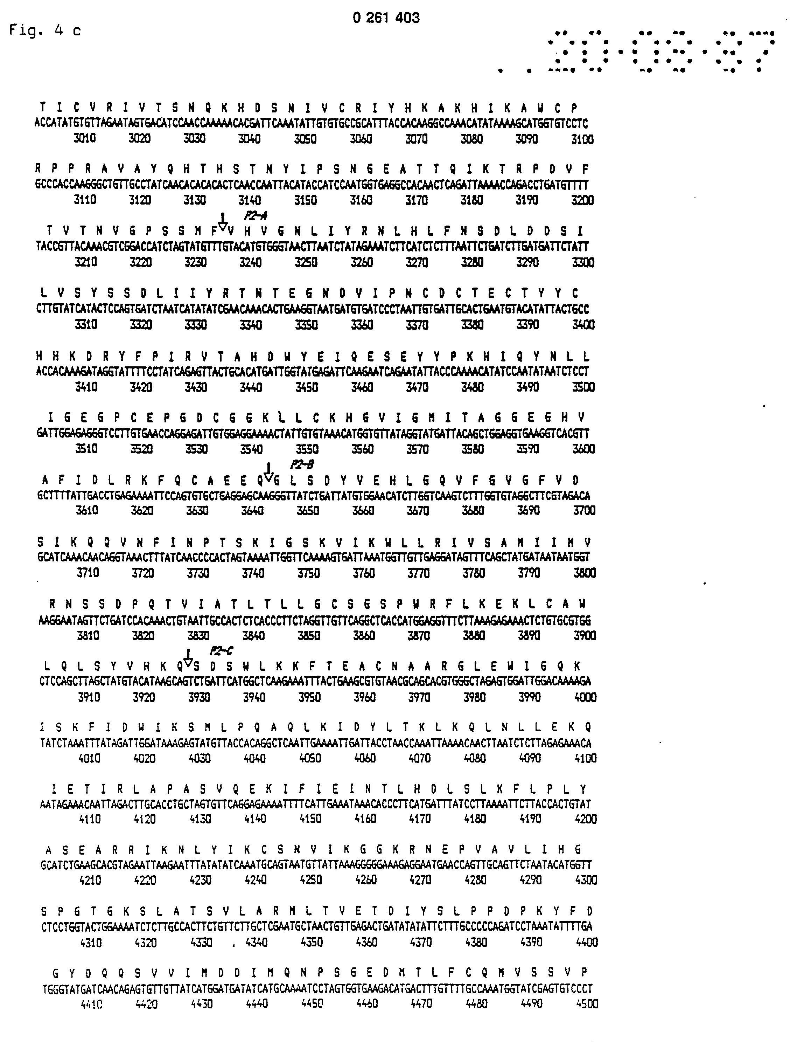

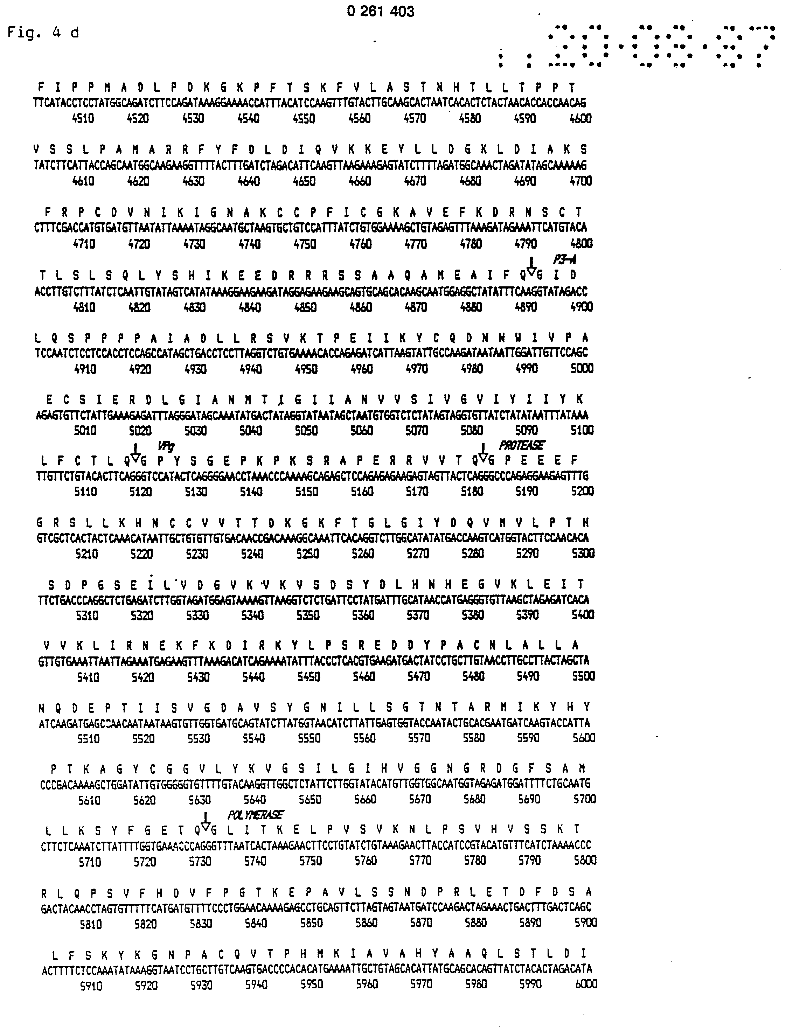

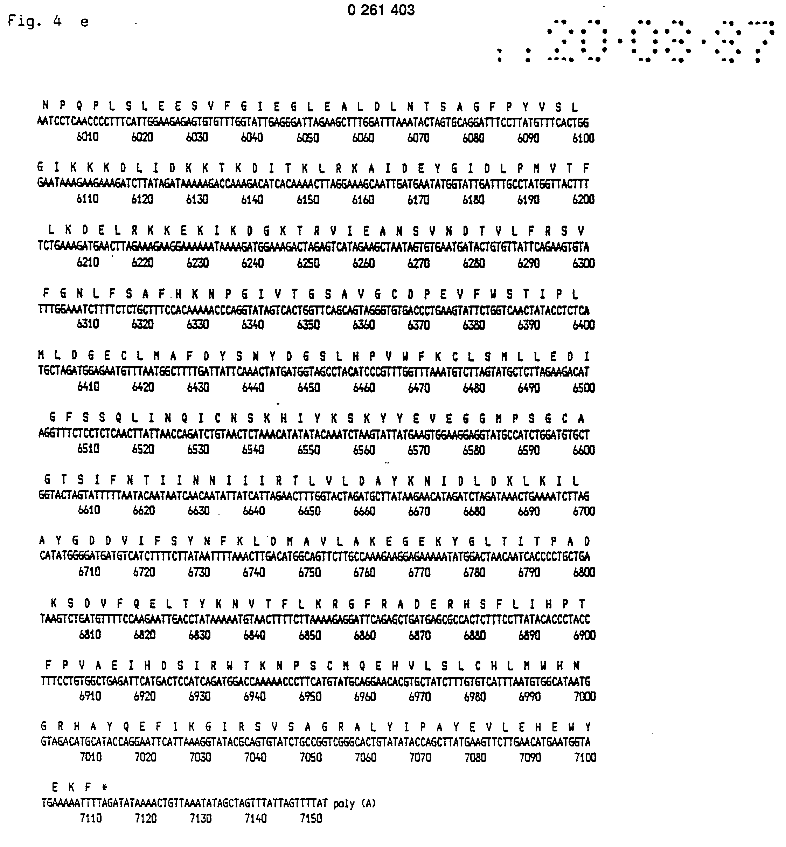

Die Sequenz des HRV89 Genoms ist in Fig. 4 gezeigt. Sie ist in Form der DNA dargestellt, wie sie aus der Sequenzierung der Rekombinanten-DNA erhalten worden ist. Zur Bestimmung der Sequenz wurden 21 Klone, die in Fig. 3 gezeigt sind, und zusätzlich 5 weitere Klone verwendet. Die Sequenz umfaßt 7152 Nukleotide, wobei das 3'-terminale Poly-A nicht mitgezählt ist. Sie enthält einen offenen Leserahmen, der für ein Polyprotein einer Länge von 2164 Aminosäuren kodiert. Der Leserahmen beginnt mit einem AUG in Position 619 und endet mit einem UAA Stop Codon 42 Nukleotide vor dem Beginn des Poly-A.

Die 5'-terminale Sequenz wurde aus den Klonen Nr. 5 und 10 erhalten, wobei Fragment D als Primer verwendet wurde (siehe Fig. 3). Spaltung mit PstI ergab ein Fragment konstanter Länge. Durch Sequenzierung wurde festgestellt, daß beide Klone die Sequenz TTAAAAC unmittelbar anschließend an Oligo-G enthielten, das von der Integrationsstelle im Plasmid stammt. Daraus wurde der Schluß gezogen, daß diese Klone den 5'-Terminus des HRV89-Genoms darstellen. Diese Sequenz entspricht den ersten 7 Nukleotiden der drei Poliovirus-Typen, Coxsackie-Virus B1 (Hewlett, M.J. und Florkiewicz, R.Z., 1980, Proc. Natl. Acad. Sci, USA 77, 303 - 307; Stanway, G., Cann, A.J., Hauptmann, R., Hughes, P., Clarke, L.D., Mountford, R.C., Minor, P.D., Schild, G.C. und Almond, J.W., 1983, loc. cit.), HRV2 (Skern, T., Sommergruber, W., Blaas, D., Gruendler, P., Fraundorfer, F., Pieler, C., Fogy, I. und Kuechler, E., 1985, loc. cit.) und HRV-14 (Stanway, G., Hughes, P.J., Mountford, R.C., Minor, P.D. und Almond, J.W., 1984 loc. cit).

Die Analyse der 618 Nukleotide zwischen dem 5'-Terminus und dem Beginn des langen offenen Leserahmens zeigt das Vorhandensein mehrerer kurzer Leserahmen. Ein Vergleich der Nukleotidsequenz der 5'-terminalen Region des HRV89 mit dem des HRV2 zeigt - unter Berücksichtigung von Insertionen - einen auffällig hohen Grad an Homologie von 79%, während die Homologie zu HRV14 67% und zu Poliovirus Typ 1 62% beträgt. Dabei ist die Homologie in einigen Bereichen besonders groß. Insgesamt sind 9 verschiedene Blöcke von 17 oder mehr hintereinander liegenden identischen Nukleotiden im 5'-terminalen Bereich des HRV89-und HRV2-Genoms vorhanden.

4

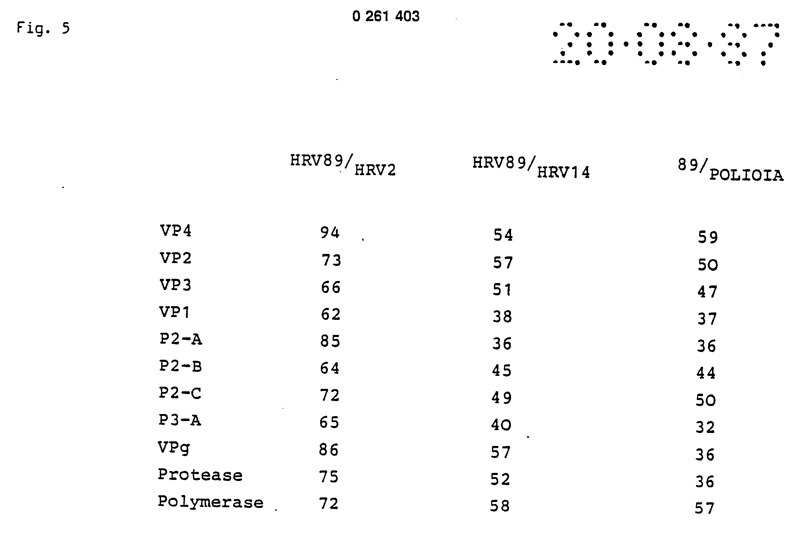

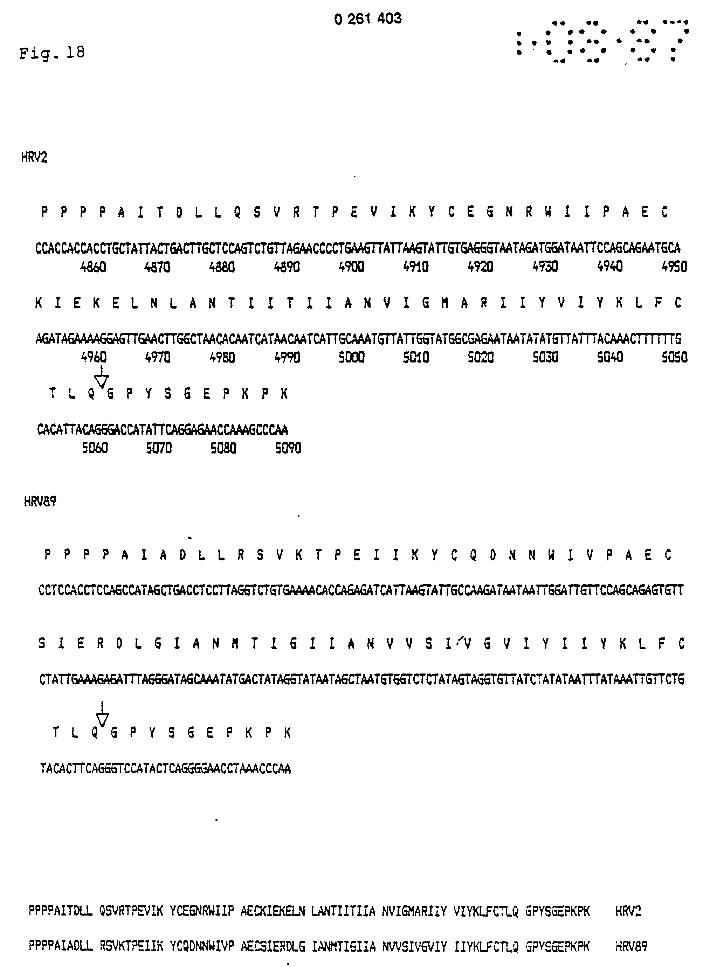

Der Vergleich der Aminosäure-Sequenzen zeigte, daß sich die Bereiche der Homologie auch in der für das Polyprotein codierenden Region fortsetzen (Fig. 5). Besonders auffällig ist die ausgeprägte Homologie zwischen HRV89 und HRV2 während überraschenderweise -entgegen den Erwartungen-die Homologie zu HRV14 viel geringer ist. Die stark konservierten Abschnitte umfassen sowohl die Hüllenproteine als auch den Bereich der nicht-strukturellen Proteine (P2A-P3D). Fig. 18 zeigt beispielsweise einen Sequenzvergleich zwischen einer Teilsequenz von HRV2 mit einer Teilsequenz aus dem HRV89, die aus dem Bereich der Gene für P3A und P3B (VPg) stammt. Die weitgehende Homologie in der Aminosäuresequenz ist leicht erkennbar. Selbst an den Stellen, an denen vereinzelt andere Aminosäuren vorkommen, findet man meist eine Substitution durch chemisch sehr nahe verwandte Aminosäuren, wobei Arginin (R) gegen Lysin (K), Valin (V) gegen Isoleucin (I), Leucin (L) gegen Isoleucin (I) etc. ausgetauscht sind. Auch die Spaltstelle zwischen P3A und P3B (VPg) ist vollkommen konserviert, wie auf Grund der Homologie leicht erkennbar ist. Auffällig ist hingegen eine Abweichung in einem kleinen Bereich (entsprechend der Sequenz zwischen den Nukleotiden 5018 und 5029 in HRV2), die zu einer Divergenz in der Aminosäuresequenz in der entsprechenden Region im P3A Anlaß gibt. Die Signifikanz dieses Befundes ist zur Zeit noch nicht geklärt. Über die Funktion des P3A ist bis jetzt nichts bekannt. Es kann daher nicht ausgeschlossen werden, daß Subtypen von HRV2 exisitieren, die in diesem Bereich eine größere Homologie zu HRV89 aufweisen, als in diesem Beispiel dargestellt ist. Dies bedeutet offenbar, daß trotz der Zugehörigkeit von HRV89 zur selben Rezeptorgruppe wie HRV14 die Verwandtschaft zu HRV2 größer ist. Aufgrund der Sequenz bilden also HRV2 und HRV89 eine gemeinsame Gruppe, die sich deutlich von HRV14 und Poliovirus absetzt. Dies könnte möglicherweise bedeuten, daß humane Rhinoviren untereinander doch größere Ähnlichkeiten besitzen, als bisher aufgrund des Homologievergleiches von HRV2 und HRV14 angenommen war. Damit - scheint auch der Vorschlag, Rhinoviren und Enteroviren gemeinsam als eine Gattung innerhalb der Familie der Picornaviren zusammenzufassen (Stanway, G., Hughes, P.J., Mountford, R.C., Minor, P.D. und Almond, J.W., 1984 loc. cit.) in Frage gestellt.

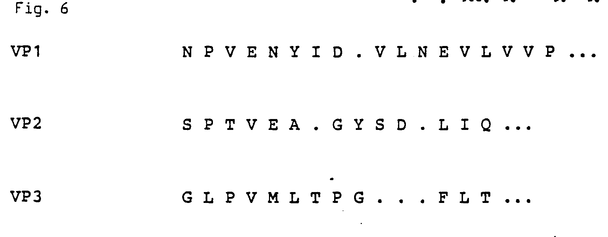

Die viralen Proteine werden aus dem Polyprotein durch proteolytische Spaltung erhalten. Zur Ermittlung der Spaltstellen wurden die viralen Hüllenproteine isoliert und die N-terminale Aminosäuresequenz bestimmt (Fig. 6). Hierdurch wurden nicht nur die Spaltstellen in den viralen Hüllenproteinen eindeutig identifiziert, sondern auch das Leseraster, das aus der Nukleotidsequenz abgeleitet worden war, wurde bestätigt. Aus dem Vergleich mit der aus der Nukleotidsequenz abgeleiteten Aminosäuresequenz ergibt sich, daß die VP4/VP2 Spaltung zwischen Glutamin und Serin, die VP2/VP3-Spaltung zwischen Glutamin und Glycin und die VP3/VP1-Spaltung zwischen Glutamin und Asparagin erfolgt. Diese Spaltstellen sind überraschenderweise mit denen von HRV2 identisch, unterscheiden sich aber von denen in HRV14 (Stanway, G., Huges, P.J., Mountford, R.C., Minor, P.D. und Almond, J.W., 1984 loc. cit.)

Die vorliegende Erfindung ermöglicht es, eine DNA herzustellen, die die Information für die virale RNA des HRV89 enthält.

Gegenstand der Erfingung sind jedoch nicht nur die Gen-Sequenzen, die spezifisch für die viralen Proteine codieren, sondern auch die Modifikationen, die beispielsweise durch Mutation, Abbau, Transposition oder Addition erhalten werden können. Jede Sequenz, die im Vergleich mit den gezeigten degeneriert ist, ist eingeschlossen. Auch die Sequenzen, die unter stringenten Bedingungen, beispielsweise unter Bedingungen, die für mehr als 85%, bevorzugt mehr als 90% Homologie selektieren, mit den gezeigten Sequenzen oder Teilen davon hybridisieren und für die Proteine mit dem viralen Aktivitätsspektrum codieren, sind eingeschlossen. Die Hybridisierungen werden in 6 $\times$ SSC/5 $\times$ Denhardt's-Lösung/0,1% SDS bei 65°C durchgeführt. Der Grad der Stringenz wird im Waschschritt festgelegt. So sind die Bedingungen, die für eine Selektionierung auf DNA-Sequenz mit ca. 85% oder mehr Homologie geeignet sind, wie folgt: 0,2 $\times$ SSC/0,1% SDS/65°C, für die Selektionierung auf DNA-Sequenzen mit ca. 90% oder mehr Homologie: 0,1 $\times$ SSC/0,01% SDS/65°C.

Diese DNA kann, wie gezeigt, sowohl vollständig als auch in Fragmenten in geeignete Plasmidvektoren eingebaut werden, um nach der Transformation von geeigneten Wirtsorganismen entweder die DNA zu vervielfältigen oder die Expression der Proteine selbst zu erreichen. Geeignete Wirte, Vektoren und die Bedingungen für diese Operationen sind dem Fachmann bestens bekannt. Ebenso sind viele Arbeiten publiziert, in denen die Synthese fremder Proteine in Bakterien mit Hilfe gentechnologischer Methoden beschrieben ist (Übersichtsartikel, siehe Harris, T.J.R. in "Genetic Engineering", Williamson, R., Hrsg., 1983, Vol.4. Academic Press, London, 127 ff.). Zu diesem Zweck wird die fremde DNA in die Nähe geeigneter bakterieller Kontrollregionen (Promotoren, Ribosomenbindungsstellen) von Plasmiden eingeführt, die es ermöglichen, diese Information -meist in Form von Fusionsproteinen-in hohen Ausbeuten zu exprimieren und so die entsprechenden Proteine zu gewinnen.Auch auf dem Gebiet der Picornaviren gibt es bereits eine Reihe von Publikationen, in denen die Expression viraler Gene in Bakterien beschrieben ist (Küpper, H., Keller, W., Kurz, C., Forss, S., Schaller, H., Franzel, R., Strohmaier, K., Marquardt, O., Zaslavsky, V.G.

und Hofschneider, P.H., 1981, Nature (London) 289, 555 - 559; Kleid, D.G.., Yansura, D., Small. B., Darbenko, D., Moore, D.M., Grubman, M.J., McKercher, P.D., Morgan, D.O., Robertson, B.H. und Bachrach, H.L., 1981, Science 214, 1125 - 1129; Wychowski, C., van der Werf, S., Siffert, O., Crainic, R., Bruneau, P. und Girard, M., 1983, EMBO J. 11, 2019 - 2024; Klump, W., Marquardt, O. und Hofschneider, P.H., 1984, Proc. Nat. Acad. Sci. USA 81, 3351 - 3355; Hanecak, R., Semler, B. L., Ariga, H. Anderson, C.W. und Wimmer, E., 1984, Cell 37, 1063 - 1073; Skern, T., Sommergruber W., Blaas, D., Gruendler, P., Fraundorfer, F., Pieler, C., Fogy, I. und Kuechler, E., 1985, loc. cit.; Strebel, K., Beck, E., Strohmaier, K. und Schaller, H., 1985, J. Virol. 57, 983-991; Toyoda, H. et al. 1986, loc. cit.)

Für die Expression bevorzugt sind Prokaryoten, beispielsweise E. coli K 12, Stamm 294 (ATCC No. 31 446). Andere Stämme, die geeignet sind, beinhalten E. coli X1 1776 (ATCC Nr. 31 537). Ebenso wie die vorher erwähnten Stämme können auch E. coli W 3110 F⁻, Lambda⁻, Prototroph, ATCC Nr. 27325), Bazillen wie Bacillus subtilis, und andere Enterobacteriaceae, wie Salmonella typhimurium oder Serratia marcescens und verschiedene Pseudomonaden verwendet werden.

Im allgemeinen können Plasmid-Vektoren, die Replikon und Kontrollsequenzen, die aus Spezies stammen, die kompatibel mit den Wirtszellen sind, enthalten, in Verbindung mit diesen Wirten verwendet werden. Der Vektor trägt üblicherweise neben einer Replikationsstelle Erkennungssequenzen, die es ermöglichen, in transformierten Zellen phenotypisch zu selektionieren. Zum Beispiel wird E. coli üblicherweise mit pBR322 transformiert, ein Plasmid, das aus E. coli Spezies stammt (Bolivar, et al., Gene 2, 95 (1977)).

pBR322 enthält Gene für Ampicillin-und Tetracyclin-Resistenz und liefert damit einfache Mittel, transformierte Zellen zu identifizieren. Das pBR322-Plasmid oder auch andere Plasmide müssen außerdem von sich aus Promotoren enthalten oder müssen dahingehend modifiziert sein, daß sie Promotoren enthalten, die von mikrobiellen Organismus zur Expression ihrer eigenen Proteine verwendet werden können. Die Promotoren, die am häufigsten bei der Herstellung rekombinanter DNA verwendet werden, beinhalten die Beta-Lactamase (Penicillinase) und Lactose-Promotor-Systeme (Chang et al., Nature 275, 615 (1978); Itakura et al., Science 198, 1056 (1977); Goeddel et al., Nature 281, 544 (1979) und Tryptophan (trp) Promotor-Systeme (Goeddel et al., Nucleic Acids Res. 8, 4057 (1980); Europa-Anmeldung, Offenlegungs-Nr. 0036 776).

Während die erwähnten die gebräuchlichsten Promotoren sind, sind darüber hinaus auch andere mikrobielle Promotoren entwickelt und benutzt worden. Die erfindungsgemäße Gen-Sequenz kann beispielsweise unter der Kontrolle des Leftward-Promotors des Bakteriophagen Lambda (P$_L$) eingesetzt werden. Dieser Promotor ist einer der als besonders stark bekannten Promotoren, der steuerbar ist. Die Steuerung wird möglich durch den Lambda-Repressor, von dem benachbarte Restriktionsschnittstellen bekannt sind.

Ein temperaturempfindliches Allel dieses Gens kann in einem Vektor, der eine virale DNA-Sequenz enthält, eingefügt werden. Wird die Temperatur auf 42°C erhöht, wird der Repressor inaktiviert und der Promotor bis zu seiner maximalen Konzentration exprimiert. Die Summe der mRNA, die unter diesen Bedingungen produziert wird, sollte ausreichend sein, um eine Zelle zu erhalten, die unter ihren neuen synthetischen Ribonukleinsäuren ungefähr 10% enthält, die von dem P$_L$-Promotor stammt.

Auf diese Weise ist es möglich, eine Clon-Bank zu etablieren, in der eine funktionelle virale DNA-Sequenz in Nachbarschaft zu einer Ribosom-Bindungsstelle plaziert wird und zwar in varierenden Abständen zu dem Lambda-P$_L$-Promotor. Diese Klone können dann überprüft und der mit der höchsten Ausbeute selektiert werden.

Die Expression und Translation einer viralen DNA-Sequenz kann auch unter Kontrolle anderer Regulationssysteme, die als "homolog" zu dem Organismus in siener untransformierten Form gelten können, ablaufen. So enthält z.B. chromosomale DNA von einem Lactose-abhängigen E. coli ein Lactose oder Lac-Operon, das durch Ausschüttung des Enzyms Beta-Galactosidase den Lactose-Abbau ermöglicht.

Die Lac-Kontrollelemente können aus dem Bacteriophagen Lambda-pLac5, der infektiös für E. coli ist, erhalten werden. Das Lac-Operon des Phagen kann durch Transduktion aus derselben Bakterien-Spezies stammen. Regulationssysteme, die bei dem erfindungsgemäßen Verfahren Verwendung finden können, können aus plasmidischer DNA stammen, die dem Organismus eigen ist. Das Lac-Promotor-Operator-System kann durch IPTG induziert werden.

Andere Promotor-Operator-Systeme oder Teile hiervon können genausogut verwendet werden: beispielsweise Arabinose-Operator, Colicin E₁-Operator, Galactose-Operator, alkalischer Phosphatase-Operator, trp-Operator, Xylose A Operator, tac-Promotor u.ä.

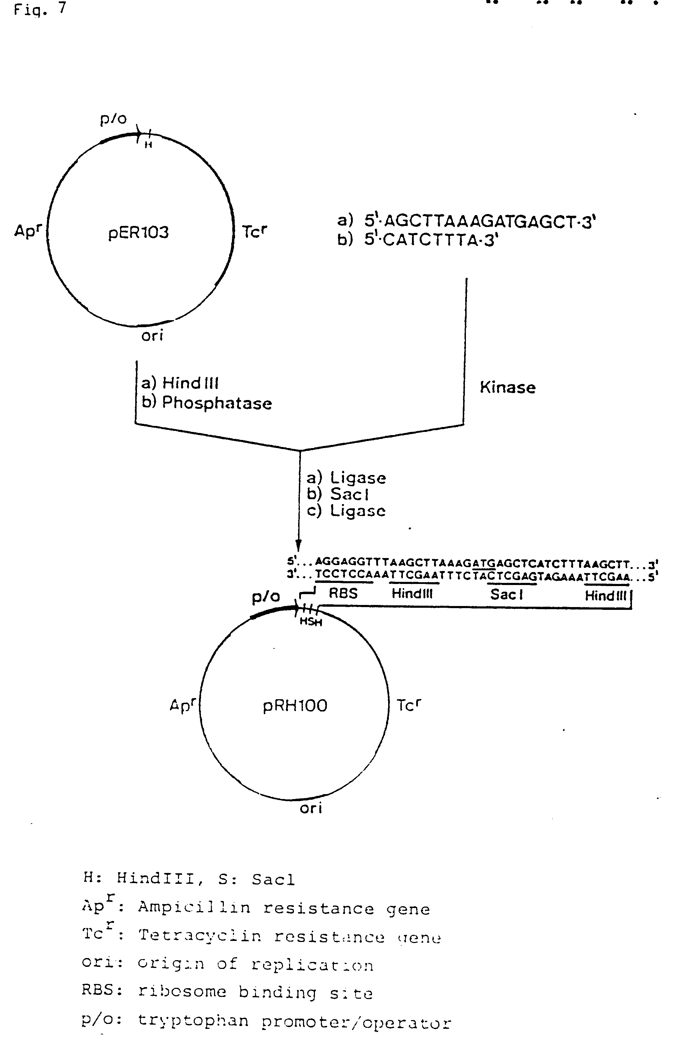

Die Gene können vorzugsweise in dem Expressions-Plasmid pER103 (Rastl-Dworkin et al., Gene 21, 237 - 248 (1983); vergleiche auch europäische Patentanmeldung No. 83 112 812.9, Hinterlegung DSM 2773, 20. Dezember 1983)oder in hiervon abgeleiteten Plasmiden, beispielsweise dem pRH100 (Figur 7), exprimiert werden. Diese Vektoren enthalten alle Regulationselemente, die zu einer hohen Expressionsrate der klonierten Gene führen.

Die vorliegende Erfindung umfaßt sowohl den Einbau der gesamten DNA der humanen Rhinovirus Typen 2 und 89, den Einbau von Teilen dieser DNA als auch variierende Kombinationen dieser DNA-Fragmente in geeignete Plasmidvektoren, die Plasmidvektoren selbst, die damit transformierten Wirtsorganismen, weiterhin die Synthese der betreffenden Proteine, die Proteine selbst und deren Anwendung.

Wie bereits erwähnt, werden zur gentechnologischen Herstellung von Proteinen in Wirtsorganismen üblicherweise sogenannte Expressionsvektoren verwendet. Diese enthalten sogenannte regulative Sequenzen, die für eine optimale Transkription und Translation der heterologen DNA verantwortlich sind. Zu diesen regulativen Sequenzen zählen starke Promotoren vorzugsweise regulierbare Promotoren und in Transkriptionsrichtung des Promotors eine oder mehrere singuläre Schnittstellen für Restriktionsenzyme, die im geeigneten Abstand zum Promotor liegen müssen.

Einige Expressionsvektoren sind so konstruiert, daß vor der ersten singulären Schnittstelle "downstream" vom Promotor ein Startcodon (Initationscodon) eingefügt ist. Bei Verwendung von Vektoren ohne Startcodon wird die zu exprimierende DNA mit einem Startcodon versehen und eingefügt. Weiterhin muß der zu exprimierenden DNA im Vektor ein sogenanntes Stop-oder Terminationscodon folgen. Wichtig bei der Konstruktion eines Strukturgen-tragenden Expressionsvektors ist, daß dieses Strukturgen in Phase zum Startcodon eingefügt wird.

Das Einfügen der heterologen DNA in den Expressionsvektor kann auf bekannte Art und Weise vorgenommen werden. Da in den seltensten Fällen der Beginn der DNA dieselbe Erkennungsstelle aufweist wie die singuläre Schnittstelle des Expressionsvektors, müssen in den meisten Fällen die Schnittstellen kompatibel gemacht werden.

Das ist beispielsweise möglich durch Auffüllen oder Abverdauen überhängender Einzelstrangenden oder durch Anfügen entsprechender Linker. Besitzt der ausgewählte Expressionsvektor kein Startcodon, kann gleichzeitig mit dem Anfügen eines Linkers auch das Startcodon angefügt werden.

Bei all diesen Operationen muß sichergestellt sein, daß die heterologe DNA in Phase mit dem Startcodon steht.

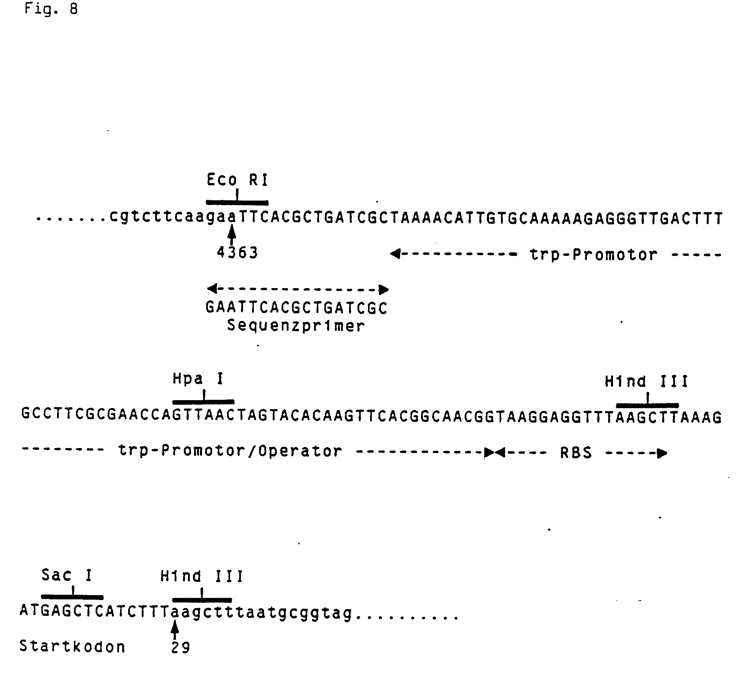

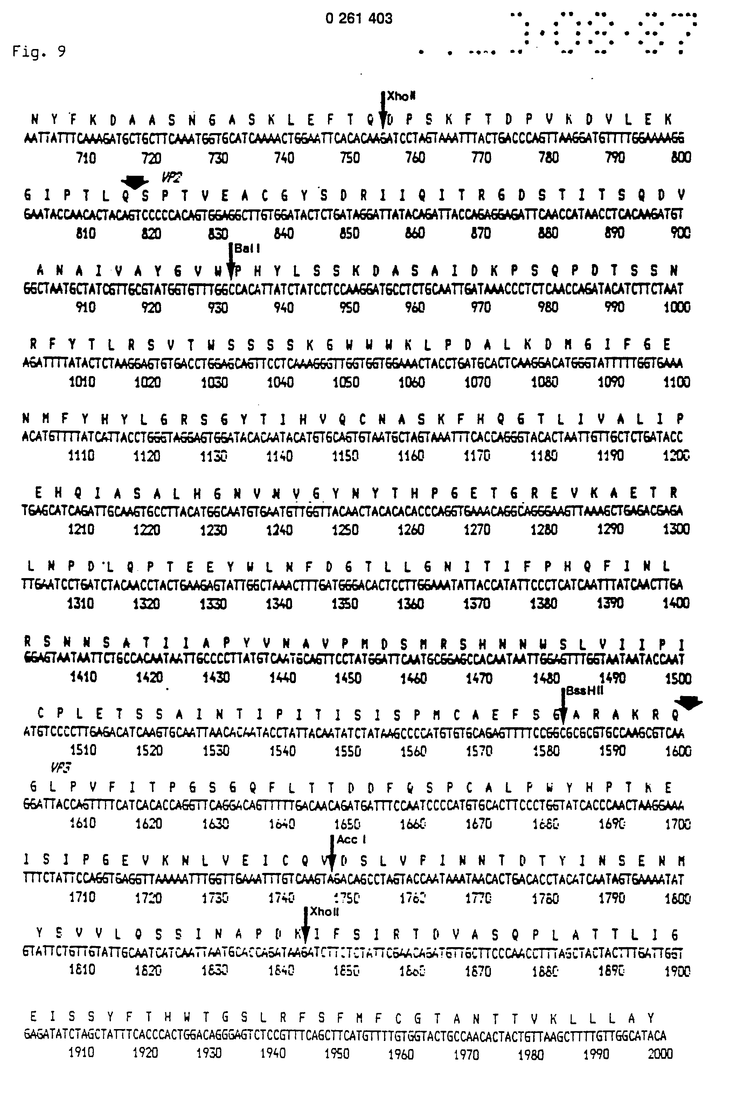

Besonders geeignet zur Lösung der erfindungsgemäßen Aufgabe ist die Verwendung des Vektors pRH100 (Fig. 7) und die daraus abgeleiteten Expressionsplasmide für die DNA oder DNA-Fragmente des HRV2 oder HRV89. Dieser Vektor - ein pBR322-Derivat - enthält als wesentliches Strukturmerkmal den Tryptophanpromotor von Serratia marcescens, der eine wirksame Signalsequenz für die Auslösung der Proteinsynthese darstellt und mit 3-β-Indolacrylsäure (=IAA; Induktor des trp-Operons) aktiviert werden kann. Hinter dem trp-Promotor befindet sich eine synthetische Ribosomenbindungsstelle und das Startkodon ATG. Unmittelbar hinter dem Startkodon befindet sich eine SacI-Schnittstelle (siehe Fig. 8). Für die Zwecke der Erfindung wurden die Einzelstrangenden dieser Schnittstelle mit einer Einzelstrang spezifischen Nuklease entfernt. Ligiert man das so modifizierte Plasmid mit einer DNA oder einem DNA-Fragment ("blunt-end"), beispielsweise dem "blunt-end" XhoII-Fragment 969/1 der HRV2-cDNA (siehe Fig. 9), so entstehen Expressionsplasmide, die in hiermit transformierten Wirtszellen, beispielsweise prokaryotischen oder eukaryotischen Zellsystemen zur Produktion von Proteinen ohne Fusionsanteil führen. Die Expression solcher Proteine hat gegenüber einem Fusionsprotein den Vorteil, daß das exprimierte Protein in seiner nativen Struktur zur Verfügung steht und immunologischen oder enzymatischen Tests unterworfen werden kann.

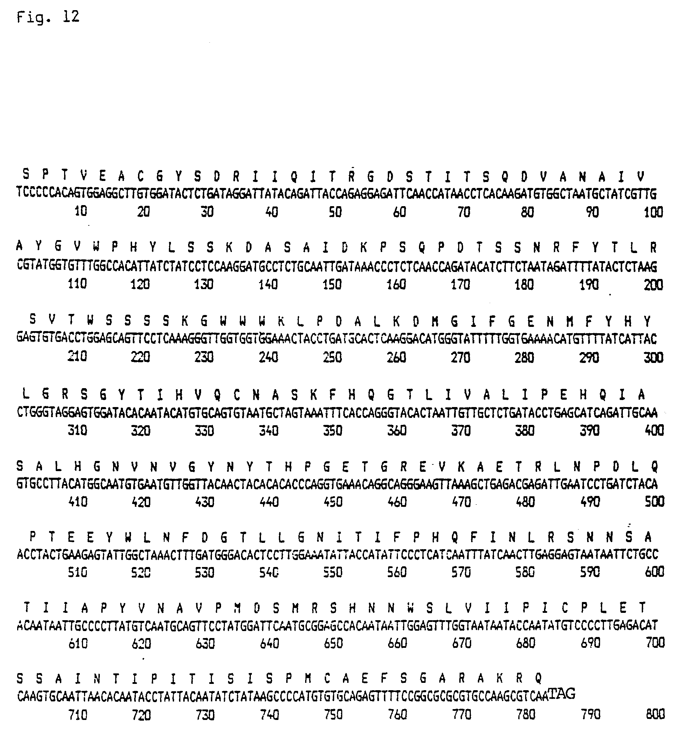

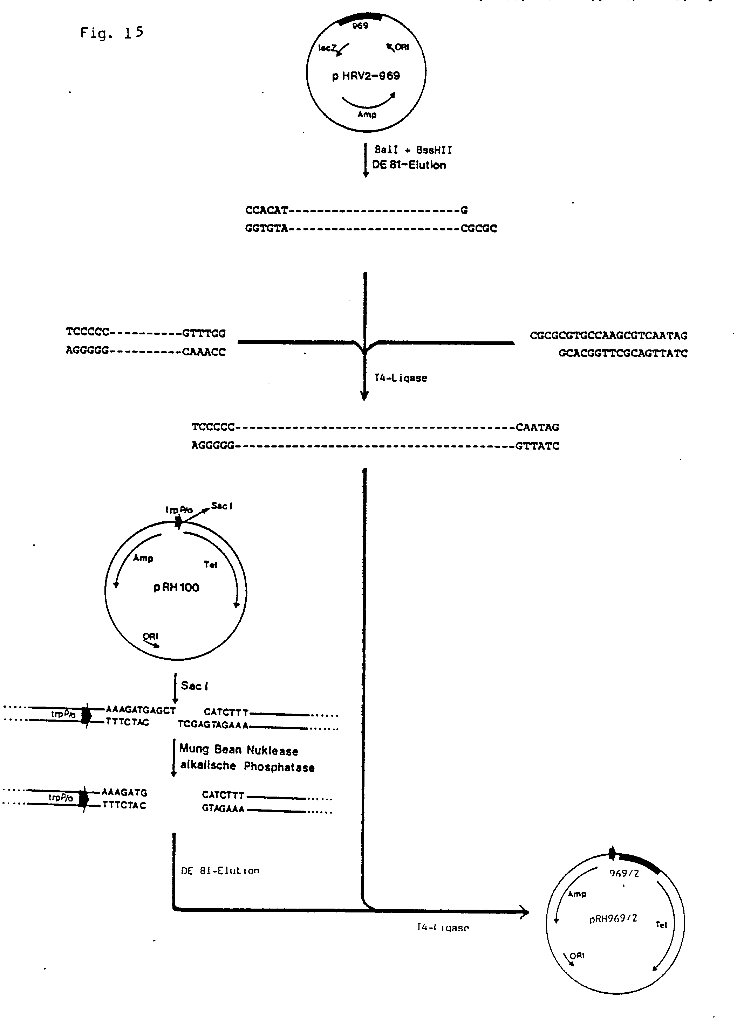

Durch die Kenntnis der vollständigen DNA Sequenz des HRV2 und des HRV89 ist es erfindungsgemäß möglich, jedes gewünschte Fragment der DNA auf die gezeigte Weise in geeignete Expressionsplasmide beispielsweise in das Expressionsplasmid pRH100 einzufügen und zur Expression zu bringen, wenn man beachtet, daß die Fragmente im richtigen Abstand zu den regulativen Sequenzen und im richtigen Leserahmen zum Initiationscodon ATG stehen. Die gewünschten Fragmente können dabei eintweder durch vollständige chemische Oligonukleotidsynthese oder durch Fragmentieren der nach der EPA 0 192 175 oder der DE 36 28 658.3 erhältlichen Plasmide hergestellt werden. Auch möglich ist die Kombination beider Verfahren. So kann man beispielsweise das in Fig. 9 dargestellte DNA-Molekül, z.B. aus dem Plasmid pHRV2-969 durch Doppelverdau mit den Restriktionsenzymen BalI und BssHII an den Positionen 930 bzw. 1581 fragmentieren. Um dann beispielsweise das reife VP2 zur Expression zu bringen, benötigt man zwei Oligonukleotidstücke, die die Nukleotide 818 bis 931 bzw. 1582 bis 1600 + TAG aus Fig. 9 repräsentieren. Die Oligonukleotide lassen sich nach an sich bekannter Weise chemisch herstellen beispielsweise mit Hilfe eines Applied Biosystems Model 381A DNA Synthesizer. Nach Verknüpfung dieser Fragmente (Fig. 12) und Einfügen in die "stumpfe" SacI Schnittstelle des Plasmides pRH100 erhält man ein zur Expression des reifen VP2 geeignetes Expressionsplasmid pRH969/2 (Fig. 15).

Diese aufgezeigte Strategie ist, wie oben bereits erwähnt, auch für die anderen viralen Proteine des HRV2 oder HRV89 anwendbar.

Unter Ausnutzung der drei HindIII-Schnittstellen in der cDNA des HRV89 läßt sich ein Expressionsplasmid konstruieren, das die Nukleotide von 2251-4703 des HRV89 enthält. Hierzu schneidet man das, den gesamten viralen Proteinbereich kodierende DNA-Molekül mit HindIII, isoliert und reinigt das ca. 3860 bp große Fragment H-H und schneidet anschließend mit SphI. Diesem Fragment S-H, das die Nukleotide 2273-4703 repräsentiert, wird gegebenenfalls an einem oder beiden Enden ein solcher Linker angefügt, der die Einfügung des Fragmentes in einen geeigneten linearisierten Expressionsvektor im korrekten Leserahmen ermöglicht.

Die Wahl des bzw. der Linker richtet sich nach den in dem Expressionsvektor verfügbaren Restriktionsstellen. Besonders vorteilhaft sind solche Linker zu verwenden, die in Phase mit der zu exprimierenden DNA-Sequenz ein Startcodon wie ATG und Terminationscodone wie z.B. TAA aufweisen, wenn diese nicht bereits durch den Expressionsvektor bzw. durch die zu exprimierenden Sequenz bereitgestellt werden.

Ein geeigneter Expressionsvektor ist beispielsweise der von Pharmacia unter der Kat.-Nr. 27-4935-01 erhältliche Vektor pKK233-2 (4602 bp) (E.Amann und J. Brosius; Gene, 40 (1985) 183-190). Dieser Vektor wird mit NcoI und HindIII doppelverdaut, das große Fragment isoliert und gereinigt. Um das Fragment S-H in den linearisierten Vektor einfügen zu können, wird es mit einem NcoI-SphI Linker der Formel

CATGGTGTGCATGGTTTCTGCATG
CACACGTACCAAAGAC

versehen. Das mit diesem Linker versehene Fragment S-H wird mit dem linearisierten Vektor ligiert. Die für die jeweiligen Operationen erforderlichen Reagenzien sind dem Fachmann bekannt und werden in bekannter Weise eingesetzt.

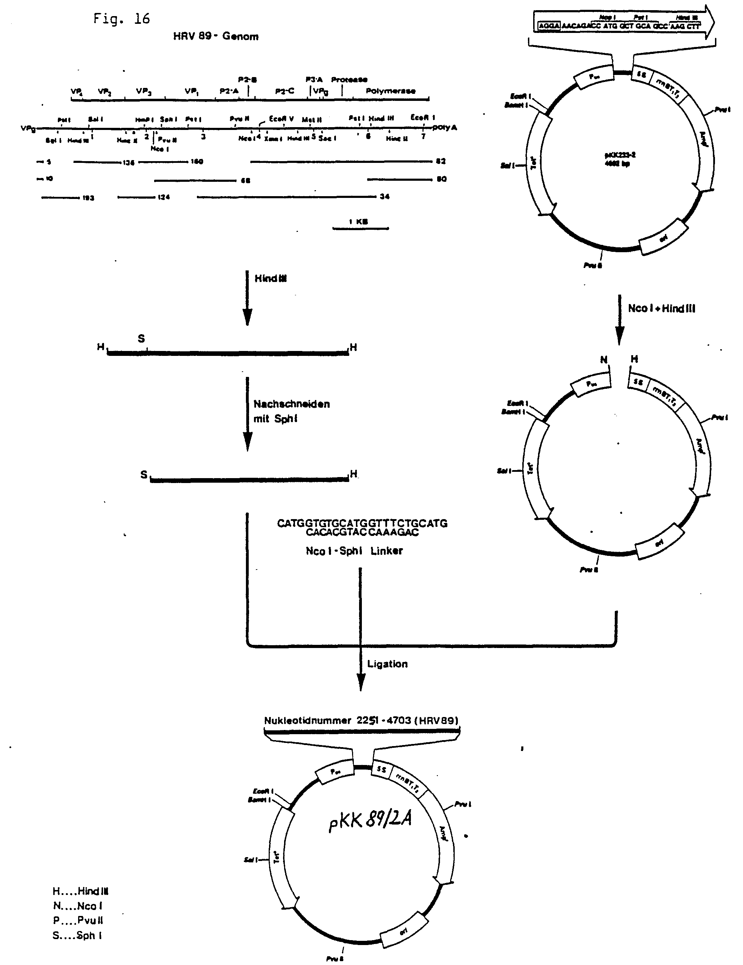

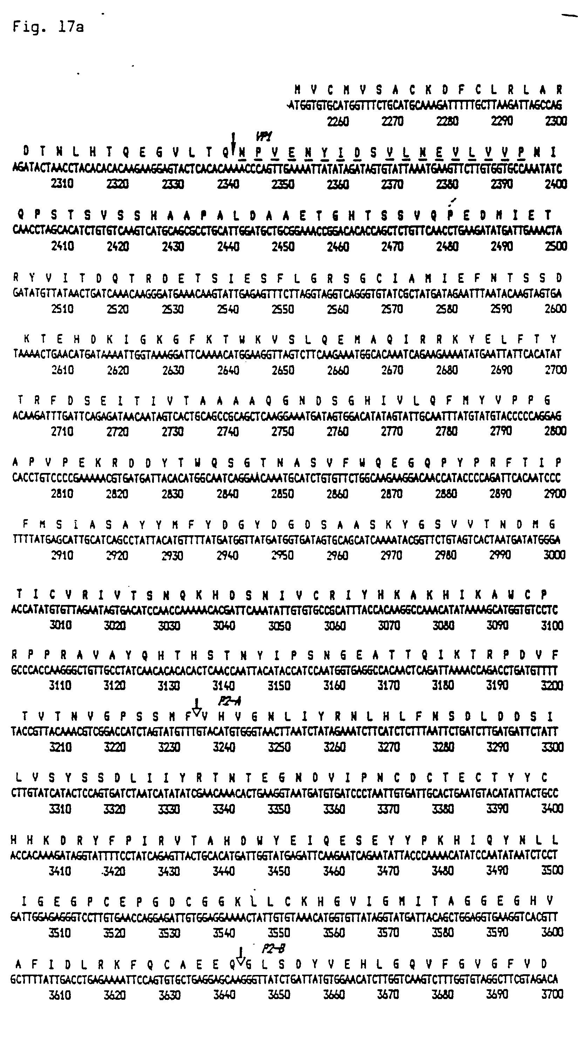

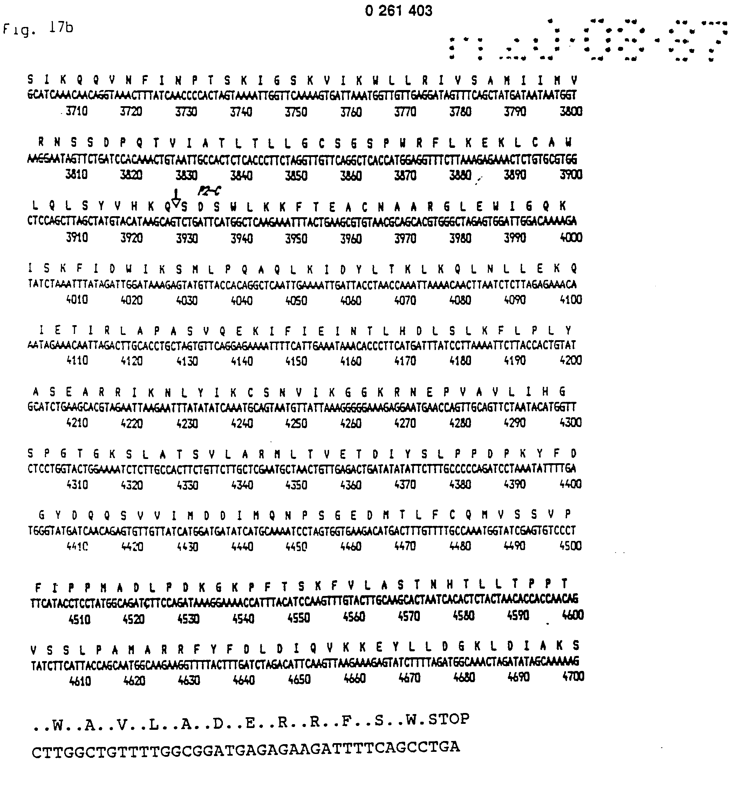

Das so erhältliche Expressionsplasmid pKK 89/2A (Fig. 16) ist durch IPTG-Induktion regulierbar und ermöglicht in E.coli, beispielsweise in dem Stamm JM 105 die Expression des in Figur 17 dargestellten reifen Proteins (die zusätzlichen Aminosäuren stammen aus dem Vektor).

Dieses Protein wird dann exprimiert, wenn das in optimalem Abstand zu den regulativen Regionen befindliche ATG für das Met an Position 545 als Start von dem Ribosom genutzt wird. Denkbar ist jedoch auch, daß erst das ATG für Met an Position 548 als Startsignal genutzt wird; ein um drei Aminosäuren verkürztes Protein wäre die Folge.

Zusätzlich zu Prokaryoten können auch eukaryotische Mikroorganismen, wie Hefekulturen verwendet werden. Saccharomyces cerevisiae ist der am meisten verwendete unter den eukaryotischen Mikroorgansimen, obwohl eine Anzahl anderer Spezies allgemein erhältlich ist. Zur Expression in Saccharomyces wird beispielsweise das Plasmid YRp7 (Stinchcomb, et al. Nature 282, 39 (1979); Kingsman et al., Gene 7, 141 (1979); Tschumper, et al., Gene 10, 157 (1980) und das Plasmid YEp 13 (Bwach et al., Gene 8, 121-133 (1979)) üblicherweise verwendet. Das Plasmid YRp7 enthält das TRP1-Gen, das eine Selektionierungsmarkierung für eine Hefemutante, die unfähig ist, in tryptophanhaltigem Medium zu wachsen, bereitstellt; beispielsweise ATCC Nr. 44076.

Das Vorhandensein des TRP1 Schadens als Charakteristikum des Hefe-Wirts Genoms stellt dann ein wirksames Hilfsmittel dar, um die Transformation nachzuweisen, indem ohne Tryptophan kultiviert wird. Ganz ähnlich verhält es sich bei dem Plasmid YEp13, das das Hefe-Gen LEU 2, das zur Ergänzung einer LEU-2 Mutante verwendet werden kann, erhält. Geeignete Promotor-Sequenzen für Hefe Vektoren beinhalten die 5'-flankierende Region des ADH I (Ammerer, G., Methods of Enzymology 101, 192-201 (1983), 3-Phosphoglycerat-Kinase (Hitzeman, et al., J. Biol. Chem. 255, 2073 (1980) oder andere glykolytische Enzyme (Kawasaki und Fraenkel, Biochem.Biophys.Res. Comm. 108, 1107-1112 (1982), wie Enolase, Glycerinaldehyd-3-phosphat-Dehydrogenase, Hexokinase, Pyruvat-Decarboxylase, Phosphofructokinase, Glucose-6-Phosphat-Isomerase und Glucokinase.

Bei der Konstruktion geeigneter Expressionsplasmide können die mit diesen Genen assoziierten Terminationssequenzen ebenfalls in den Expressions-Vektor am 3'-Ende der zu exprimierenden Sequenz eingesetzt werden, um Polyadenylierung und Termination der mRNA vorzusehen.

Andere Promotoren, die zudem noch den Vorteil der durch Wachstumsbedingungen kontrollierten Transkription besitzen, sind die Promotor-Regionen der Alkohol-Dehydrogenase-2, Isocytochrom C, Saure Phosphatase, abbauende Enzyme, die mit dem Stickstoff-Metabolismus gekoppelt sind, die oben erwähnte Glyceraldehyd-3-Phosphat-Dehydrogenase und Enzyme, die für die Verarbeitung von Maltose und Galaktose verantwortlich sind. Promotoren, die durch den Hefe Mating Typ Locus reguliert werden, beispielsweise Promotoren der Gene BARI, ME 1, STE2, STE3, STE5 können bei temperaturregulierten Systemen durch die Verwendung von temperaturabhängigen sir Mutationen eingesetzt werden. (Rhine, Ph. D. Thesis, University of Oregon, Eugene, Oregon (1979), Herskowitz and Oshima, The Molecular Biology of the Yeast Saccharomyces, part I, 181 - 209 (1981), Cold Spring Harbor Laboratory). Diese Mutationen beeinflussen die Expression der ruhenden Mating Typ Kassetten von Hefen und dadurch indirekt die Mating Typ abhängigen Promotoren. Generell ist jedoch jeder Plasmid-Vektor, der einen Hefe-kompatiblen Promotor, originäre Replikations-und Terminationssequenzen enthält, geeignet.

Zusätzlich zu Mikroorganismen sind Kulturen multizellulärer Organismen ebenfalls geeignete Wirtsorganismen. Im Prinzip ist jede dieser Kulturen einsetzbar, ob von Wirbeltier-oder wirbellosen Tierkulturen. Größtes Interesse besteht jedoch an Wirbeltier-Zellen, so daß die Vermehrung von Wirbeltierzellen in Kultur (Gewebe-Kultur) in den letzten Jahren zu einer routinemäßigen Methode wurde. (Tissue Culture, Academic Press, Kruse and Patterson, Editors (1973)). Beispiele solcher nützlichen wirtszellinien sind VERO-und HeLa-Zellen, Hamster-Eierstock (CHO)-Zellen und WI38, BHK, COS-7 und MDCK-Zellinien. Expressionsvektoren für diese Zellen enthalten üblicherweise (wenn nötig) eine Replikationsstelle, einen Promotor der vor dem zu exprimierenden Gen lokalisiert ist, gemeinsam mit jeder notwendigen Ribosomenbindungsstelle, RNA-Spleißstelle, Polyadenylierungsstelle und transkriptionelle Terminations-Sequenzen.

Bei der Verwendung in Säugetierzellen werden die Kontrollfunktionen auf den Expressions-Vektoren oftmals aus viralem Material vorgesehen. Beispielsweise stammen die üblicherweise verwendeten Promotoren aus Polyoma, Adenovirus 2, und besonders häufig aus Simian Virus 40 (SV 40). Die Anfangs-und Endpromotoren des SV 40 sind besonders nützlich, da beide leicht aus dem Virus als Fragment zu erhalten sind, das auch noch die virale Replikationsstelle des SV 40 enthält (Fiers et al., Nature 273, 113 (1978). Auch können kleinere oder größere Fragmente des SV 40 verwendet werden, vorausgesetzt, sie enthalten die annähernd 250 bp lange Sequenz, die von der HindIII Schnittstelle bis zur BglI Schnittstelle in der viralen Replikationsstelle reicht. Außerdem ist es ebenfalls möglich und empfehlenswert, Promotor-oder Kontroll-Sequenzen zu verwenden, die normalerweise mit den gewünschten Gensequenzen verknüpft sind, vorausgesetzt; diese Kontroll-Sequenzen sind kompatibel zu den Wirtszellsystemen.

Eine Replikationsstelle kann entweder durch entsprechende Vektorkonstruktion vorgesehen werden, um eine exogene Stelle einzubauen, beispielsweise aus SV 40 oder anderen viralen Quellen (z.B. Polyoma, Adeno, VSV, etc.)oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Transformation der Zellen mit den Vehikeln kann durch eine Vielzahl an Verfahren erreicht werden. Beispielsweise kann sie durch Kalzium erfolgen, wobei entweder die Zellen in Magnesium gewaschen werden und die DNA den in Kalzium suspendierten Zellen zugegeben wird oder die Zellen einem Kopräzipitat von DNA und Kalziumphosphat ausgesetzt werden. Bei nachfolgender Genexpression werden die Zellen auf Medien übertragen, die für transformierte Zellen selektieren.

Nach erfolgter Transformation des Wirtes, Expression des Gens und Fermentation oder Zellkultivierung unter Bedingungen, bei denen das Protein exprimiert wird, kann das Produkt üblicherweise durch bekannte chromatographische Trennmethoden extrahiert werden, um so ein Material zu erhalten, das das virale Protein mit oder ohne Leader und Tailing Sequenzen enthält. Das Protein kann mit einer Leader-Sequenz am N-Terminus exprimiert werden (Pre-Protein), die von einigen Wirtszellen entfernt werden kann. Wenn nicht, so ist eine Abspaltung des Leader-Polypeptids (wenn vorhanden) erforderlich, um reifes Protein zu erhalten. Alternativ kann das reife Protein im Mikroorganismus direkt produziert werden. Dazu kann die Precursor Sequenz des Hefe-Mating-Pheromons MF-alpha-1 verwendet werden, um eine korrekte "Reifung" des fusionierten Proteins und die Ausscheidung der Produkte in das Wachstumsmedium oder den periplasmischen Raum zu gewährleisten. Die DNA-Sequenz für funktionelles oder reifes Protein kann mit MF-alpha-1 an der vermuteten Schnittstelle verbunden sein.Neben der Verwendung der erfindungsgemäßen DNA zur Herstellung der betreffenden Proteine in Bakterien oder eukaryotischen Zellen kann sie auch dazu dienen, die aus der Nukleotidsequenz abgeleiteten Aminosäuresequenzen synthetisch, ganz oder in Teilen davon, herzustellen, beispielsweise durch Verwendung automatischer Peptid-Synthetisierer (z.B. Beckman Modell 990).

Diese Oligopeptide können dann, ebenso wie die gentechnologisch hergestellten Proteine entweder zur Anregung einer gegen intakte Viren gerichteten Immunantwort verwendet oder zur Bindung und Blockierung von zellulären Rezeptoren eingesetzt werden. Studien über die Verwendung von Oligopeptiden zur Anregung einer gegen Polioviren gerichteten Immunantwort sind publiziert worden (Emini, E.A., Jameson, B.A. und Wimmer, E., 1983, Nature (London) 30, 669-703); ähnliche Untersuchungen sind auch bei Maul-und Klauenseucheviren durchgeführt worden (Bittle, J.L., Houghton, R.A., Alexander, H., Shinnick, T.M.,

Sutcliffe, J.G., Lerner, R.A., Rowlands, D.J. und Brown, F., 1982 Nature (London), 298, 30 - 33; Pfaff, E., Mussgay, M., Böhm, H.O., Schulz, G.E. und Schaller, H., 1982, EMBO J. 1, 869-874). Die vorliegende Erfindung erstreckt sich auch auf die Oligo-und Polypeptidanteile von HRV89-Proteinen, die als Folge der Synthese oder zum Zwecke der Applikation, z.B. als Vakzine, mit anderen Oligo-oder Polypeptiden verbunden sind.

Ein weiterer Gegenstand der vorliegenden Erfindung sind monoklonale Antikörper und deren Herstellung, Antikörper, die gegen strukturelle und nicht strukturelle virale Proteine (in nativer und denaturierter Form) gerichtet sind. Die Bindung monoklonaler Antikörper an zelluläre Rezeptoren für HRV14 und deren Blockierung, ist bereits durch Colonno et al. (Europäische Patentanmeldung 0 169 146) beschrieben worden.

Die Eigenschaft von Antikörpen, spezifische Antigene zu binden, findent außerhalb des Körpers praktische Anwendung bei der qualitativen und quantitativen Bestimmung (Immuno-Assay) und bei der Reinigung der Antigene (Immunoaffinitätschromatographie). Serum immunisierter Tiere enthält normalerweise eine Vielzahl verschiedener Antikörper, die mit dem gleichen Antigen an verschiedenen Bindungsstellen mit verschiedener Affinität reagieren, dazu aber auch Antikörper gegen andere Antigene. Die erfolgreiche Anwendung von Antikörpern zur Bestimmung und Reinigung von Antigenen erfordert aber hohe Spezifität und Reproduzierbarkeit.

Homogene Antikörper, die diese Anforderungen erfüllen, sind durch die von Köhler und Milstein beschriebene Hybridoma-Technik zugänglich geworden. Prinzipiell besteht die Technik darin, daß Antikörper ausscheidende B-Lymphozyten, z.B. aus der Milz, immunisierter Tiere mit Tumorzellen verschmolzen ("fusioniert") werden. Die gebildeten Hybridoma-Zellen kombinieren die Fähigkeit zur unbegrenzten Vermehrung durch Teilung mit der Fähigkeit, einen einheitlichen Typ Antikörper zu bilden und auszuscheiden. Durch Kultivierung in einem selektiven Medium, in dem nicht fusionierte Tumorzellen absterben, Hybridoma-Zellen sich aber vermehren, und durch geeignete Manipulationen können Klone, d.h. Zellpopulationen, die sich von einer einzigen Hybridoma-Zelle ableiten und genetisch identisch sind, gewonnen und kultiviert und die durch die Zellen produzierten monoklonalen Antikörper isoliert werden.

Die vorliegende Erfindung betrifft monoklonale Antikörper gegen Rhinoviren der Stämme HRV2 und HRV89, Hybridomazellen, die solche Antikörper produzieren, Verfahren zu ihrer Herstellung und ihre Verwendung. Sie betrifft weiterhin monoklonale Antikörper, die gegen erfindungsgemäße virale Proteine gerichtet sind, die sie produzierenden Hybridomazellen sowie Verfahren zu deren Herstellung und ihre Verwendung. Bevorzugt sind Hybridomazellinien und die von diesen ausgeschiedenen monoklonalen Antikörper, die spezifisch mit den genannten viralen Proteinen oder Teilen davon reagieren. Das Verfahren zur Herstellung von monoklonalen Anti-HRV2 und Anti-HRV89-Antikörpern ist dadurch gekennzeichnet, daß man Mäuse mit den Rhinoviren bzw. den viralen Proteinen immunisiert, B-Lymphozyten derart immunisierter Tiere mit Myelomazellen fusioniert, die gebildeten Hybridomazellen kloniert, dann in vitro oder durch Injektion in Mäusen kultiviert und aus den Kulturen Antikörper isoliert.

Die Erfindung betrifft ferner Immuno-Affinitätschromatographie-Säulen und Test-Kits für Immunoassays, die diese Antikörper enthalten.

Nach dem erfindungsgemäßen Verfahren werden Mäuse, z.B. Balb/c-Mäuse, auf an sich bekannte Weise immunisiert. In einer bevorzugten Ausführungsform werden die Rhinoviren etwa wöchentlich oder auch in größeren Abständen während mehrerer Wochen, beispielsweise 5 bis 12 Wochen, injiziert, bis sich eine genügende Zahl Antikörper-produzierender B-Lymphozyten gebildet hat.

B-Lymphozyten enthaltende Organe, z. B. Milzzellen, der immunisierten Mäuse werden entnommen und mit solchen Myelomazellen fusioniert. die aufgrund einer Mutation in einem selektiven Kulturmedium nicht wachsen. Solche Myelomazellen sind bekannt und sind beispielsweise jene mit der Bezeichnung X63-Ag8, X63-Ag8.6.5.3, MPC-11 NS1-Ag4/1, MOPC-21 NS/1, NS-1 (ATCC TIB 18) oder SP 2/0. In einer bevorzugten Ausführungsform werden Milzzellen immunisierter Mäuse mit Myelomazellen der Zell-Linie NS-1 (ATCC TIB 18) fusioniert.

Die Fusion wird nach an sich bekannten Verfahren durch Mischen der B-Lymphozyten und der Myelomazellen unter Zugabe eines Zellfusionsagens, wie Polyethylenglykol, Sendai-Virus, Calciumchlorid oder Lysolecithin durchgeführt. Vorzugsweise wird in Gegenwart von Polyethylenglykol, beispielsweise mit einem Molekulargewicht zwischen 1000 und 4000, fusionert. Nach der Fusion werden die entstandenen Hybride nach einem an sich bekannten Verfahren in einem selektiven Kulturmedium, das mit Hypoxanthin, Aminopterin und Thymidin (HAT-Medium) komplementiert ist, kultiviert. Nicht fusionierte Myelomazellen können in diesem Medium nicht wachsen und sterben ebenso wie normale Lymphozyten.

Die Überstände der Hybridoma-Kulturen können mit an sich bekannten Verfahren auf ihren Gehalt an spezifischen Antikörpern geprüft werden, beispielsweise mit Radio-Immunoassay oder Agglutinierung. Dabei wurde überraschenderweise festgestellt, daß mit dem beschriebenen Verfahren Hybridoma-Zellen gewonnen werden können, die Antikörper spezifisch gegen die Rhinoviren ausscheiden. Die Hybridomazellen, die Antikörper der gewünschten Spezifität produzieren, werden aus dem aus der Fusionierung hervorgegangenen Gemisch verschiedenster Hybridomazellen durch Klonieren herausselektioniert. Dazu werden nach einem an sich bekannten Verfahren, das "limiting dilution" genannt wird, Kulturen ausgehend von einer einzigen wachsenden Zelle angesetzt.

Zur Massenproduktion werden die Hybridoma-Zellklone, die Antikörper der gewünschten Spezifität produzieren, entweder in an sich bekannten Medien in vitro kultiviert oder zur Vermehrung in Mäuse injiziert. In einer bevorzugten Ausführungsform werden Hybridomazellen in mit Pristan vorbehandelte Mäuse injiziert, Aszites-Flüssigkeit entnommen und daraus durch Fällung mit Ammoniumsulfat-Lösung Antikörper isoliert.

Die erfindungsgemäßen, gegen rhinovirale Proteine gerichteten monoklonalen Antikörper, z. B. der Antikörper 8F5, lassen sich therapeutisch einsetzen.

Die mit Hilfe dieser Hybridomazellen gewonnenen gegen die Rhinoviren gerichteten Antikörper können auch auf an sich bekannte Weise für die Herstellung von Immuno-Affinitätschromatographie-Säulen verwendet werden. In einer bevorzugten Ausführungsform der Erfindung wird ein geeignetes Trägermaterial (suspendiert in einer Pufferlösung) mit einer Antikörper-Lösung versetzt, ungebundene Anteile werden anschließend ausgewaschen und unbesetzte Stellen des Trägermaterials blockiert. Die mit Hilfe der Hybridomazellen gewonnenen spezifischen Antikörper können auf an sich bekannte Weise für die Herstellung von Test-Kits verwendet werden. Diese Test-Kits können auf verschiedenen Methoden beruhen, beispielsweise auf Radio-Immuno-Assay, Latex-Agglutinierung, Tüpfel-Tests, Kompetitive oder Sandwich-Radio-Immunoassay, Enzym-Immunoassay, Immuno-Fluoreszenz oder immunochemischen Enzym-Tests. Solche Kits können neben gewöhnlichen Antikörpern verschiedener Herkunft Antikörper-Konjugate mit Enzymen oder Fluoreszenzträgern enthalten, dazu z.B. HRV2 bzw. HRV89 markiert mit radioaktiven Isotopen wie $J^{125}$, oder konjugiert mit Enzymen, beispielsweise mit Meerrettich-Peroxidase oder alkalischer Phosphatase, ferner Enzymsubstrate, geeignete Puffer, Gele, Latex, Polystyrol oder andere Füllmaterialien und Träger.

Weitere Eigenschaften und Merkmale der vorliegenden Erfindung sind in den folgenden Beispielen beschrieben, die Ausführungsbeispiele der vorliegenden Erfindung betreffen und diese nicht einschränken.

Wird im Rahmen dieser Anmeldung von Eigenschaften und/oder von biologischer Aktivität im Zusammenhang mit Proteinen (Polypeptiden) gesprochen, so bedeutet dies, daß das betreffende Protein (Polypeptid) im biologischen Test eine Immunantwort stimulieren und/oder irgendeine Reaktion mit den zellulären Rezeptoren für die Rhinoviren eingeht.Gegenstand der vorliegenden Erfindung sind im Einzelnen:

DNA-Moleküle, die für mindestens ein virales Protein des Rhinovirusstammes HRV89 codieren,

-die der gesamten viralen RNA oder Teilen der viralen RNA des Rhinovirusstammes HRV89 entsprechen

-die für ein virales Protein codieren ,das aus den viralen Proteinen VP1, VP2, VP3, VP4, P2A, P2B, P2C, P3A, P3B und P3C zusammengesetzt ist,

-die für ein virales Protein codieren, das aus mindestens zwei der genannten, in beliebiger Kombination miteinander verknüpften Proteine besteht

-oder DNA-Moleküle, die für die einzelnen viralen Proteine selbst codieren.

Degenerierte Formen und DNA-Moleküle, die Allele codieren sind ebenfalls Gegenstand der vorliegenden Erfindung.

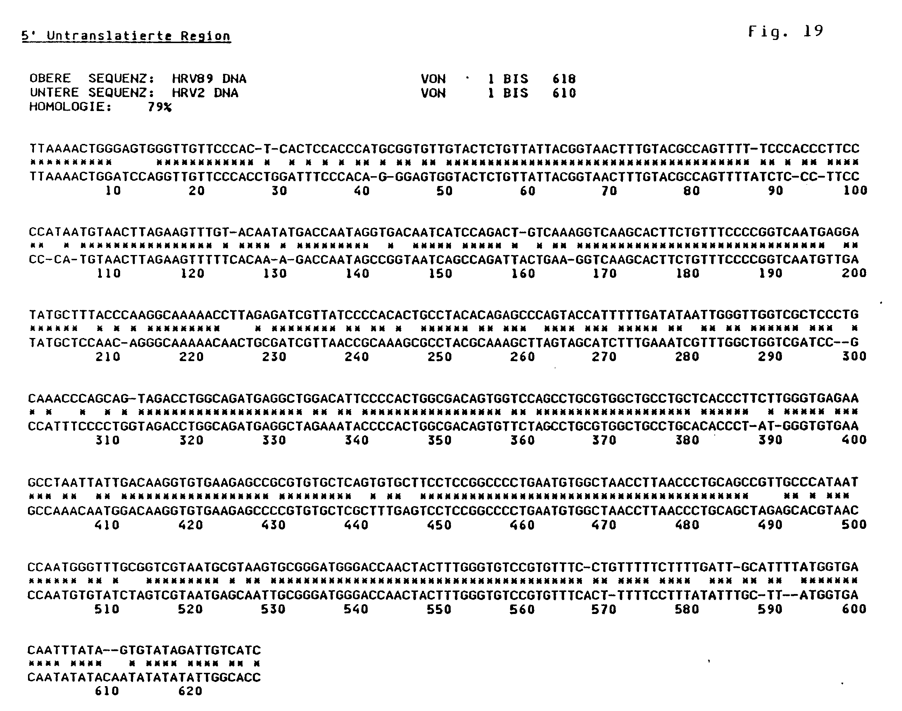

Bevorzugt sind DNA-Moleküle, die vollständig oder in Teilen der Sequenz gemäß Fig.4 und Fig. 19 entsprechen.

Ebenfalls Gegenstand der Erfindung sind DNA-Moleküle, die für mindestens ein virales Protein des Rhinovirusstammes HRV89 codieren, die unter stringenten Bedingungen, die es erlauben, eine Homologie zu erkennen, die größer als 85% ist, mit einem der angegebenen DNA-Moleküle oder mit degenerierten Variationen dieser Moleküle hybridisieren. Die erfindungsgemäßen DNA-Moleküle können in ein geeignetes Expressionsvehikel, beispielsweise in ein Plasmid, das replizierbar in Mikroorganismen, vorzugsweise in Prokaryoten, Eukaryoten oder in Säugetierzellen ist, eingebaut werden. Auch sind DNA-Moleküle, die für Proteine codieren, die die biologische Aktivität zumindest von einem der erfindungsgemäßen Proteine aufweisen, Gegenstand der vorliegenden Erfindung.

Weiterhin sind Gegenstand der Erfindung transformierte Wirtsorganismen, die die für die viralen Proteine gemäß der Erfindung codierenden genetischen Informationen enthalten, vorzugsweise Prokaryoten, Eukaryoten oder Säugetierzellinien insbesondere E.coli.

Die genetische Information ist dabei vorzugsweise in Vehikeln enthalten, die in dem betreffenden Wirtsorganismus replizierbar sind.

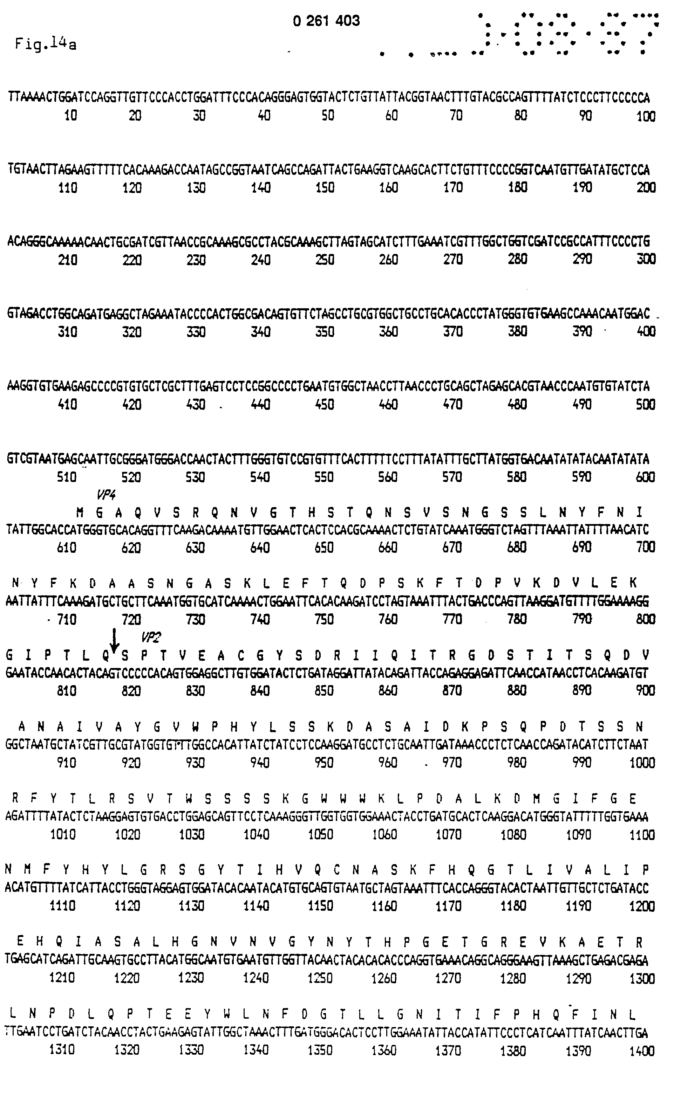

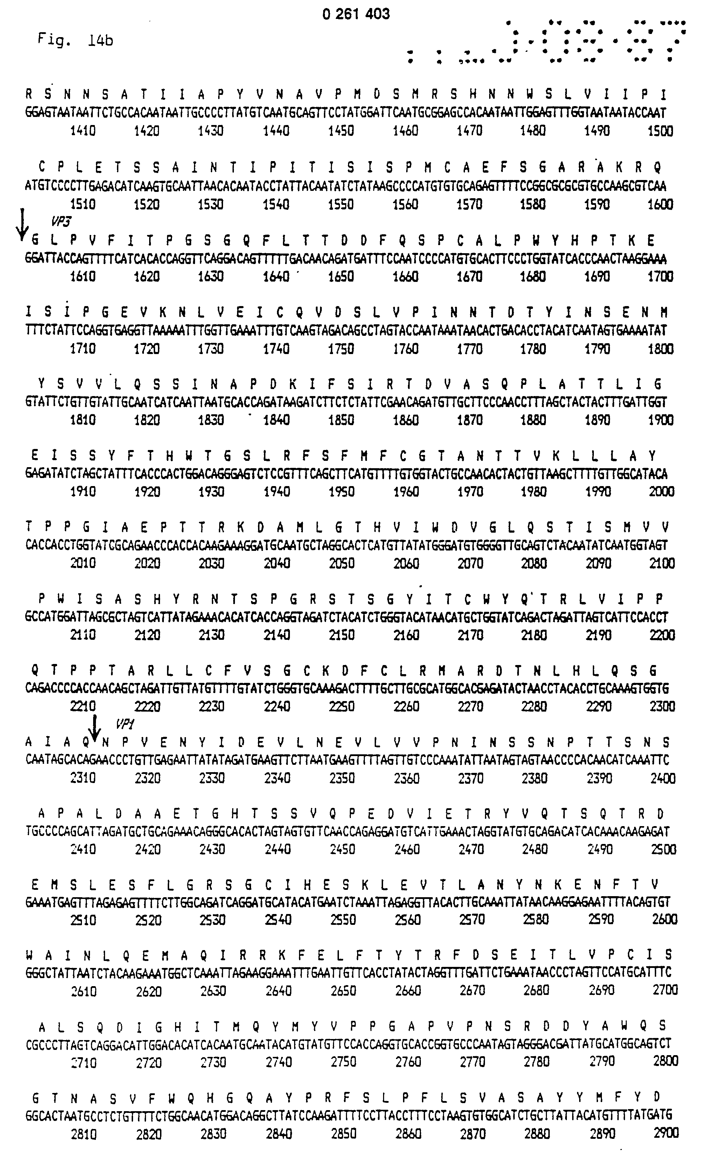

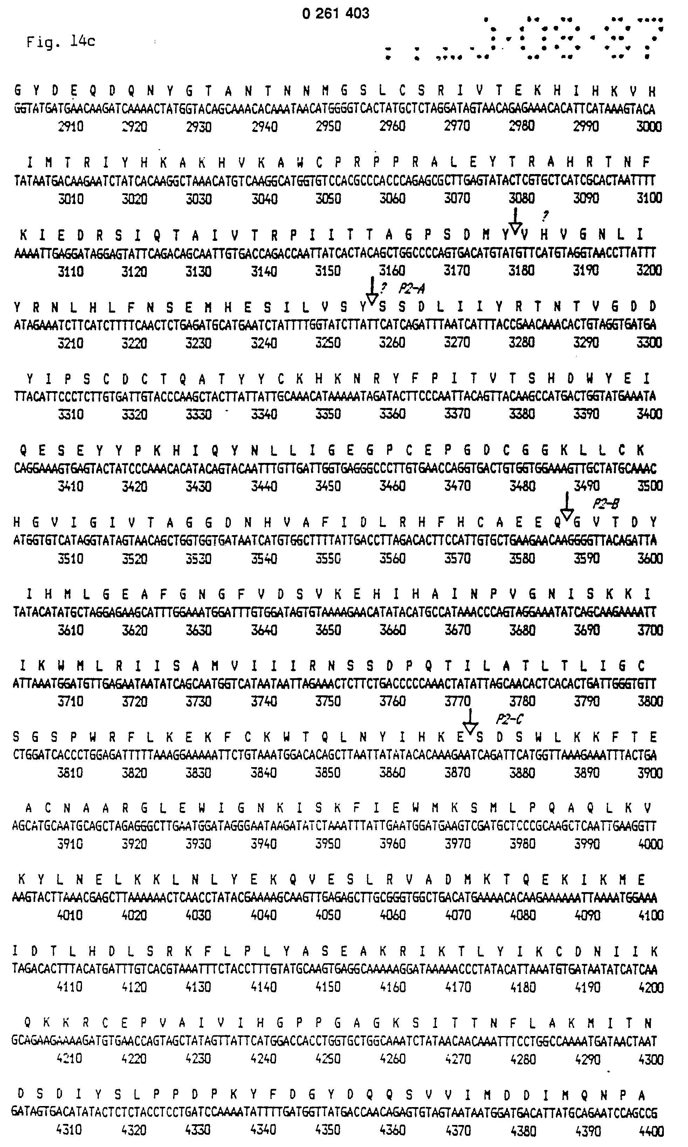

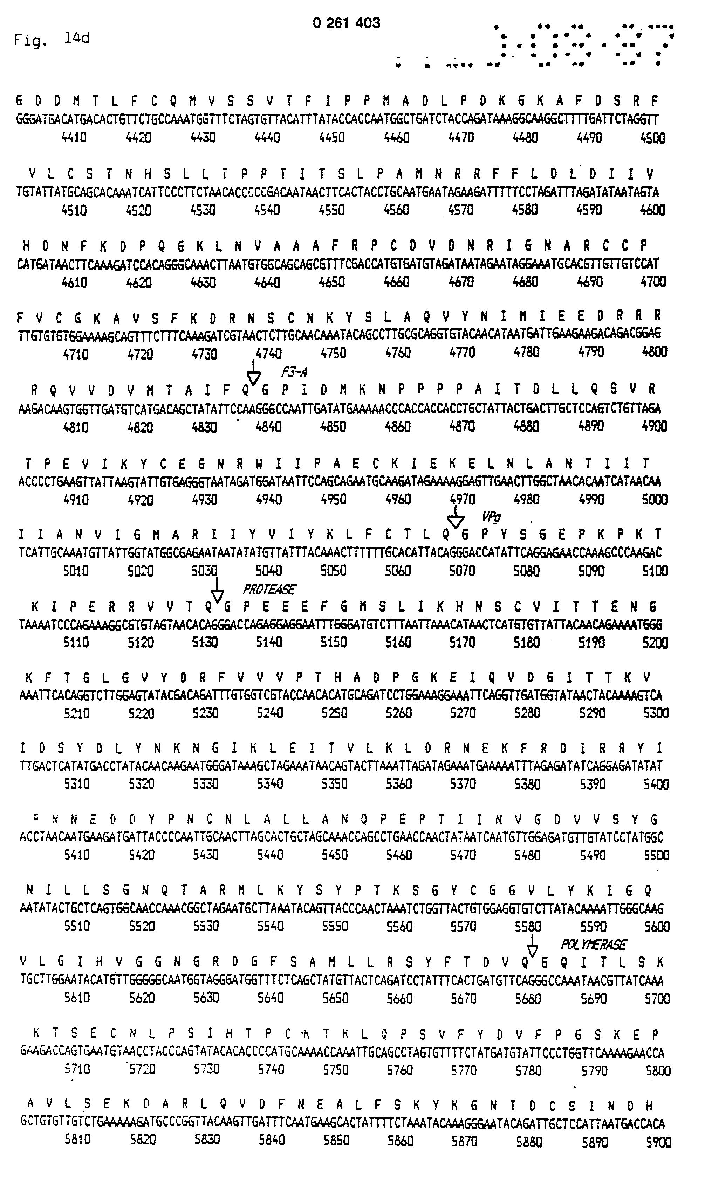

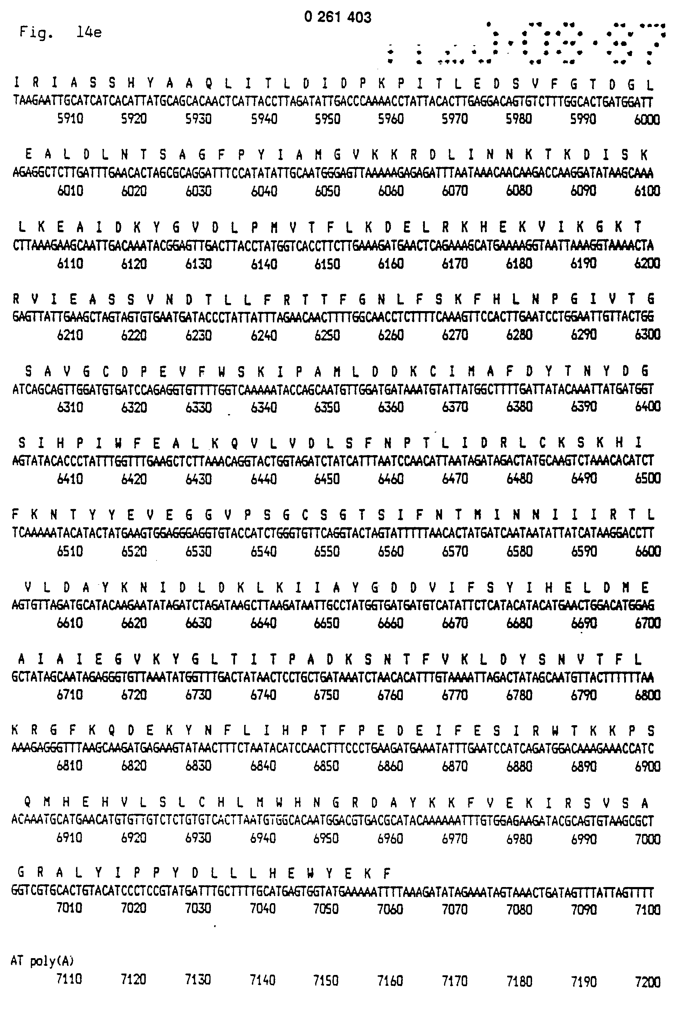

Gegenstand der Erfindung sind ferner Expressionsplasmide, beispielsweise pRH969/1, pRH969/2 und pKK89/2A, die als Insert die DNA von Polypeptiden enthalten, die die biologische Aktivität mindestens eines der viralen Proteine der Rhinovirusstämme HRV2 oder HRV89 aufweisen, insbesondere von Polypeptiden mit der Aminosäuresequenz für VP1, VP2, VP3, VP4, P2A, P2B, P2C, P3A, P3B oder P3C vorzugsweise mit der Aminosäuresequenz gemäß Figuren 4 oder 14 oder Teilen hiervon, von Polypeptiden, die in Teilen oder insgesamt mindestens einem der viralen Proteine des Rhinovirusstammes HRV2 oder HRV89 entsprechen, von Polypeptiden, die mindestens zwei der viralen Proteine in beliebiger Kombination und Reihenfolge verknüpft enthalten und von Polypeptiden, die aus der Aminosäuresequenz gemäß Fig. 4 oder 14 oder aus Teilen hiervon abgeleitet sind und/oder an die zellulären Rezeptoren für die Rhinoviren Stamm HRV2 oder HRV89 binden und/oder diese blockieren, sowie die entsprechenden Polypeptide.

Gegenstand der Erfindung sind ferner Polypeptide, die die biologische Aktivität mindestens eines der viralen Proteine des Rhinovirusstammes HRV89 aufweisen und/oder von einem der beschriebenen DNA-Moleküle codiert werden, insbesondere Polypeptide mit der Aminosäuresequenz für VP1, VP2, VP3, VP4, P2A, P2B, P2C, P3A, P3B oder P3C vorzugsweise mit der Aminosäuresequenz gemäß Figur 4 oder Teilen hiervon. Polypeptide, die in Tielen oder insgesamt mindestens einem der viralen Proteine des Rhinovirusstammes HRV89 entsprechen, Polypeptide, die mindestens zwei der viralen Proteine in beliebiger Kombination und Reihenfolge verknüpft enthalten und Polypeptide, die aus der Aminosäuresequenz gemäß Fig.4 oder aus Teilen hiervon abgeleitet sind und/oder an die zellulären Rezeptoren für die Rhinoviren Stamm HRV89 binden und/oder diese blockieren sind ebenfalls Gegenstand der vorliegenden Erfindung.

Die DNA-Moleküle gemäß der Erfindung werden entweder hergestellt, indem die virale RNA des Rhinovirusstammes HRV89 isoliert, die hierzu komplementäre DNA hergestellt, das virale cDNA/RNA Hybrid in einen geeigneten replizierbaren Vektor eingebaut und ein geeigneter Wirtsorganismus mit diesem Vektor transformiert wird oder sie werden durch enzymatischen Abbau von Teilen oder der vollständigen c-DNA und gegebenenfalls Verknüpfungsreaktionen mit geeigneten Linkern durch chemische DNA-Synthese oder durch Kombination von enzymatischer und chemischer Reaktion hergestellt.

Die erfindungsgemäßen Polypeptide werden erhalten, indem ein geeigneter Wirtsorganismus, vorzugsweise ein Prokaryot, Eukaryot oder eine Säugetierzelle insbesondere E.coli mit den erfindungsmäßen genetischen Informationen, die für ein virales Polypeptid gemäß der Erfindung codieren, transformiert, die Information exprimiert und ein virales Polypeptid gemäß der Erfindung isoliert wird.

Verwendet werden können die erfindungsgemäßen Polypeptide zur therapeutischen Behandlung, beispielsweise zur Stimulierung des Immunsystems, zur Bindung und/oder Blockierung der zellulären Rezeptoren für die Rhinoviren Stamm HRV89 und zur Herstellung von monoklonalen Antikörpern oder polyklonalen Antiseren.

Eingesetzt werden können die erfindungsgemäßen Polypeptide in Form von Arzneimitteln, die neben pharmazeutisch inerten Hilfs-oder Trägerstoffen eine wirksame Menge mindestens eines der erfindungsgemäßen Polypeptide enthalten.

Gegenstand der Erfindung sind weiterhin Hybridzellinien. die durch Immunisierung von Wirtstieren mit HRV2, HRV89 bzw. Teilen ihrer viralen Proteine, Fusionierung der B-Lymphozyten dieser Wirtstiere mit Myelomzellen und in vitro oder in vivo Kultivierung erhältlich sind, die aus diesen Hybriden erhältlichen Antikörper, die spezifisch die Wirkung der erfindungsgemäßen Polypeptide ganz oder teilweise neutralisieren oder spezifisch an eines der besagten Polypeptide binden, monoklonale Antikörper, die neben neutralisierenden Eigenschaften auch die denaturierten Formen ihrer Antigene binden, sowie monoklonale Antikörper zur qualitativen und/oder quantitativen Bestimmung eines der erfindungsgemäßen Polypeptide.

Außerdem ist die Verwendung der monoklonalen Antikörper zur Reinigung eines der erfindungsgemäßen Polypeptide und die Verwendung der monoklonalen Antikörper zu therapeutischen oder diagnostischen Zwecken Gegenstand der vorliegenden Erfindung.

Weiterhin sind auch Test Kits zur Bestimmung der erfindungsgemäßen Polypeptide, die monoklonale Antikörper gemäß der Erfindung enthalten, Gegenstand der vorliegenden Erfindung.

Legende zu den Abbildungen

Fig. 1 Elektrophoretische Trennung der Hüllenproteine von HRV89 auf einem 12,5%igen Polyacrylamid-Gel in Gegenwart von Natriumdodecylsulfat. VP4 ist nicht sichtbar, da es mit der Front aus dem Gel herausgewandert ist.

Fig. 2 Elektrophorese von HRV89-RNA auf einem 1,2%igen Agarose-Gel in Gegenwart von Natriumdodecylsulfat. Rechts sind die Positionen der ribosomalen RNA Marker gezeigt.

Fig. 3 Restriktionskarte des HRV89 Genoms. 21 überlappende Klone, die zur Sequenzierung herangezogen wurden, sind gezeigt. Charakteristische Schnittstellen einiger Restriktionsenzyme sind angeführt. Die Pfeile (A-D) repräsentieren Fragmente, die als Primer für die reverse Transkription verwendet wurden.

Fig. 4 Sequenz des klonierten HRV89 Genoms und die davon abgeleitete Aminosäuresequenz. Spaltstellen im Polyprotein sind durch Pfeile dargestellt. Ausgefüllte Pfeile ( ) zeigen experimentell ermittelte Spaltstellen an, offene Pfeile ( ↓ ) zeigen auf Grund der Homologie mit anderen Picornaviren vorhergesagte Spaltstellen im Polyprotein an.

Fig. 5 Vergleich der Homologie in der Aminosäuresequenz (in %) einzelner Gene zwischen HRV89, HRV2, HRV14 und Poliovirus Typ 1.

Fig. 6 N-terminale Aminosäuresequenzen der Hüllenproteine von HRV89.

Fig. 7 Konstruktionschema für das Expressionsplasmid pRH100

Fig. 8 Tryptophan-Promotor/Operatorregion, synthetische Ribosomenbindungsstelle (RBS) und Startkodon des Expressionsvektors pRH100. Ebenso angeführt ist der Primer (17mer), der zur Sequenzierung des Expressionsplasmides pRH969/1 herangezogen wurde. Kleinbuchstaben und Numerierung kennzeichnen die Sequenz von pBR322; Großbuchstaben stellen die zwischen pBR322-Nukleotidnummer 1 und 29 eingefügte Sequenz dar.

Fig. 9 Sequenzabschnitt der HRV2-cDNA zwischen Nukleotidnummer 700 und 2000. Die kurzen dicken Pfeile kennzeichnen die Sequenz von VP2, die schlanken Pfeile zeigen die wichtigsten Restriktionsstellen dieses Abschnittes an.

Fig. 10 Konstruktionsschema für das Expressionsplasmid pRH969/1 (NCR = "non coding region", untranslatierte Region; trp$^P$/o = Tryptophan-promotor-operator-region).

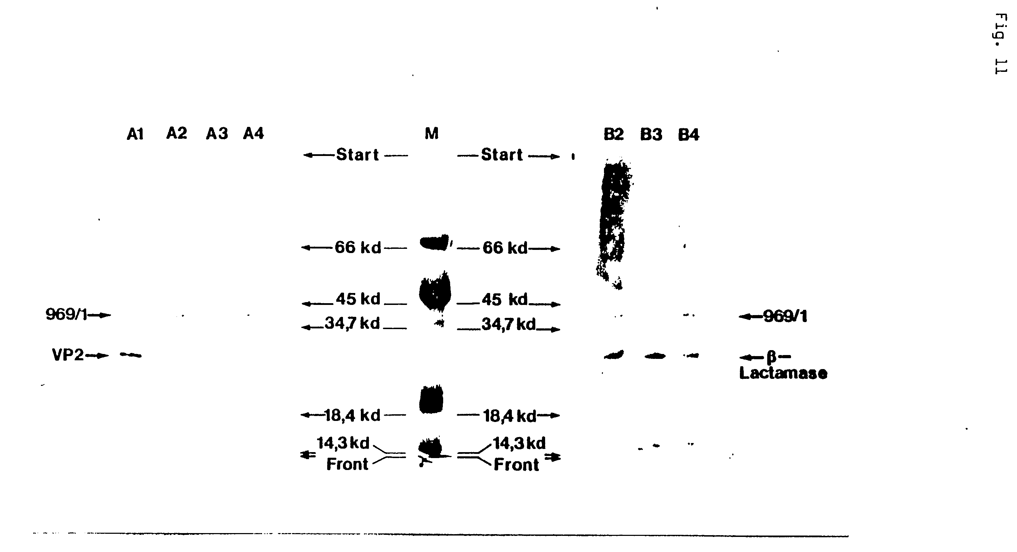

Fig. 11 Ergebnis der Expression von 969/1 im Maxizellsystem CSR603. Die Spuren A1 bis A4 zeigen ein "Western Blot"-Experiment nach Anfärben mit NBT und BCIP. In Spur A1 wurde als Kontrolle das gesamte denaturierte HRV2-Partikel (ca. 50 ng HRV2) mit aufgetrennt. In Spur A2 und A4 wurden die Zellysate der mit dem Plasmid pRH969/1 (aus Klon 307 und 319 stammend) transformierten E. coli Stamm CSR 603-Zellen aufgetrennt. Als Negativkontrolle in Spur A3 wurde das Zellysat von E. coli Stamm CSR 603-Zellen aufgetrennt, welche mit dem Plasmid aus Klon 224 transformiert worden waren (invertierte Orientierung des Inserts 969/1). Spur M zeigt die Coomassie blue - Anfärbung der Markerproteine. Die Spuren B2 - B4 stellen das Autoradiogramm von Spur A2 - A4 dar.

Fig. 12 Insert des Plasmides pRH969/2.

Fig. 13 Bindungsstelle des 8F5 auf dem viralen Hüllenprotein VP2. Die Zahlen 1-261 auf der oberen Linie beziehen sich auf die Aminosäuresequenz des VP2, die unter der unteren Linie auf die DNA-Sequenzen des HRV2. Die DNA und Aminosäuresequenzen im Bereich der Bindungsstelle sind dargestellt mit der exakten Angabe jeder Deletion.

Fig. 14 Sequenz des klonierten HRV2 Genoms und die davon abgeleitete Aminosäuresequenz. Spaltstellen im Polyprotein sind durch Pfeile dargestellt. Ausgefüllte Pfeile zeigen experimentell ermittelte Spaltstellen an, offene Pfeile zeigen auf Grund der Homologie mit anderen Picornaviren vorhergesagte Spaltstellen im Polyprotein an.

Fig. 15 Konstruktionsschema für das Expressionsplasmid pRH 969/2.

Fig. 16 Konstruktionsschema für das Expressionsplasmid pKK89/2A.

Fig. 17 Das durch pKK89/2A exprimierte Polypeptid

Fig. 18 Vergleich der Aminosäuren-und Nukleotidsequenzen aus dem Bereich der Gene für P3A und P3B (VPg) von HRV2 und HRV89. Die auf Grund der Homologie mit anderen Picornaviren vorhergesagte Spaltstelle ist mit einem offenen Pfeil markiert.

Fig. 19 Vergleich der Nukleotidsequenzen in der 5'-nichttranslatierten Region von HRV2 und HRV89.

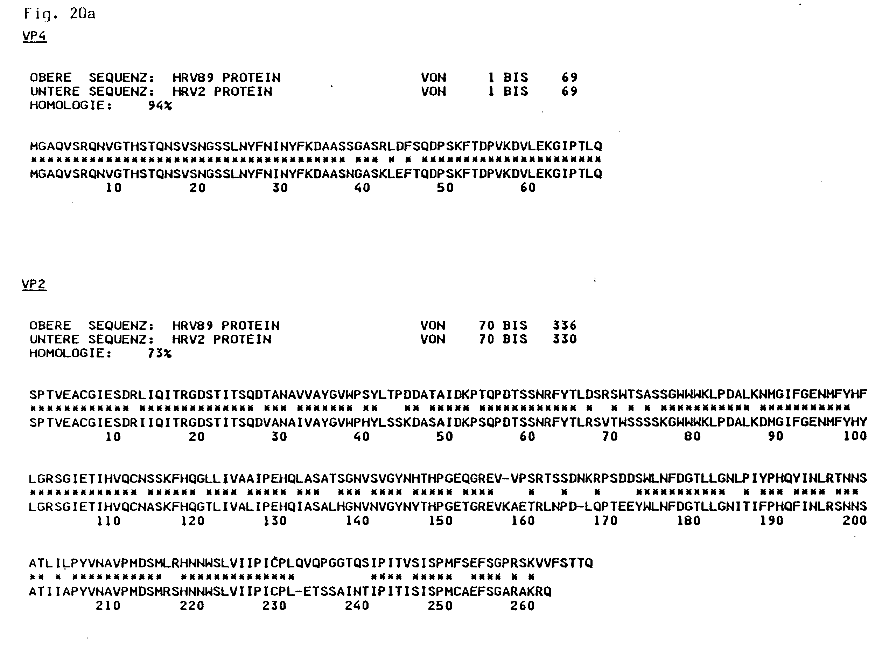

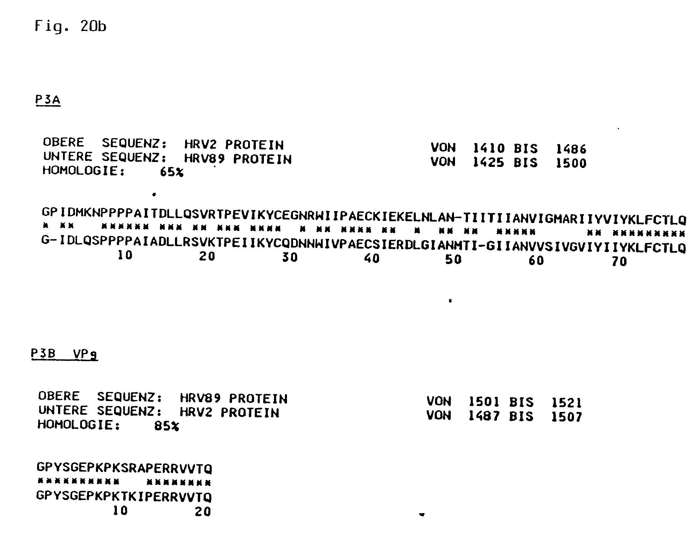

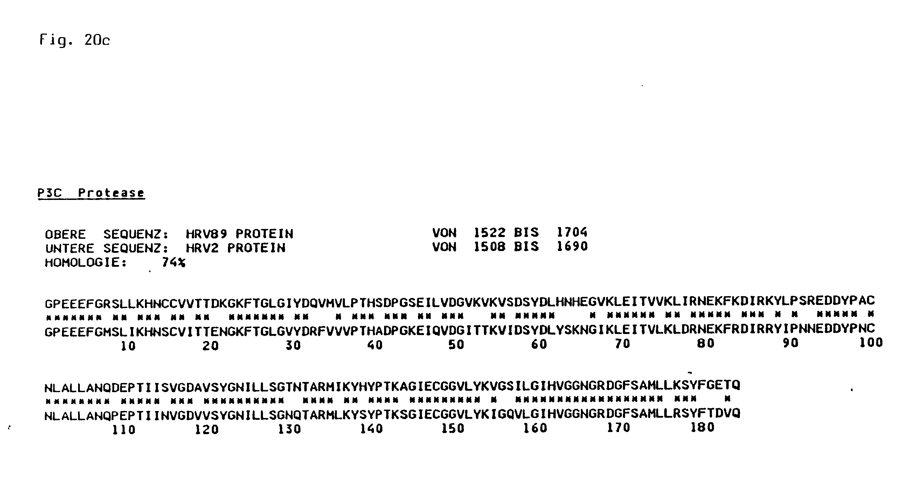

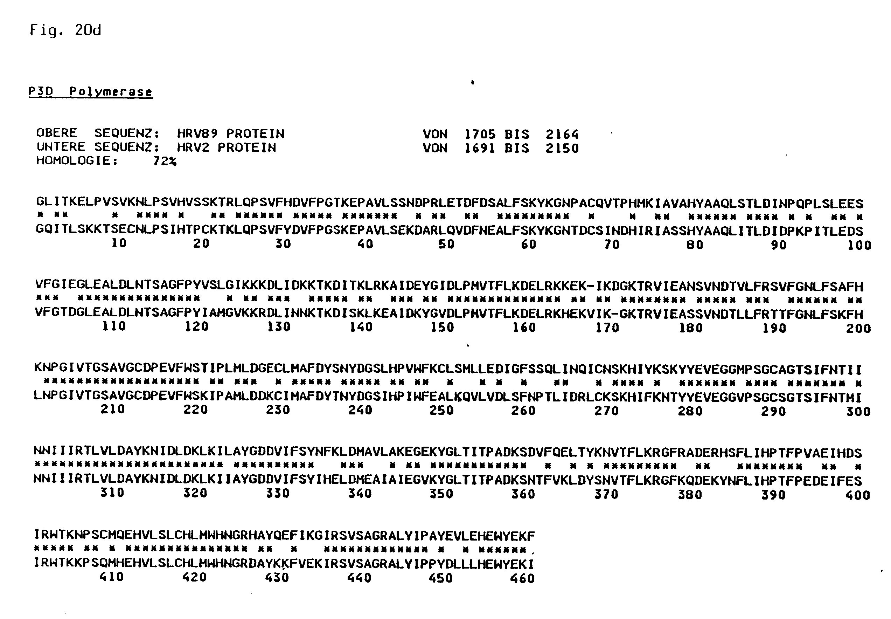

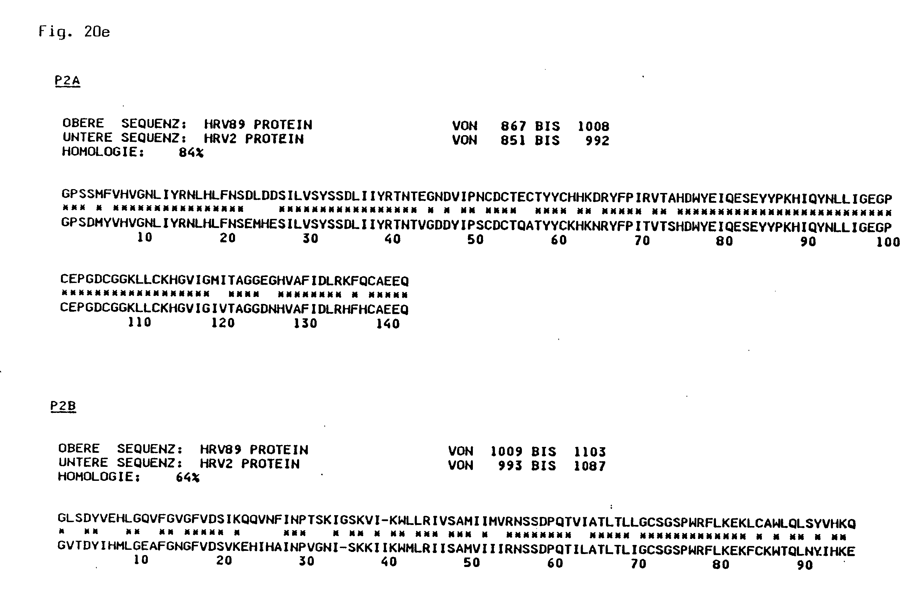

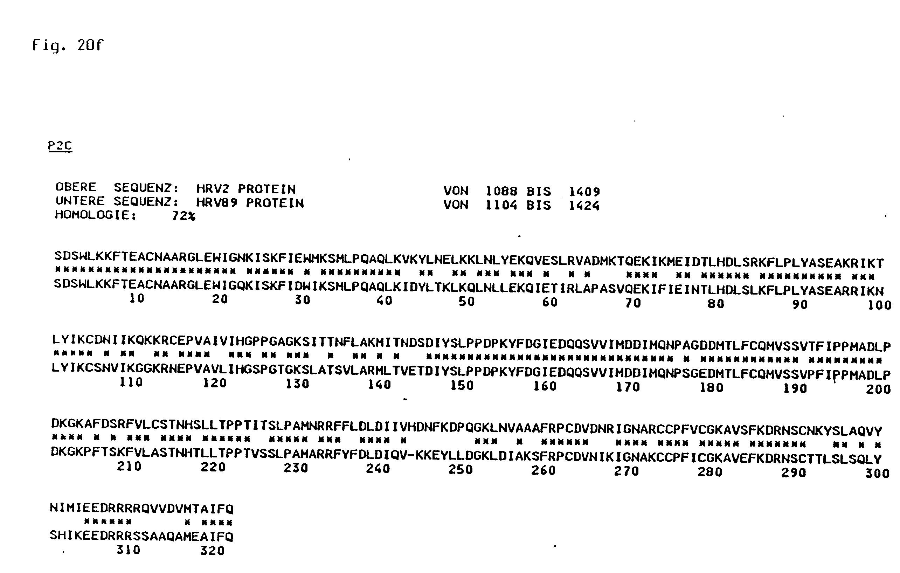

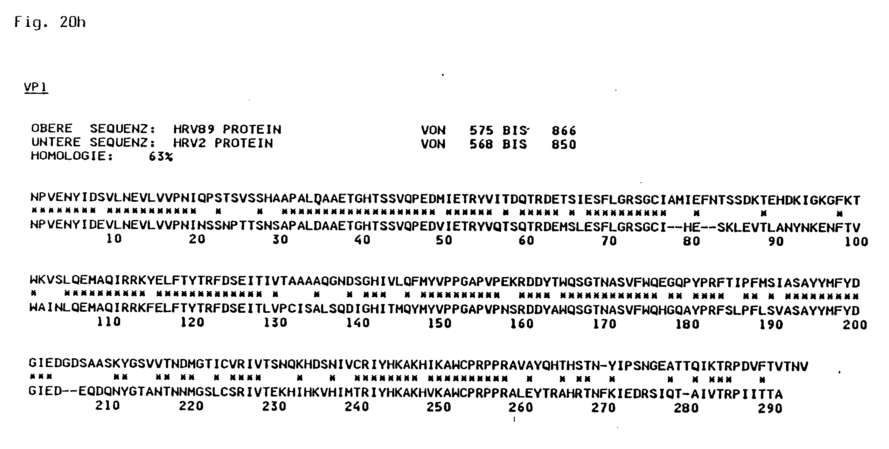

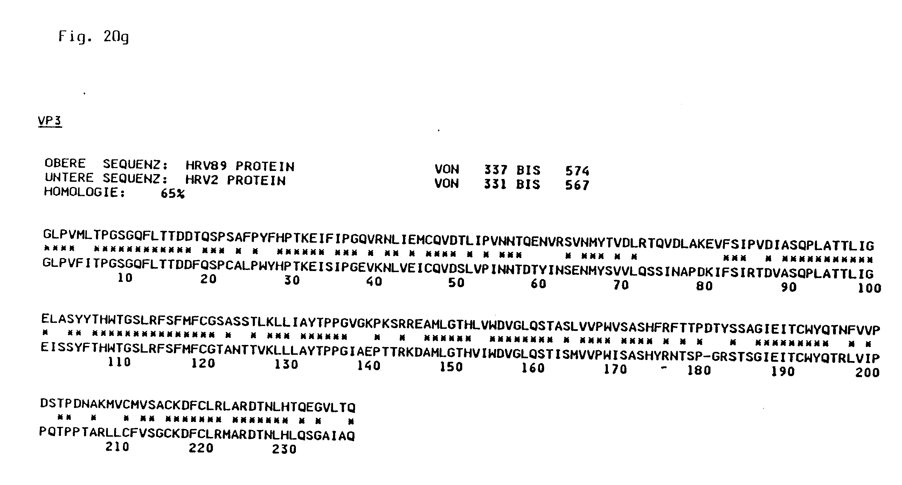

Fig. 20 Vergleich der Aminosäuresequenzen im Bereich der viralen Proteine von HRV2 und HRV89.


## Beispiel 1


### Präparation von HRV89


Hela-Zellen (Stamm HeLa-Ohio, 03-147, Flow Laboratories, England) wurden in Suspension bei 37°C gezüchtet. Das Suspensionsmedium (Thomas, D.C., Conant, R.M. und Hamparian, V.U., 1970, Proc. Soc. Exp. Biol. Med. 133, 62 - 65; Stott, E.J. und Heath, G.F., 1970, J. gen. Virol, 6, 15 - 24) bestand aus einer Joklik-Modifikation von MEM für Suspension (Gibco 072-1300) und 7% Pferdeserum (Seromed 0135). Die Inokulationsdichte betrug 5 - 10 $^\times$ 10$^4$ Zellen/ml, das Volumen 500 ml. Die Suspension wurde bei einer

Zelldichte von 1 $\times$ 10$^6$ Zellen/ml unter sterilen Bedingungen bei 300 g 10 min zentrifugiert. Der Überstand wurde abgesaugt und die Zellen in 100 ml Infektionsmedium (Joklik-Modifikation von MEM für Suspensionskultur mit 2% Pferdeserum und 2 mM MgCl$_2$) resuspendiert. Durch mehrmaliges vorsichtiges Aufsaugen in einer 20 ml Pipette wurden die Zellen homogen im Infektionsmedium verteilt. Danach wurde auf 500 ml aufgefüllt. Anschließend wurde die Zellsuspension auf 34°C gebracht und mit HRV89 (zweimal Plaque - gereinigt) bei einer Multiplizität von 0,1 Viren/Zelle infiziert. Der HRV89 Stamm wurde von der American Type Culture Collection (ATCC VR-1199) bezogen. Der verwendete Stamm wurde durch Antiserum gegen HRV89 (American Type Culture Collection, Cat.No. ATCC VR-1199 AS/GP) neutralisiert.

Als Kontrollserum diente ein Antiserum gegen HRV2 (Cat. No. ATCC VR - 1112 AS/GP), das keine Neutralisation zeigte. Nach 60 Stunden bei 34°C wurde das Virus geerntet.

Virus wurde sowohl aus den Zellen bzw. Zellfragmenten als auch aus dem Medium gewonnen. Zu diesem Zweck wurde das Medium von infizierten Zellen und Zellfragmenten durch eine 10 min Zentrifugation bei 1500 g abgetrennt und abgesaugt. Der Niederschlag wurde bei -70°C eingefroren.

Die Zellniederschläge von 12 l Suspensionskultur wurden vereinigt, in 40 ml TM-Puffer (20mM Tris/HCl, pH 7,5, 2mM MgCl$_2$) resuspendiert, 15 min auf Eis gestellt, anschließend im Dounce-Homogenisator aufgebrochen und das Gemisch 30 min bei 6000 g zentrifugiert. Der Niederschlag wurde anschließend noch einmal in 10 ml TM-Puffer gewaschen. Die beiden Überstände wurden vereinigt und 3 Stunden bei 110 000 g zentrifugiert, um das Virus zu pelletieren. Das Viruspellet wurde dann in 10 ml KTMP-Puffer (50 mM KCl, 50 mM Tris/HCl, pH 7,5,5 mM MgCl$_2$, 2 mM Mercaptoethanol, 1 mM Puromycin, 0,5 mM GTP) aufgenommen und nach Zugabe von 150 µg DNase I (Sigma, Ribonukleasefrei) 1 Stunde auf Eis inkubiert.

Aus dem Infektionsmedium wurde das Virus unter Rühren bei 4°C mit Polyethylenglykol 6000 (PEG 6000; Merck) bei einer Konzentration von 7% und 450 mM NaCl gefällt (Korant, B.D., Lonberg-Holm, K., Noble, J. und Stasny, J.T., 1972, Virology 48, 71 - 86). Nach 4 Stunden in der Kälte wurde das Virus 30 min bei 1500 g abzentrifugiert, der Niederschlag in 10 ml KTMP-Puffer, der 75 µg DNase I enthielt, resuspendiert, das Gemisch 1 Stunde auf Eis inkubiert und anschließend bei -70°C eingefroren.

Die Virussuspensionen, die aus den Zellen und aus dem Medium gewonnen worden waren, wurden vereinigt, 5 min bei 37°C inkubiert, durch Zugabe von 60 ml kaltem TE-Puffer (10 mM Tris/HCl, pH 7,4, 1mM EDTA) abgekühlt und anschließend 5 min lang im Eisbad soniziert.

Danach wurde 30 min bei 6000 g zentrifugiert. Zum Überstand wurden 920 ml TE-Puffer, der 7% PEG 6000 und 450 mM NaCl enthielt, hinzugefügt, 4 Stunden vorsichtig bei 4°C gerührt und das entstandene Präzipitat 30 min bei 6000 g pelletiert. Der Niederschlag wurde abermals in 100 ml TM-Puffer aufgenommen, das Virus wie oben durch Zugabe von PEG 6000 und NaCl gefällt und pelletiert. Der Niederschlag wurde in 40 ml TM-Puffer resuspendiert, die Suspension 30 min bei 6000 g zentrifugiert, und das Virus 3 Stunden bei 110 000 g pelletiert. Das Präzipitat wurde in 1 ml TM-Puffer gelöst, nach Zugabe von 50 µg DNase I 1 Stunde bei 4°C inkubiert und anschließend 1 ml TE-Puffer hinzugefügt. Zur weiteren Reinigung wurde die Virussuspension auf Saccharosegradienten (10 - 30 % w/w in TE-Puffer) 4 Stunden bei 4°C bei 110 000 g zentrifugiert. Aus der Extinktion bei 260 nm wurden die das Virus enthaltenden Fraktionen ermittelt und mit TM-Puffer verdünnt, so daß die Endkonzentration an Saccharose 10% betrug. Danach wurde 8 Stunden bei 85 000 g zentrifugiert. Das Viruspellet wurde in 1 ml TM-PUffer aufgenommen und bei -70°C aufbewahrt. Zur Überprüfung der Reinheit der Viruspräparation wurde eine Elektrophorese auf einem 12,5%igem Polyacrylamid-Gel in Gegenwart von 0,1% Natriumdodecylsulfat durchgeführt (Laemmli, U.K., 1970 Nature (London) 277, 680-685) und die Proteinbanden mit Coomassie-Brillant-Blau angefärbt. Ein typisches Bild des Proteinmusters einer Präparation von HRV89 ist in Fig. 1 gezeigt.

Beispiel 2

Klonierung des cDNA-RNA-Hybrids

RNA-Extraktion aus der HRV89-Präparation

Das Virus wurde in 1 ml NTES-Puffer (100 mM NaCl, 10 mM Tris/HCl, pH 9, 1 mM EDTA, 0,1% Natriumdodecylsulfat) suspendiert und mit Phenol extrahiert. Zur besseren Phasentrennung wurde Chloroform zugesetzt. Zur wässrigen Phase wurden 20 µl 5 M NaCl und 20 µl 3 M Natriumacetat (pH 5,6) zugegeben und die virale RNA mit dem zweifachen Volumen Ethanol gefällt.

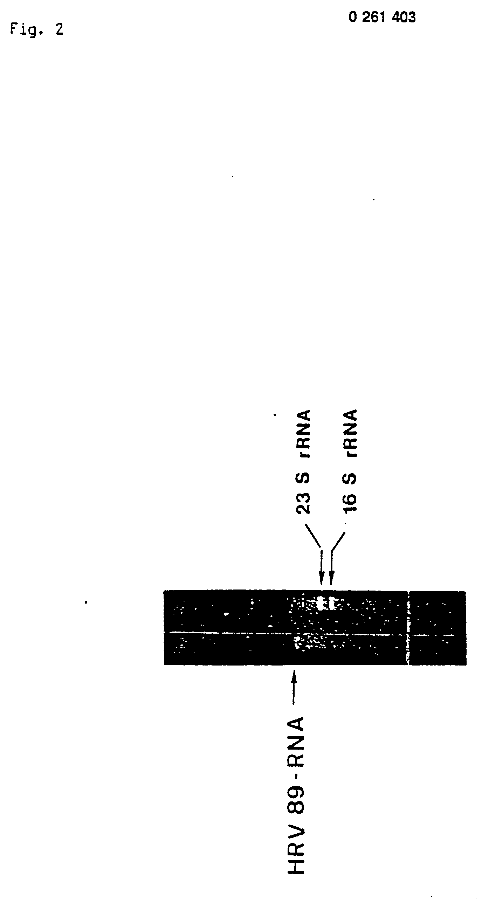

Um das kovalent an das 5′-Ende der viralen RNA gebundene VPg zu entfernen, wurde die RNA anschließend in NTES-Puffer mit 1 mg/ml Proteinase K (Merck) 15 min bei 37°C verdaut. Um Kontaminationen von Ribonuklease zu entfernen, war die Proteinase K Stamm-Lösung (50 mg/ml) vor Gebrauch 15 min bei 37°C vorinkubiert worden. Nach Beendigung des Proteinase K-Verdaus wurde die Lösung, wie oben beschrieben, mit Phenol/Chloroform extrahiert und die RNA durch Zugabe von Ethanol gefällt. Ein kleiner Teil der RNA wurde anschleißend auf einem 2%igen Agarose-Gel in TAE-Puffer (10 mM NaAcetat, 40 mM Tris/Acetat, pH 8,2, 2 mM EDTA) mit 0,1% Natriumdodecylsulfat elektrophoretisch aufgetrennt. Nach Anfärben mit Ethidiumbromid war die Bande der intakten HRV89-RNA sichtbar (Fig. 2). Eine darunter leigende, schwache, diffuse Bande zeigte einen geringfügigen Abbau der RNA an.

## Reverse Transkription der HRV89-RNA mit Oligo-dT als Primer

4 μg HRV89-RNA wurden in 10 μl $H_2O$ gelöst, 5 μl 10 $^\times$ RT-Puffer (1 $^\times$ RT-Puffer = 100 mM KCl, 10 mM $MgCl_2$, 50 mM Tris/HCl, pH 8,3), 5 μg Oligo-dT (12 - 18) (Pharmacia P-L Biochemicals), 10 μCi [$\alpha^{32}$P]-dCTP (3000 Ci/mmol Amersham International, England), 100 U Reverse Transkriptase (Anglian Biotechnology Co, Cambridge) und je 20 nmol von dATP, dGTP, dTTP, dCTP hinzugefügt, und in einem Gesamtvolumen von 50 μl 2 Stunden bei 42°C inkubiert.

Nach Zugabe von 2 μl 250 mM EDTA (pH 8) wurde mit Phenol/Chloroform extrahiert und die wässrige Phase auf eine Biogel P30 (oder Sephadex G-25)-Säule in einer Pasteur-Pipette aufgetragen. Zur Elution diente ein TE-Puffer. Das gebildete cDNA-RNA-Hybrid wurde so von überschüssigem [$\alpha^{32}$P]-dCTP abgetrennt und nach Zugabe von 1/10 Volumenanteilen 3 M Natriumacetat (pH 5,6) und 2 Volumenanteilen Ethanol präzipitiert.

## Homopolymeres Verlängern der HRV89-RNA-cDNA-Hybride ("Tailing")

Das Verlängern der HRV89-RNA-cDNA-Hybride wurde nach der Methode von Roychoudhury und Wu (1980, loc.cit.) durchgeführt. Dazu wurde das HRV89-RNA-cDNA-Hybrid in 50 μl TT-Puffer (200 mM Kaliumkakodylat, 25 mM Tris/HCl, pH 6,9, 2mM $MnCl_2$, 2 mM Dithiothreit, in Gegenwart von 2 nmol [$\alpha^{-32}$P]-dCTP (5Ci/mmol) mit 25 U terminaler Transferase (Pharmacia P-L Biochemicals) 5 min bei 37°C inkubiert (Deng, G.R. und Wu, R., 1983, Methods Enzymol. Vol. 100, Part B, 96-116). Nach Zugabe von 2 μl 0,25 M EDTA (pH 8) wurde mit Phenol/Chloroform extrahiert, das Reaktionsgemisch anschließend an einer Biogel P30-Säule, wie oben beschrieben, chromatographiert und das Oligo-dC tragende RNA-cDNA-Hybrid mit Ethanol präzipitiert.

## Einbau des Oligo-dC-tragenden HRV89-RNA-cDNA-Hybrid in das Plasmid pBR322 ("annealing") und Transformation von Escherichia coli HB 101.

Das Oligo-dC tragende RNA-cDNA-Hybrid wurde in 100 μl NTE-Puffer (100 mM NaCl, 10 mM Tris/HCl, pH 7,6, 1 mM EDTA) mit 0,3 pmol pBR322-Plasmid (das mit PstI geschnitten war und an das Oligo-dG-Reste anpolymerisiert waren; Bethesda Research Laboratories) versetzt, erst 5 min bei 65°C, dann 2 Stunden bei 42°C erhitzt, über Nacht langsam auf Raumtemperatur abgekühlt und bei 4°C aufbewahrt.

Für die Zelltransformation wurde der Stamm HB 101 (DSM 1607) in 50 ml LB-Medium (10 g Trypton, 5 g Hefeextrakt, 10 g NaCl in 1 l) gezüchtet (Mandel, M. und Higa, A., 1979, J.Mol.Biol. 53, 159 - 162). Um für die Transformation geeignete Zellen ("kompetente Zellen") zu erhalten, wurden die Bakterien pelletiert und in 25 ml TR-Puffer (150 mM KCl, 50 mM $CaCl_2$, 1 mM Tris/HCl, pH 7, 3 mM $MgCl_2$) aufgenommen, 30 min auf Eis gestellt, abermals zentrifugiert, erneut in 2 ml TR-Puffer resuspendiert und 1 Stunde auf Eis gestellt. Zu 200 μl der Zellsuspension wurden 100 μl der Mischung, die pBR322 mit dem eingefügten HRV89-RNA-cDNA-Hybrid enthielt, und 5 μl 1 M $CaCl_2$ zugegeben, 1 Stunde bei 0°C und danach 90 sek. bei 42°C inkubiert. Dann wurden 2 ml kaltes LB-Medium zugesetzt und 15 min. bei 37°C inkubiert. Die Zellsuspension wurde auf LB-Agar-Platten (1,5% Agar in LB-Medium), die 10 μg/ml Tetracyclin (Sigma) enthielten, aufgebracht und über Nacht inkubiert.

Tetracyclin-resistente Klone wurden anschließend auf Ampicillin-Agar-Platten (100 μg Ampicillin/ml; Sigma) auf Ampicillin-Sensitivität geprüft.

## Charakterisierung und Isolierung der rekombinanten DNA-Moleküle

Klone von Tetracyclin-resistenten, Ampicillin-sensitiven Bakterien wurden in 6 ml LB-Medium (10 μg Tetracyclin/ml) über Nacht hochgezüchtet, Plasmid DNA nach der "Mini-Plasmid-Präparationstechnik" isoliert (Birnboim, H.C. und Doly, J., 1979, Nucleic Acids Res. 7, 1195 - 1204) und die Größe der rekombinanten DNA durch Verdau mit dem Restriktionsenzym PstI ermittelt. Die Plasmid-DNA wurde in 25 μl RE-Puffer (6 mM MgCl₂, 10 mM Tris/HCl, pH 7,5, 6 mM Mercaptoethanol) mit 50 mM NaCl in Gegenwart von 2 U des Restriktionsenzyms PstI (Bethesda Research Laboratories) und 5 μg Ribonuklease A 2 Stunden bei 37°C inkubiert. Anschließend wurden die Proben auf einem 1,4%igen Agarose-Gel elektrophoretisch aufgetrennt. Durch Anfärben mit Ethidiumbromid und Vergleich mit Lambda-HindIII-Marker DNA konnten die Größen der Inserte bestimmt werden. Diese lagen zwischen 300 und 3300 Basenpaaren.

Um größere Mengen der DNA-Inserte zu isolieren, wurden Plasmide wie oben beschrieben aus 200 ml Kulturen von Tetracyclin-resistenten, Ampicillin-sensitiven Bakterienklonen gewonnen und mit PstI verdaut. Die rekombinanten DNA-Fragmente wurden wie oben beschrieben, über ein präparatives Agarose-Gel aufgetrennt, die Banden herausgeschnitten, die DNA in 0,5 ˣ TBE-Puffer (1 ˣ TBE-Puffer = 100 mM Tris/Borat, pH 8,3, 2 mM EDTA) elektroeluiert und mit Ethanol ausgefällt.

## Subklonierung in Escherichia coli Stamm JM 101-Zellen mit Hilfe des pUC9-Vektors

Das Plasmid pUC9 (Vieisa, J. and Messing, J.G. 1982, Gene 19, 259 - 268) enthält ein Gen für die Ampicillin-resistenz, eine Region für den Start der Replikation, die aus dem Plasmid pBR322 stammen, und einen Teil des Lac-Z-Gens von E. coli Ein kleiner DNA-Abschnitt, der eine Reihe von Restriktionsstellen enthält, befindet sich in dieser Lac-Z-Region, so daß die Klonierung von DNA in eine dieser Schnittstellen die Lac-Z-Gen-Region unterbricht. Kolonien, die DNA-Inserte enthalten, erscheinen daher auf X-Gal (X-Gal = 5-Bromo-4-chloro-3-indolyl-β-D-galactosid, Bethesda Research Laboratories)-Indikatorplatten weiß, diejenigen ohne Inserte erscheinen blau (Rüther, U., 1980, Mol. Gen. Genet. 178, 475 - 478). Um DNA-Inserte in pUC9 zu subklonieren, wurden rekombinante pBR322-Klone (ca. 7 μg) mit PstI verdaut und nach Auftrennung am Agarose-Gel (1,2% - 1,4%) die DNA-Inserte von der Vektor-DNA abgetrennt. Die DNA wurde aus dem Gel, wie oben beschrieben, durch Elektroelution und Ethanolfällung wiedergewonnen. Das isolierte DNA-Insert wurde in 20 μl RE-Puffer mit 0,4 μg pUC9-Vektor (mit PstI geschnitten und mit bakterieller alkalischer Phosphatase vorbehandelt), in Gegenwart von 1 mM ATP und 3 U T₄-Ligase (Bethesda Research Laboratories) 1 Stunde bei 15°C inkubiert und bei 4°C aufbewahrt (Vieisa, J. und Messing, J.G., 1982, loc.cit). Gleichzeitig wurden E.coli Stamm JM101-Zellen, die für die Transformation kompetent waren, in der oben beschriebenen Weise hergestellt. 200 μl der kompetenten Zellsuspension wurden mit 20 μl Gemisch der pUC9-Ligase-Reaktion vermischt und 1 Stunde bei 0°C inkubiert.

Nach einem Hitzeschock (90 sek., 42°c) wurden die Zellen mit 10μl 200mM Isopropylthiogalactosid (Sigma), 50 μl einer Lösung von 20 mg X-Gal in 1 ml Dimethylformamid und 1 ml LB-Medium versetzt und 1 Stunde bei 37°C inkubiert. Je 200 μl dieser Zellsuspension wurden anschließend auf je eine Ampicillin-LB-Agarplatte (100 μg/ml) transferiert und über Nacht bei 37°C im Brutschrank inkubiert. Positive Transformanten wurden als weiße Kolonien identifiziert, die mit Hilfe der "Mini-Plasmid-Präparationstechnik" auf DNA-Inserte hin untersucht wurden.

## Präparation von pUC9-Plasmiden mit Inserten von rekombinanter DNA

Die gewonnenen Subklone von E. coli JM 101, die mit pUC9 transformiert waren und DNA-Inserte enthielten, wurden in 200 ml LB-Medium (mit 100 μg Ampicillin/ml) hochgezüchtet und die Plasmid-DNA isoliert ( Birnboim, H.C. und Doly, J., 1979 loc.cit.) Die Plasmid-DNA wurde anschließend in 100 μl TE-Puffer gelöst und die Lösung über eine Sephacryl-1000-Säule (1 ˣ 20 cm) mit TE-Puffer chromatographiert. Die Fraktionen, die das gereinigte Plasmid enthielten, wurden an Hand der Extinktion lokalisiert, vereinigt und lyophilisiert. Das Plasmid wurde in 500 μl TE-Puffer aufgenommen, 5 min bei 65° inkubiert und nochmals nit Phenol/Chloroform extrahiert und mit Ethanol gefällt.

Gewinnung von Primer-Fragmenten aus den Plasmiden pHRV89-100, pHRV89-136, pHRV89-193 und auf synthethischem Wege

Klone 100, 136 und 193, die entsprechende DNA-Inserte enthielten, wurden in pUC9 subkloniert, in 200 ml LB-Medium (mit 100 μg Ampicillin/ml) hochgezüchtet und die Plasmide, wie oben beschrieben, isoliert. Das Primer-Fragment (54 Nukleotide) aus Klon 100 wurde durch Verdau mit den Restriktionsenzymen NcoI und PvuII erhalten (das Primer-Fragment ist in Fig. 3 als Fragment A bezeichnet). Zu diesem Zweck wurden 200 μg gereinigtes Plasmid aus Klon 100 in 200 μl RE-Puffer in Gegenwart von 50 mM NaCl mit 30 U NcoI (New England Biolabs.) 15 Stunden bei 37°C inkubiert. Nach Beendigung der Reaktion wurde mit Ethanol gefällt und das geschnittene Plasmid in 100 μl RE-Puffer in Gegenwart von 50 mM NaCl mit 20 U PvuII (New England Biolabs) 15 Stunden bei 37°C inkubiert. Der Verlauf des Verdaues mit Restriktionsenzymen wurde durch Elektrophorese auf 1,4%igen Agarose-Gelen überprüft. Das NcoI/PvuII-Fragment wurde in 100 μl OG-Lösung (1% Ficoll, 1 mM EDTA, 0,01% Orange G) aufgenommen und die DNA auf einem präparativen 15%igem Polyacrylamid-Gel (Acrylamid/ Bisacrylamid = 19 : 1, Geldicke 1,2 mm) in 1 × TBE-Puffer aufgetrennt. Die Bande, die dem 54 Basen-NcoI/PvuII-Bruchstück entsprach, wurde nach Ethidiumbromid-Anfärbung herausgeschnitten, das Fragment in 0,5 × TBE-Puffer elektroeluiert und die DNA mit Ethanol präzipitiert. Das DNA-Fragment wurde anschließend in 50 μl 100 mM Tris/HCl, pH 8, mit 200 U bakterieller alkalischer Phosphatase (Bethesda Research Laboratories) 1 Stunde bei 65°C inkubiert. Nach der Reaktion wurden 2,5 μl 0,5 M EDTA, pH 8, zugesetzt, die wässrige Phase 2 × mit Phenol/Chloroform extrahiert und die DNA durch Ethanolfällung präzipitiert. Die DNA wurde in Wasser gelöst und in 50 μl K-Puffer (10 mM MgCl$_2$, 50 mM Tris/HCl, pH 8, 5 mM Dithioerythrit) mit 20 μCi γ-[$^{32}$P]-ATP (spez. Aktivität 5000 Ci/mmol; Amersham International) und 5 U Polynukleotidkinase (Pharmacia-PL Laboratories) bei 37°C 30 min inkubiert. Das mit $^{32}$P markierte Fragment wurde anschließend ausgefällt und in 30 μl 30%igem Dimethylsulfoxid, das 1 mM EDTA und 0,01 % Xylencyanol-Bromphenolblau enthielt, aufgenommen. Diese Lösung wurde 2 min bei 90°C inkubiert, in Eis abgekühlt, auf ein 15%iges Polyacrylamid-Gel (Acrylamid/Bisacrylamid = 59 : 1, Geldicke 1,2 mm) in TBE-Puffer aufgetragen und die beiden DNA-Stränge elektrophoretisch voneinander getrennt (15 Stunden bei 200 Volt). An Hand des Autoradiogramms wurden die beiden Stränge lokalisiert, herausgeschnitten und elektroeluiert. Der mit HRV89-RNA hybridisierende Strang wurde durch ein "dot-blot"-Experiment ermittelt. Dazu wurden zwei 2 × 2 cm große Nitrozellulosestreifen (Schleicher & Schüll, BA 85, 0,45 μm) mit H$_2$O befeuchtet, einmal mit 20 × SSC (1 × SSC = 150 mM NaCl, 15 mM Natriumcitrat, pH 7,4) gewaschen und an der Luft getrocknet. Auf jedem Streifen wurde punktförmig ca. 1 μg HRV89-RNA aufgetragen, getrocknet und bei 80°C 2 Stunden inkubiert. Anschließend wurden die Streifen mit 2 × SSC befeuchtet und in einer Plastikfolie gemeinsam in 1 ml H-Puffer (400 mM NaCl, 40 mM PIPES, pH 6,4, 1 mM EDTA, 80% Formamid) mit 4 μg denaturierter Lachsspermien-DNA (Sigma; 2 min bei 100°C inkubiert und auf 0°C abgekühlt) 1 Stunde bei 42°C inkubiert. Danach wurden die beiden Nitrozellulosestreifen mit je 0,5 ml H-Puffer, 4 μg denaturierter Lachsspermien-DNA und je einem Aliquot der isolierten Stränge (20 000 cpm) getrennt in Plastikfolien eingeschweißt und über Nacht bei 42°C hybridisiert. Nach dieser Inkubation wurden die Filter 2 mal mit 2 × SSC 10 min lang bei 50°C und 2 mal mit 0,1 × SSC, 0,1% Natriumdodecylsulfat 30 min bei 50°C gewaschen und die Radioaktivität bestimmt.

Die Primer-Fragmente aus HRV89-136 und HRV89-193 wurden in gleicher Weise isoliert. Das Fragment aus pHRV89-136 (in Fig. 3 als Fragment B bezeichnet) liegt zwischen einer DraI - und einer HindIII-Restriktionsstelle (122 Nukleotide) und wurde durch Verdau mit diesen Restriktionsenzymen erhalten. Fragment C wurde aus pHRV89-193 isoliert, ist 139 Nukleotide lang und liegt zwischen zwei RsaI-Restriktionsstellen (Fig. 3). Strang-Trennung und Hybridisierung wurden, wie oben beschrieben, durchgeführt. Primer D wurde chemisch nach etablierten Methoden auf einem Applied Biosystems Gerät synthetisiert.

Reverse Transkription von HRV89-RNA mit Hilfe von Primerfragmenten

5 pmol der zu HRV89-RNA komplementären Einzelstrang-DNA die, wie oben beschrieben, aus den Klonen 100 (Fragment A), 136 (Fragment B) und 193 (Fragment C) isoliert oder chemisch synthetisiert waren, wurden jeweils gemeinsam mit 0,25 pmol der HRV89-RNA aus einer wässrigen Lösung mit Ethanol präzipitiert. Der Niederschlag wurde in 20 μl H-Puffer aufgenommen, in eine Kapillare eingeschweißt, 10 min bei 72°C inkubiert, auf 50°C transferiert, langsam auf 35°C abgekühlt und anschließend auf Eis gestellt. Die Lösung wurde dann in 100 μl RT-Puffer in Gegenwart von 140 U reverser Transkriptase (Anglian Biotechnology Co, Cambridge), 8 U Ribonuklease-Inhibitor (RNasin, Bethesda Research Laboratories), je 0,2 mM dATP, dCTP, dGTP und dTTP, 30 μCi [α-32P]-dCTP und 5 mM Dithioerythrit 2 Stunden bei 42°C inkubiert. Das entstandene reverse Transkript wurde, wie oben beschrieben, aufgearbeitet.

### Nachweis von HRV89-Sequenzen in rekombinanter DNA und Restriktionskartierung.

Plasmid-DNA der Rekombinanten wurden von 3 ml Kulturen isoliert (Birnboim, H.C. und Doly, J. 1979, loc.cit). Die DNA wurde dann mit dem Restriktionsenzym PstI inkubiert und die Proben durch Elektrophorese auf 1,4%igem Agarose-Gel analysiert. Die Gele wurden mit Ethidiumbromid angefärbt. Anschließend wurde die DNA vom Gel auf Nitrozellulosefilter transferiert (Southern, E.M., 1975, J. Mol, Biol. 98, 503 - 517) und durch eine 2-stündige Inkubation bei 80°C auf der Nitrozellulose fixiert. Die Filter wurden in 50% Formamid, 1 × Denhardts-Lösung (Denhardt, D.T., 1966, Biochem.Biophys.Res. Comm., 23, 641 - 646), 900 mM NaCl, 50 mM Natriumphosphat, pH 7,4, 5 mM EDTA mit 80 μg/ml denaturierter Lachsspermien-DNA 2 Stunden bei 42°C in einer Plastikfolie präinkubiert. Radioaktive HRV89-cDNA wurde, wie oben beschrieben, hergestellt, nur enthielt der Reaktionsansatz 50 μCi [α-32P]dCTP. Das cDNA-HRV89--RNA-Hybrid wurde bei 100°C 90 sek. denaturiert. Zur Hybridisierung wurden die Filter mit radioaktiver HRV89-cDNA, wie oben beschrieben, bei 42°C 18 Stunden inkubiert, anschließend 2 mal in 2 × SSC und 2 mal in 0,1% Natriumdodecylsulfat 30 min bei 50°C gewaschen, an der Luft getrocknet und bei -70°C exponiert (Kodak XAR-5, mit Verstärkerfolie 18 - 40 Stunden). Das Auftreten einer radioaktiven Bande zeigte das Vorhandensein von rekombinanter DNA an, die zur HRV89-RNA komplementär war.

Zur Kartierung wurden die DNA-Inserte unter Verwendung von Restriktionsenzymen (New England Biolabs und Bethesda Research Laboratories) verdaut, wobei die von den Herstellern angegebenen Inkubationsbedingungen verwendet wurden. Das Ergebnis der Restriktionskartierung ist in Fig. 3 gezeigt. Die Plasmide pHRV89-80 und pHRV89-82 enthielten-unmittelbar anschließend an das Oligo-C, das auf die Kettenverlängerung durch die terminale Transferase-Reaktion zurückzuführen ist - eine längere Sequenz von A-Resten, die einen Teil des 3'-terminalen Poly-A der HRV89-RNA darstellen. Die weiteren Plasmide wurden relativ zu pHRV89-80 und pHRV89-82 unter Zuhilfenahme charakteristischer Spaltstellen einzelner Restriktionsenzyme angeordnet. DNA-Inserte, die kleiner als 500 Basenpaare waren, wurden nicht durch Restriktionsenzymkartierung eingeordnet, sondern wurden sofort in pUC9 subkloniert und sequenziert (siehe Beispiel 3). Die Identifizierung der restlichen Klone erhält man durch Koloniehybridisierung unter Verwendung bereits kartierter DNA-Inserte als "Nick-Translations"-Proben nach der Methode von Grunstein und Hogness (Grunstein, M. und Hogness, D.S., 1975, Proc. Natl. Acad. Sci USA 72, 3961 - 3965). 32P-markierte DNA-Proben erhält man mit Hilfe eines "Nick-Translation-Kit" der Firma Amersham International (England; Amersham Kit No. 5000) nach Vorschrift des Herstellers mit [α-32P]-dCTP (3000 Ci/mmol). Die markierte DNA wurde über eine Biogel-P-30-Säule in einer Pasteurpipette in TE-Puffer aufgetrennt. Fraktionen, die dem Exklusionsvolumen entsprachen, wurden vereinigt, 2 min bei 100°C erhitzt und schnell in Eiswasser gestellt. Die Hybridisierung wurde wie oben beschrieben durchgeführt. Von Kolonien, die ein positives Hybridisierungssignal zeigten, wurden 50 ml Kulturen (über Nacht in LB-Medium mit 10 μg Tetracyclin/ml) hergestellt und aus der Plasmid-DNA die DNA-Inserte mit PstI isoliert. Diese wurden anschließend durch Verdau mit verschiedenen Restriktionsenzymen und durch Sequenzierung charakterisiert. In dieser Weise wurden Klone erhalten, die das Genom von HRV89 repräsentieren.

### Beispiel 3

### DNA-Sequenzierung

cDNA-Klone von HRV89 wurden nach einer Modifikation der Methode von Maxam und Gilbert (Maxam, A. und Gilbert, W., 1980, Methods Enzymol. 65, 499 - 560) bzw. nach der M 13-Kettenabbruchmethode nach Sanger et al. (Sanger, F., Nicklen, S. und Coulsen, A.R., 1977, Proc. Natl. Acad. Sci USA 74, 5463 - 5467) sequenziert.

Zur Sequenzierung nach Maxam und Gilbert wurden die DNA-Inserte, wie oben beschrieben, in pUC-9 subkloniert und damit kompetente E.coli JM 101-Zellen transformiert. Positive Transformanten wurden als weiße Kolonien isoliert und im LB-Medium (mit 100 μg/ml Ampicillin) hochgezüchtet. 10 - 20 μg DNA wurden in 100 μl über Nacht unter Standardreaktionsbedingungen mit Restriktionsenzymen verdaut, die eine Spaltstelle im Plasmid z.B. in der Polylinker-Region von pUC9 besitzen (z.B. BamHI, EcoRI, AccI,

HindIII). Das restringierte Fragment wurde anschließend dephosphoryliert. Zum Restriktionsverdau wurden 5 μl 2 M Tris/HCl, pH 8, und 100 U bakterieller, alkalischer Phosphatase (Bethesda Research Laboratories) zugesetzt und 3 Stunden bei 65°C inkubiert. Nach Zugabe von EDTA auf 20 mM wurde zweimal mit Phenol/Chloroform extrahiert und die DNA durch Ethanol gefällt. Die DNA wurde anschließend in 50 μl 50 mM Tris/HCl, pH8, 10 mM $MgCl_2$, 5 mM Dithioerythrit. mit 25 μCi [$\gamma$-$^{32}$P]-ATP (5000 Ci/mmol, Amersham International) und 4 U T$_4$-Polynukleotidkinase (Pharmacia-P.L. Biochemicals) 30 min bei 37°C inkubiert und die markierte DNA mit Ethanol präzipitiert. Unter Zuhilfenahme eines weiteren Restriktionsenzyms, das in der Polylinker-Region von pUC9 spaltet, wurde rekombinante DNA, die in einem Strang mit $^{32}$P markiert war, durch Elektrophorese im Agarose-Gel (1,2 - 1,4%) mit nachfolgender Elektroelution und Ethanolfällung wie oben beschrieben, erhalten.

## DNA-Sequenzierung nach einer Modifikation der Methode von Maxam und Gilbert

Die Sequenzierungsreaktion wurden mit folgenden Modifikationen nach Maxam und Gilbert (Maxam, A. und Gilbert, W., 1980, loc.cit.) durchgeführt:
-Es wurde keine Träger-DNA zugesetzt.

-Die DNA-Lösung wurde in Aliquote aufgeteilt: Guanin (G)-spezifische Reaktion 7,5 μl, Guanin und Adenin (G/A)-spezifische Reaktion 10 μl, Cytosin und Thymin (C/T)-spezifische Reaktion 10 μl, Cytosin (C)-spezifische Reaktion 6 μl.

-Dem (G/A)-Reaktionsansatz wurden 25 μl 96% Ameisensäure zugesetzt und die Mischung 4,25 min bei 19°C inkubiert. Um die Reaktion abzustoppen, wurden 200 μl Hydrazin-Stop-Lösung und 750 μl 96% Ethanol zugesetzt. Die (G/A)-Reaktionen wurden dann gleich wie alle 3 anderen Reaktionen behandelt.

-Anstelle von Hydrazin wurde Hydraziniumhydroxid (Merck) bei den (C/T)-und C-Reaktionen verwendet. Die Reaktionszeiten betrugen 7,5 min.

-Die Piperidinreaktionen wurden 30 min bei 95°C inkubiert. Nach Lyophilisierung wurden die Fragmente in 3 - 20 μl Puffer (80% entionisiertes Formamid, 1 $^\times$ TBE, 0,05% Bromphenolblau und 0,05% Xylencyanol) 90 sec auf 95°C erhitzt und schnell auf 0°C gekühlt.

Zur Sequenzierung wurden 6%ige Polyacrylamid-Gele (40 cm $^\times$ 20 cm $^\times$ 0,4 mm) mit 8 M Harnstoff und 1 $^\times$ TBE verwendet, die vor dem Auftragen der Proben 1 Stunde bei 50 Watt einer Elektrophorese unterworfen wurden. Zwischen 1 μl und 3μl von jedem Reaktionsansatz wurden auf das Gel aufgetragen. Üblicherweise wurden zwei zeitlich verschobene Gelbeladungen durchgeführt. Der erste Gellauf eines Reaktionsansatzes dauerte solange bis der Bromphenolblau-Marker, der zweite bis der Xylencyanol-Marker das Gelende erreichte. Die Gele wurden anschließend 20 min in 10% Essigsäure und 10% Methanol (ca. 2 Liter) fixiert, auf 3 MM-Filterpapier transferiert und bei 80°C auf einem Geltrockner getrocknet. Dann wurden die Gele ohne Verstärkerfolie bei -70°C unter Verwendung eines XAR-5 Films (Kodak) exponiert (ca. 18 bis 36 Stunden).

## M 13 Sequenzierung

DNA-Inserte mit einer Größe von mehr als 1000 bp wurden nach der "shotgun"-Methode sequenziert (Deininger, P.L. 1983, Anal. Biochem. 129, 216 - 223). Hierfür wurden rekombinante Klone mit geeigneten Restriktionsenzymen verdaut, die DNA-Inserte wie oben beschrieben, durch Auftrennung am präparativen Agarosegel (1.2 - 1.4%) mit anschließender Elektroelution und Ethanolfällung isoliert.

In 20 μl RE-Puffer wurden die DNA-Inserte in Gegenwart von 1mM ATP und 3U T4-DNA-Ligase (New England Biolabs) 15 Stunden bei 14° inkubiert. Die zirkularisierte DNA wurde mit TE-Puffer (10 mM Tris/HCl, pH 7,5, 1 mM ETDA) auf ein Endvolumen von 500 μl verdünnt, unter Eiskühlung soniziert (4 mal 5 sek. maximale Energie; MSE ultrasonic power unit) und mit Ethanol gefällt.

Die Enden der erhaltenen DNA-Fragmente wurden in 20 µl RE-Puffer, der je 50 µM dATP, dCTP, dGTP und dTTP war und 2 U Klenow-Fragment (Boehringer Mannheim) enthielt, 2 Stunden bei 14°C inkubiert. Nach Auftrennung am Agarosegel (1,4%) wurden DNA-Fragmente mit einer Größe von 400 - 900 bp durch Elektroelution und Ethanolfällung wiedergewonnen. Diese DNA-Fragmente wurden in den M13 mp9-Vektor, der mit dem Restriktionsenzym Sma I linearisiert und mit bakterieller alkalischer Phosphatase behandelt worden war (Insert: Vektor = 5 : 1), in Gegenwart von 1 mM ATP und 1 U T4-DNA-Ligase inseriert (15 h bei 15°C).

Für die Transformation kompetente E.coliStamm JM101 Zellen wurden mit dem Gemisch der Ligase-Reaktion mindestens 2 Stunden bei 0°C inkubiert. Nach einem Hitzeschock (90 sek., 42°C) wurden die Zellen mit 40 µl 100 mM IPTG, 40 µl einer Lösung von 2% X-Gal in DMF und 3 ml Top-Agar (10 g Trypton, 8 g NaCl, 8 g Agar in 1 l), der aufgeschmolzen und auf 42°C temperiert worden war, vermischt und auf leicht vorgewärmte H-Platten (10 g Trypton, 8 g NaCl, 12 g Agar in 1 l) aufgetragen. Nach Erstarren des Top-Agars wurden die Platten bei 37°C über Nacht inkubiert.

Zur Isolierung der Einzelstrang-Phagen-DNA wurde je ein farbloses Plaque in ein Röhrchen mit 1,5 ml einer verdünnten E.coli JM101 Kultur (1 ml stationäre Kultur in 100 ml 2 × TY Medium verdünnt (16 g Trypton, 10 g Hefeextrakt, 5 g NaCl in 1 l)) transferiert. Nach 6 Stunden Inkubation bei 37°C wurden die Bakterien abzentrifugiert und die Phagen aus dem Überstand durch Zugabe von 200 µl einer Lösung von 20% PEG 8000, 2,5 M NaCl während 15 min bei Raumtemperatur ausgefällt. Das Präzipitat wurde pelletiert und der Überstand restlos entfernt. Das Pellet wurde in 100 µl TE-Puffer gelöst, mit Phenol/Chloroform - wie bereits beschrieben-extrahiert und die Einzelstrang-DNA aus der wässrigen Phase mit Na-Acetat/Ethanol ausgefällt. Die Größe jeder Einzelstrang-DNA wurde durch Elektrophorese am 0,8% Agarosegel relativ zur Wildtyp-Phagen-DNA bestimmt. Die Sequenzierung wurde ohne Modifikationen nach der Kettenabbruchmethode durchgeführt (Sanger, F., Nicklen, S. und Coulsen, A.S., 1977, loc.cit.)

Analyse der Sequenzierungsdaten

Die Sequenzierungsergebnisse wurden mit Hilfe eines Cyber 170 Computers analysiert. Dabei wurden die Programme von Staden (Staden, R. 1980, Nucleic Acids Res. 8, 3673 -3694) und die modifizierten Programmformen von Isono (Isono, K. 1982, Nucleic Acids Res. 10, 85 - 89) verwendet.

Beispiel 4

Proteolytische Spaltstellen in der PI-Hüllenprotein-Region des Polyproteins

Die viralen Proteine werden durch proteolytische Spaltung aus dem Polyprotein erhalten. Um die Spaltstellen zu identifizieren, wurden die viralen Hüllenproteine isoliert und die N-terminale Aminosäuresequenz bestimmt. Zu diesem Zweck wurden die Proteine von 2 mg HRV89 elektrophoretisch auf einem 12,5%igen Polyacrylamid-Gel aufgetrennt (Laemmli, U.K., 1970, loc.cit.). Die Gele wurden mit einer gesättigten Lösung von Coomassie-Brillant-Blau in 50 mM Tris/HCl, pH 7,4 gefärbt und die Proteinbanden herausgeschnitten. Die einzelnen Proteine wurden in einer ISCO-Elutionsapparatur bei 50 V 16 Stunden elektroeluiert und mit Trichloressigsäure präzipitiert. Die N-terminalen Aminosäuren wurden mit Hilfe eines AB-470A Protein-Sequenators (Applied Biosystems, Inc. Foster City, CA, USA) bestimmt. Zur Sequenzierung wurden je 2 nmol VP1 und VP2 und 1 nmol VP3 verwendet. Die derivatisierten Aminosäuren wurden mittels HPLC analysiert. Die N-terminalen Sequenzen der einzelnen Proteine sind in Fig. 6 angegeben

Beispiel 5

Konstruktion des Expressionsplasmides pRH 100

Alle Enzymreaktionen wurden unter den von den Herstellern angegebenen Bedingungen durchgeführt.

7 µg Plasmid pER103 (Eva Dworkin-Rastl et al., 1983, Gene 21, 237-248: EP-A-O.115-613) wurden in 50 µl Reaktionsmedium mit der Restriktionsendonuklease HindIII linearisiert. Nach einer einstündigen Inkubation bei 37°C wurden 50 µl 2× CIP-Puffer zugesetzt (2× CIP-Puffer = 20 mM Tris, pH = 9,2, 0,2 mM EDTA). Durch Zugabe von 2 Einheiten alkalischer Phosphatase aus Kälberdarm (CIP) wurden die 5'terminalen Phosphatreste entfernt; inkubiert wurde bei 45°C 30 Minuten lang. Die Reaktion wurde durch Zugabe von 4 µl 0,5 M EDTA-Lösung sowie Zugabe von 10 µl 1M Tris, pH = 8,0 Lösung gestoppt. Die Proteine wurden durch zweimalige Phenol-und einmalige Phenol-/Chloroformextraktion entfernt. Die DNA wurde aus der wäßrigen Phase nach Zugabe von 0,1 vol 3M Natriumacetatlösung pH = 5,5 und 250 µl Äthanol ausgefällt, das DNA-Präzipitat nach Zentrifugieren einmal mit 70%-iger Äthanollösung gewaschen. Die DNA wurde getrocknet, und das Pellet anschließend in 20 µl TE-Puffer (10 mM Tris pH = 8,0, 1 mM EDTA) gelöst.

Jeweils 1 µg der synthetisch hergestellten Oligonukleotide d(AGCTTAAAGATGAGCT) und d-(CATCTTTA) wurden in je 10 µl Reaktionslösung unter Zugabe von 10 Einheiten T4-PNK sowie 1 mM rATP phosphoryliert. Die Reaktion fand bei 37°C statt und dauerte 45 Minuten. Die Reaktion wurde durch 10-minütiges Erhitzen auf 70°C abgebrochen.

Jeweils 5 µl der Plasmidlösung und der phosphorylierten Oligonukleotide wurden gemischt und 5 Minuten auf 70°C erwärmt. Danach wurde die Lösung auf 0°C abgekühlt, 2 µl 10× Ligasepuffer (500 mM Tris, pH = 7,5, 100 mM MgCl₂ 200 mM DTT, 1 mM rATP, 500 µg/ml BSA), sowie 2 µl Wasser und 10 Einheiten T4-DNA Ligase zugesetzt. Die Reaktion dauerte 40 Stunden und wurde bei 4°C durchgeführt. Sie wurde durch 10-minütiges Erhitzen auf 70°C abgebrochen.

2 µl dieser Ligasereaktion wurden in insgesamt 30 µl Lösung mit 10 Einheiten der Restriktionsendonuklease SacI (New England Biolabs) 3 Stunden bei 37°C verdaut. Die Reaktion wurde durch 10-minütiges Erhitzen auf 70°C abgebrochen. 5 µl dieser Reaktionsmischung wurden in insgesamt 30 µl durch Zugabe von 10 Einheiten T4-PNK bei 14°C 16 Stunden lang ligiert.

200 µl kompetenter E.coli HB101 wurden mit 10 µl dieser Ligasereaktion versetzt. Die Bakterien wurden 45 Minuten auf Eis gehalten und anschließend 2 Minuten auf 42°C erwärmt, um die DNA-Aufnahme zu ermöglichen. Anschließend wurde die Bakteriensuspension wieder bei 0°C 10 Minuten inkubiert. Abschließend wurden die transformierten Bakterien auf einem LB-Agar, der 50 µg/ml Ampicillin enthielt, ausgestrichen.

Von den entstandenen Bakterienkolonien wurden 12 willkürlich ausgesucht und daraus im Mikromaßstab die Plasmide isoliert (Birnboim, H.C. und Doly, J.K., 1979, Nucl. Acids Res. 7 1513-1523). Die resultierende DNA wurde mit der Restriktionsendonuklease SacI geschnitten und die DNA auf einem Agarosegel (1%, 1× TBE-Puffer) aufgetrennt. Die Wanderung der DNA als lineares Molekül der Größe von etwa 4.400 bp bestätigte die Einführung einer SacI-Erkennungsstelle in das Plasmid. Eines dieser Plasmide wurde willkürlich ausgesucht. Mit der DNA aus der dazugehörigen Minipräparation wurde nochmals E.coli HB101 transformiert. Von den resultierenden transformierten Bakterien wurde eine Kolonie ausgewählt und in größerem Maßstab angezüchtet. Das daraus isolierte Plasmid wurde mit den Restriktionsendonukleasen EcoRI und BamHI geschnitten, die DNA auf einem 1%-igem Agarosegel aufgetrennt, und das kleinere Fragment aus dem Gel durch Elektroelution isoliert. Dieses etwa 460 bp lange EcoRI-BamHI DNA-Fragment wurde nach Sanger sequenziert (Sanger, F., Nicklen, S. und Coulson, A.R., 1977, Proc.Natl.Acad.Sci. 74, 5463-5467). Das dermaßen analysierte Plasmid wurde pRH100 genannt (Fig. 7).

Beispiel 6:

Isolierung des XhoII-Fragmentes (969/1) und Herstellung des Expressionsplasmides pRH969/1

Ausgehend beispielsweise von der in Fig. 9 dargestellten DNA, ein Fragment des nach EPA 192 175 erhältlichen HRV2 Genoms, die sich als Insert in einem Vektor, beispielsweise pUC9, befindet wurde durch Doppelverdau mit den Restriktionsenzymen HindIII (Biolabs) und EcoRI (Boehringer Mannheim) in 100 µl Eco-Hind-Puffer (7,5mM MgCl₂, 75mM TrisHCl pH = 7,5, 50mM NaCl) mit je 15 U an EcoRI und HindIII 15 Stunden bei 37°C das DNA-Insert herausgeschnitten. Die Abtrennung der Insert-DNA vom linearisierten Vektor erfolgte durch Auftrennung am 1%igen Agarosegel. Dazu wurden die 100 µl mit 11 µl 10× Agarosegel-"loading buffer" (0,1% Bromphenolblau, 0,1% Xylencyanol, 35% Ficoll, 0,5% SDS) versetzt. Das abgetrennte EcoRI/HindIII-Fragment von pHRV2-969 (ca. 1,1 kb) wurde mit Hilfe von DE81-Papier (Whatman) aus dem Agarosegel isoliert (Dretzen, G.M., Bellard, P., Sassone-Corsi und Chambon, P.; 1981, Anal. Biochem. 112, 295 - 298). Dazu wurde nach Auftrennung der DNA-Fragmente am Agarosegel vor und hinter die zu isolierende DNA-Bande ein Schlitz geschnitten, in den ein Streifen DE81-Papier gesteckt wurde. Die Elektrophorese wurde weitergeführt (Gel soll nicht mit Puffer bedeckt sein), bis das gewünschte DNA-Fragment vollständig an den vorderen DE81-Streifen gebunden worden war. Der hintere DE81-Streifen verhinderte eine Verunreinigung durch größere DNA-Fragmente. Das DE81-Papier mit dem gebundenen DNA-Fragment wurde in ein 1,5 ml Eppendorfröhrchen (mit einem Ausflußloch im Gefäßboden und darübergelagerter Polyalomerwolle) transferiert und zweimal 5 min mit je 500 µl Waschpuffer (0,2M NaCl,

25mM TrisHCl pH = 8,0, 1M EDTA) gewaschen, wobei durch kurze Zentrifugation (ca. 1 sek) die Waschlösung im zweiten darunter befindlichen Eppendorfgefäß aufgefangen wurde. Anschließend wurde die gebundene DNA wie oben beschrieben zweimal durch 15 Minuten dauernde Inkubationen in je 200 µl Elutionspuffer (1mM NaCl, 25mM TrisHCl pH = 7,5, 1mMEDTA) vom DE81-Papier gewaschen (das Ethidiumbromid bleibt adsorbiert). Die 400 µl Eluat wurden 10 min in der Eppendorfzentrifuge (15000g) zentrifugiert, um Papierstückchen zu entfernen. Der Überstand wurde vorsichtig in ein neues Eppendorfgefäß transferiert, mit 800 µl 96% Ethanol versetzt, bei -20°C ausgefällt (ca. 2 Stunden), zweimal mit 70% Ethanol gewaschen, getrocknet und in 50 µl (10mM TrisHCl pH = 8, 10mM MgCl₂) mit 10 U Xho II (Boehringer) 2 Stunden bei 37°C nachgeschnitten. Um das Restriktionsenzym zu inaktivieren wurde 2 min bei 70°C inkubiert und anschließend der Reaktionsansatz langsam auf Raumtemperatur abgekühlt. Zu diesen 50 µl wurden 6 µl 10× Klenow-Puffer (660mM TrisHCl pH = 7,5, 66mM MgCl₂, 3mM dNTPs, 1mM DTT, 0,1 mg/ml BSA), 2 µl Klenowenzym (4 U/µl; Amersham) und 2 µl H₂O zugesetzt und 30 min bei 22°C inkubiert. Die Reaktion wurde durch Zugabe von 2 µl 0,5M EDTA pH = 8 gestoppt, die wässrige Phase einmal mit gleichem Volumen Phenol/CHCl₃/Isoamylalkohol (25:24:1), einmal mit gleichem Volumen CHCl₃ extrahiert und mit 7 µl 10× Agarosegel-"loading-buffer" versetzt. Zur Abtrennung des 1092 bp langen Xho II-Fragmentes von den entstandenen Oligonukleotiden isolierte man mit Hilfe von DE81-Papier das mit einer "blunt-end"-Struktur versehene Fragment (969/1) aus einem 1%igem Agarosegel. Der verwendete Expressionsvektor pRH100 stellt im wesentlichen ein pBR322-Derivat dar, dessen Modifikation darin besteht, daß zwischen den Basen 4362/0 (EcoRI-Stelle in pBR322) und 29 (HindIII-Stelle in pBR322) ein 123 bp langes Fragment eingefügt wurde, welches die Tryptophanpromotorregion von Serratia marcescens, eine synthetische Ribosomenbindungsstelle und das Startkodon ATG besitzt (siehe Fig. 8).

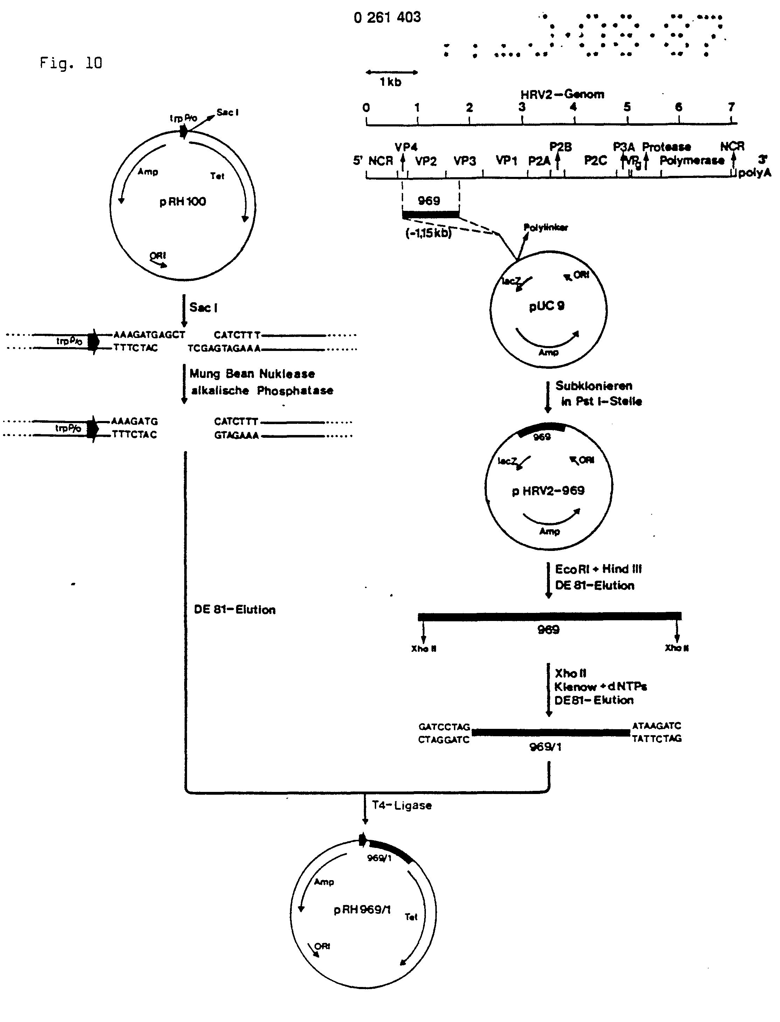

Die Konstruktion des Expressionsplasmids pRH969/1 ist in Fig. 10 schematisch dargestellt. Etwa 10 µg des Expressionsvektors pRH100 wurden in 200 µl (6mM TrisHCl pH = 7,5, 6mM BME, 6mM MgCl₂) mit 100 U Sac I (Biolabs) bei 37°C über Nacht verdaut. Die Plasmid-DNA wurde in üblicher Weise mit Ethanol präzipitiert, gewaschen, getrocknet und in 200 µl (200mM NaCl, 1mM ZnCl₂ 30mM Natriumacetat pH = 4,75, 5% Glycerin) aufgenommen und mit 150 U Mung bean Nuklease behandelt. Der Ansatz wurde vorher in zwei 100 µl Portionen aufgeteilt und einerseits 2 Stunden bei 14°C andererseits 30 min bei 37°C inkubiert. Die Reaktion wurde durch Zugabe von 5 µl 0,5M EDTA und Extraktion mit Phenol/CHCl₃/Isoamylalkohol (25:24:1) bzw. mit CHCl₃ abgestoppt und die beiden Ansätze vereinigt. Das linearisierte Plasmid wurde in üblicher Weise mit Natriumacetat und Ethanol gefällt, gewaschen und getrocknet. Die DNA wurde anschließend in 100 µl (100mM TrisHCl pH = 8) mit 40 U alkalischer Phosphatase (Boehringer Mannheim) 1 Stunde bei 37°C inkubiert. Nach Zugabe von EDTA auf 20mM wurde in üblicher Weise zweimal mit Phenol/CHCl₃/Isoamylalkohol (25:24:1) und zweimal mit CHCl₃ extrahiert und die DNA durch Ethanol gefällt. Für die Ligation wurden pro Ansatz ca. 50-175 ng pRH100 (linearisiert mit Sac I; mit Mung bean Nuklease und alkalischer Phosphatase behandelt) und ca. 1-2 µg des Xho II-Fragmentes vom Subklon pHRV2-969 (mit Klenow behandelt) gemeinsam mit Ethanol präzipitiert, getrocknet und in 10 µl frisch zubereiteten 1× Ligasepuffer (50mM TrisHCl pH = 7,5, 10mM MgCl₂ 20mM, DTT, 0,1mM rATP und 50 µg/ml BSA) aufgenommen. Die Ligation wurde 70 Stunden bei 14°C mit 1 U T4-Ligase (Boehringer Mannheim) durchgeführt). Um für die Transformation kompetente Zellen zu erhalten, wurde eine modifizierte Vorschrift von Mandel und Higa (Mandel, M. und Higa, A., 1970, J. Mol. Biol. 53, 159-162) angewandt. Dazu wurden 0,5 ml einer "über Nachtkultur" von E.coli Stamm HB 101 in 50 ml LB-Medium (10g/l Trypton, 5 g/l Hefeextrakt, 10 g/l NaCl) überimpft, bis zu einer OD₆₀₀ von ca. 0,4 hochgezüchtet und anschließend 5 min bei 5 k und 4°C pelletiert. Die Bakterien wurden dann in 25 ml 0,1M MgCl₂ (eiskalt) vorsichtig resuspendiert, 5 min auf Eis gestellt und abermals 5 min bei 5 k und 4°C zentrifugiert. Das Pellet wurde in 25 ml 0,1M CaCl₂ (eiskalt) resuspendiert, 4 Stunden auf Eis gestellt und 5 min bei 5 k und 4°C zentrifugiert. Die Zellen wurden in 2,5 ml 1× Lagerpuffer (0,1M CaCl₂/Glycerin = 4/1% v/v) aufgenommen, 20 min auf Eis gestellt, in 100 µl Portionen aliquotiert und in flüssigem Stickstoff schockgefroren, sowie bei -80°C gelagert. Zu 100 µl auf Eis aufgetauter kompetenter Zellsuspension wurden 5 µl des oben beschriebenen Ligationsansatzes zugegeben, die Zellen 1 Stunde auf Eis und 2 min bei 42°C inkubiert und abschließend 5 min auf Eis gestellt. Vor dem Ausplattieren der Zellen wurden 900 µl LB-Medium zugesetzt, 10 min bei 37°C inkubiert und je 200 µl Zellsuspension auf LB-Agar-Platten (1,5%iger Agar in LB-Medium mit 100 mg/l Ampicillin) aufgebracht und über Nacht inkubiert. Insgesamt konnten 351 Amp ʳ-Klone isoliert werden, aus denen die Plasmid-DNA nach der "Mini-Plasmid-Präparationstechnik" isoliert wurde (Birnboim, H.C. und Doly, J. Loc. cit. Durch HindIII-Verdau der Plasmid-DNA konnten 8 Klone mit einem der Größe des Xho II-Fragmentes entsprechenden Insert bestimmt werden. Da bei dieser Konstruktion eine beidseitige "blunt end"-Ligation notwendig war und daher prinzipiell 2 Orientierungsmöglichkeiten des Inserts gegeben waren, wurde eine Restriktionsanalyse durchgeführt. Durch Verdau mit 3,5 U Bal I/100 ng Plasmid-DNA in 10 mM TrisHCl pH = 7,5, 10 mM MgCl₂, 10mM BME und durch Doppelverdau mit 8 U BssHII/100 ng Plasmid-DNA

in 25mM NaCl, 6mM TrisHCl pH = 7,5, 6mM MgCl₂, 20mM BME (2 Stunden bei 50°C) bzw. mit 20 U BamHl/100 ng Plasmid-DNA in 150mM NaCl, 6mM TrisHCl pH = 7,5, 6mM MgCl₂, 20mM BME (2 Stunden bei 37°C) wurden 6 Klone (Nr.: 156, 165, 289, 301, 307 und 319) mit korrekter und 2 Klone (Nr.: 1 und 224) mit invertierter Insertorientierung identifiziert. Um zu überprüfen ob das Xho II-Fragment von 969 in Phase mit dem Startkodon ATG steht, wurde mit Hilfe eines 17mer-Sequenzprimers, direkt am cccPlasmid die Basenfolge der Übergangsregion zwischen dem nichtkodierenden und kodierenden Abschnitt ermittelt. Der Sequenzprimer ist zu den ersten 17 Basen des nicht-kodogenen Stranges der Trp-Promotorregion komplementär (siehe Fig. 8).

Dazu wurde aus den oben angeführten Klonen die Plasmid-DNA nach der "Mini-Präparationstechnik" gewonnen, in je 100 µl TE-Puffer, mit je 100 µl 5M LiCl versetzt und 5 min bei 0°C inkubiert.Nach der Zentrifugation in der Eppendorfzentrifuge (15000g, 5min, 4°C) wurde der Überstand mit 400 µl 96% Ethanol versetzt, abermals 2 min bei Raumtemperatur zentrifugiert, das Pellet einmal mit 70% Ethanol gewaschen und getrocknet. Die Plasmid-DNA wurde anschließend in je 11 µl H₂O gelöst, wovon je 5 µl wässrige Plasmidlösung mit 5 µl 1× Denaturierungslösung (267mM NaOH, 0,267mM EDTA) versetzt wurden. Nach einer Inkubation von 15 min bei Raumtemperatur wurde je 1 µl 17mer-Sequenzprimer (50 ng/µl) und je 2 µl 2M CH₃COONH₄ (pH = 4,5) zugesetzt und 5 min bei Raumtemperatur inkubiert. Nach Zugabe von 75 µl 96% Ethanol und Präzipitation bei -70°C (15 min) wurde 5 min bei 15000g pelletiert, mit 70% Ethanol gewaschen, getrocknet und die mit dem Primer hybridisierte Plasmid-DNA in je 8,5 µl H₂O aufgenommen. Zu diesen 8,5 µl wurden je 1 µl 10× Bindungspuffer (100mM TrisHCl pH = 8, 50mM MgCl₂, 3 µl ³⁵S-ATP (1000 Ci/mmol; Amersham) und 1 µl Klenow-Enzym (4 u/µl; Biolabs) zugegeben. Der weitere Ablauf der Sequenzierung erfolgte nach der Kettenabbruchmethode von Sanger et al. (Sanger, F., Nicklen, S. und Coulson, A.R., 1977, Proc. Natl. Acad. Sci. USA 74, 5463 - 5467). Im Expressionsplasmid pRH969/1 aus Klon 307 und 319 wurde ein in Phase mit dem ATG befindliches Insert festgestellt.

Beispiel 7:

Expression des viralen Hüllenproteinbereiches 969/1 von HRV2 in E.coli Stamm CSR603 Zellen

Um die Expression plasmidkodierter Proteine in E.coli nachweisen zu können, wurde ein von Sancar (Sancar, A., Hack, A.M. und Rupp, W.D. 1979, J. Bacteriol., 137, 692-693) entwickeltes Maxizellsystem verwendet. Der E.coli Maxizellstamm CSR603 (CGSC 5830; F⁻, thr-1, leuB6, proA2, phr-1, recA1, argE3, thi-1, uvrA6, ara-14, lacY1, galK2, xyl-5, mtl-1, gyrA98(nalA98), rps31, tsx-33, lambda⁻, supE44) besitzt keine Mechanismen für die Reparatur von durch UV-Bestrahlung entstandene Schäden der DNA. Für das Experiment wird die Strahlendosis so optimiert, daß das Bakterienchromosom oft getroffen wird, die vergleichsweise sehr kleine Plasmid-DNA, die zudem in mehreren Kopien pro Zelle vorhanden ist, meist unbeschädigt bleibt. Die geschädigte chromosomale DNA wird von der Zelle abgebaut, wogegen Plasmide, die nicht von der UV-Strahlung getroffen wurden, weiter replizieren und den Hauptteil der verbliebenen DNA darstellen. Nach Abtöten aller nicht geschädigten, sich vermehrenden Zellen durch das Antibiotikum D-Cycloserin und Aufbrauchen der endogenen mRNA, werden in den verbleibenden Zellen nur noch am Plasmid kodierte Gene transkribiert und translatiert. Nach Überführung der Bakterien in ein sulfatfreies Medium können die gebildeten Proteine durch den Einbau von ³⁵S-Methionin radioaktiv markiert und nachgewiesen werden.

E.coli Stamm CSR603 - Zellen wurden mit dem geeigneten Expressionsplasmid pRH969/1 vom Klon 307 und 319 bzw. 224 (mit invertierter Orientierung von 969/1) wie oben beschrieben transformiert. Die transformierten Zellen wurden in 15 ml tryptophanfreiem Expressionsmedium bis zu einer optischen Dichte bei 600 nm von 0,5-0,7 bei 37°C gezüchtet. Das verwendete Expressionsmedium wurde wie folgt hergestellt (Angaben pro Liter Medium):

a) 10 g (NH₄)₂HPO₄ 3,5 g KH₂PO₄ und 0,5 g NaCl wurden in 500 ml H₂O gelöst, die Lösung mit NaOH auf pH = 7,3 gebracht und auf ca. 800 ml aufgefüllt.

b) 21 g Casaminosäuren (säurehydrolisiert, vitaminfrei; Merck) wurden in 100 ml H₂O gelöst.

c) 50 ml 20% Glucose

d) 1 ml 1M MgSO₄ .

e) 1 ml 0,1M CaCl₂

f) 1 mg Thiamin-HCl und 20 mg L-Cystein wurden in 1 ml H₂O gelöst.

Diese autoklavierten bzw. steril filtrierten Lösungen wurden kurz vor Gebrauch gemischt, auf 1000 ml aufgefüllt und mit 100 mg/l Ampicillin versetzt. 10 ml der geernteten Bakterienkulturen wurden in eine offene 13,5 cm Petrischale transferiert, mit einer UV-Germicid-Lampe (15 Watt) aus 50 cm Entfernung 5 sek unter leichtem Schwenken bestrahlt und bei 37°C weiterinkubiert. Die Kulturen wurden mit 100 µg/ml D-Cycloserin versetzt (frische, 100$^\times$ konzentrierte Lösung in 50mM Phosphatpuffer pH = 8) und 14-16 Stunden bei 37°C am Schüttler inkubiert. Die Bakterien wurden durch Zentrifugation geerntet (5 min, 5k bei Raumtemperatur), zweimal mit 5 ml Hershey-Salzlösung gewaschen, in 5 ml Hershey-Medium mit 20 µg/ml 3-ß-Indolacrylsäure ( = IAA; Induktor des Tryptophanoperons) suspendiert und 1 Stunde bei 37°C geschüttelt.

## Hershey-Salzlösung (pro Liter):

| | | |
|---|---|---|
| 5,4 g NaCl | 15 mg $CaCl_2 \cdot 2H_2O$ | 87 mg $KH_2PO_4$ |
| 3,0 g KCl | 0,2 g $MgCl_2 \cdot 6H_2O$ | 12,1 g Tris + HCl pH=7,4 |
| 1,1 g $NH_4Cl$ | 0,2 mg $FeCl_3 \cdot 6H_2O$ | |

## Hershey-Medium (pro 100 ml Hershey-Salzlösung):

| | | | |
|---|---|---|---|
| 2,0 ml | 20% Glucose | 1,0 ml | 2 % Prolin |
| 0,5 ml | 2% Threonin | 1,0 ml | 2 % Arginin |
| 1,0 ml | 1% Leucin | 0,1 ml | 0,1% Thiamin-HCl |

Zu jeder Kultur wurden ca. 15 µCi/ml $^{35}$S-Methionin (1000 Ci/mmol; Amersham) zugesetzt und diese eine weitere halbe Stunde bei 37°C inkubiert. Anschließend wurden die Zellen durch Zentrifugation (5k, 5 min bei Raumtemperatur) geerntet und in 200 µl SDS-Probenpuffer (10mM TrisHCl pH = 7,4, 125mM BME, 2% SDS, 10% Saccharose, 0,05% Bromphenolblau) lysiert. Die Proben wurden 5 min bei 95°C inkubiert und 2 min bei 15000g zentrifugiert. Der Überstand wurde auf die Anwesenheit von exprimierten Proteinen überprüft.

20-60 µl Zellysatüberstand in SDS-Probenpuffer (siehe oben) wurden auf einem 10%igem SDS-haltigen Polyacrylamidgel (Laemmli, U.K., 1970, Nature 277 , 680-686) nach ihrer Größe aufgetrennt. Die Gele mit einer Dicke von 1,5 mm bestanden aus einem etwa 10 cm langen 10%igen Trenngel und einem 3 cm langen 3%igen Sammelgel. Die Elektrophorese wurde im Sammelgel bei 3 Watt (konstant), im Trenngel bei 12 Watt (konstant) durchgeführt (Gesamtdauer 4,5 Stunden). Als Größenmarker wurde ein Proteingemisch bestehend aus BSA (66kd), Ovalbumin (45kd), Pepsin (34,7kd), ß-Lactoglobulin (18,4kd) und Lysozym (14,3kd) mitaufgetrennt. Die Gelspur der Markerproteine wurde vom übrigen Gel nach der Elektrophorese abgetrennt, 30 min in der Färbelösung (50% Methanol, 10% Essigsäure, 0,1% Coomassie-brillant-blue 2250) inkubiert, über Nacht in der Entfärbelösung (5% Methanol, 10% Essigsäure) geschüttelt, 30 min in ein Glycerinbad (7% Essigsäure, 2% Glycerin) gelegt und am Geltrockner ca. 1,5 Stunden bei 60°C auf Whatman 3MM-Papier getrocknet (siehe Fig. 11, Spur M). Die Proteine der Zellysate wurden nach der "Western-Blot"-Methode durch Elektrotransfer vom Gel auf Nitrozellulosefilter (Schleicher und Schüll, BA85, 0,45 um) übertragen (Burnette, W.N., 1981, Analyt. Biochem. 112, 195-203). Der Transfer wurde im Transferbuffer (20mM Tris, 150mM Glycin, 20% Methanol p.a.; pH = 8,8) bei 60 Volt, 5 Stunden in einer Biorad-Protein-Blot-Apparatur in Anwesenheit von 0,1% Empigen (Marchon Italiana S.P.A.) durchgeführt (Mandrell, R.E. und Zollinger, W.D., 1984 J. Immunol. Meth. 67, 1-11).

Die Expression der ausschließlich von Plasmid kodierten Proteine wurde durch Einbau von $^{35}$S-Methionin nachgewiesen. In Fig. 11 ist das Autoradiogramm (Spur B2 bis B3) des "Western-Blots" nach Exponierung auf Kodak X-Omat S Röntgenfilm dargestellt. Spur B2 (entspricht Spur A2 des Western Blots) und Spur B4 (entspricht Spur A4) weisen sowohl Signale des exprimierten Inserts 969/1 als auch der vom Plasmidgen für Ampicillinresistenz kodierten ß-Lactamase auf, während <u>Spur B3</u> (entspricht Spur A3) mit dem invertierten Insert 969/1 kein Signal bei 40 kd, wohl aber das für die ß-Lactamase aufweist. Dadurch, daß zu Beginn der invertiert kodierenden Region mehrere Terminationskodons vorhanden sind, wird kein Protein exprimiert.

<u>Beispiel 8:</u>

Produktion und Isolierung monoklonaler Antikörper

Erläuterung der verwendeten Lösungen und Medien:

<u>100$^\times$ Aminopterin</u>

1,8 mg Aminopterin werden in 50 ml $H_2O$ durch Zugabe von 1M NaOH gelöst und auf 100 ml aufgefüllt (Lagerung bei -20°C im Dunkeln).

<u>100$^\times$ OPI-mix:</u>

7,5 g Oxalsäure und 2,5 g Pyruvat werden in 450 ml $H_2o$ gelöst und mit 1000 U Insulin (in einigen ml 0,1M HCl gelöst) versetzt. Dann wird mit $H_2O$ auf 500 ml aufgefüllt.

<u>100$^\times$ HT-mix:</u>

0,6805 g Hypoxanthin in 200 ml $H_2O$ gelöst und mit NaOH auf pH = 10 eingestellt werden mit 200 ml einer wässrigen Thymidinlösung (0,1937 g in 200 ml) vereint und auf 500 ml aufgefüllt.

<u>HT-Medium:</u>500 ml    Delbecco's Modified Eagle's Medium (DMEM; Flow Laboratories; high glucose)
6 ml    200mM L-Glutamin
6 ml    100$^\times$ HT-mix
6 ml    100$^\times$ OPI-mix
50 ml    HIFKS (hitzeinaktiviertes fötales Kälberserum)
0,6 ml    Gentamycin (50 mg/ml)

<u>HAT-Medium:</u>100 ml    HT-Medium
1 ml    100$^\times$ Aminopterin

<u>ABTS-Lösung:</u>10 mg/ml wässrige Lösung von 2,2'-azino-di (3-ethylbenzthiazolinsulfonat).

<u>PBS:</u>137 mM    NaCl
2,7 mM    KCl
8 mM    $Na_2HPO_4$
1,5 mM    $KH_2PO_4$
0,5 mM    $MgCl_2.6H_2O$
1 mM    $CaCl_2.2H_2O$

<u>PBS def.:</u>wie PBS nur ohne Ca-und Mg-Salze.

<u>PBS-Waschlösung:</u>PBS def. mit 0,05% Tween 20.

20% FKS-DMEM:445 ml    Dulbecco's Modified Eagle's Medium (Flow Laboratories)
50 ml    HIFKS (hitzeinaktiviertes fötales Kälberserum)
5 ml    Penicillin/Streptomycin (10 000 U/ml)

RPMI kompl.:500 ml    RPMI 1640 Medium (Flow Laboratories; Nr.: 12-604-54)
50 ml    FKS
5 ml    200 mM L-Glutamin
5 ml    Penicillin/Streptomycin (10 000 U/ml)

PEG-4000-Lösung:

20 g PEG werden in 28 ml PBS def. gelöst und in einem Wasserbad (60°C) inkubiert bis das PEG gelöst ist (ca. 30 min).

RPMI-Waschlösung:500 ml    RPMI 1640 Medium
5 ml    Penicillin/Streptomycin (10.000 U/ml)
5 ml    200mM L-Glutamin

HAT-Thymozytenmedium:

Das steril entfernte Thymusorgan einer 3 - 4 Monate alten Balb-c-Maus (möglichst ohne Bindegewebe) wird in ein Sieb transferiert und mit einem sterilen Spritzenstempel durch das Sieb passiert (langsame, kreisförmige Bewegungen; ca. 1 min im Sieb rühren). Die Zellsuspension in 25 ml RPMI-Waschlösung wird 10 min bei Raumtemperatur und 300g abzentrifugiert. Mit einer Pasteurpipette wird der Überstand abgesaugt und in 20 ml RPMI kompl. resuspendiert und auf ca. $5 \times 10^6$ Zellen/ml weiterverdünnt. Dieses Thymozyten enthaltende Medium wird in kleinen Kulturflaschen bei 37°C im $CO_2$-Brutschrank aufbewahrt (ca. 14 Tage haltbar). Zur Herstellung des HAT-Thymozytenmediums werden die Zellen abzentrifugiert, wie oben beschrieben in RPMI-Waschlösung gewaschen, abermals zentrifugiert und das Zellenpellet in HAT-Medium aufgenommen.

In 2-3 Monate alte Balb-c Mäuse wurden intraperitoneal 5-10 $\mu$g gereinigtes und in komplettem Freund'schen Adjuvans emulgiertes HRV2 injiziert. In weiterer Folge wurde nach 27, 49, 100, 117 und 118 Tagen die gleiche Menge an HRV2 (in inkompletten Freund'schen Adjuvans emulgiert) verabreicht. Ein Tag nach der letzten Inokulation wurde die Milz entfernt und die Zellen mit Myeloma Zellen, z.B. NS-1 (ATCC TIB 18) fusioniert, wobei im wesentlichen nach dem Protokoll von Galfrè, G. und Milstein, C. 1981, Methods Enzymol. 73, 3-46) vorgegangen wurde.

Dabei wurde die unter sterilen Bedingungen entfernte Milz in einer Petrischale in ca. 5 ml RPMI-Waschlösung gewaschen. Die Milz wurde in 5 ml RPMI-Waschlösung zerkleinert und mit einem Spritzenstempel durch ein Sieb passiert (langsame runde Bewegungen). Anschließend ließ man die Zellsuspension auf Eis ruhen, um Zellklumpen und Teile des Bindegewebes zu sedimentieren. Mit einer Pipette wurden die feinsuspendierten Zellen abgesaugt und in ein 50 ml Zentrifugenröhrchen mit rundem Gefäßboden transferiert, mit frisch zubereiteter RPMI-Waschlösung auf 50 ml aufgefüllt und 5 min bei 300g und Raumtemperatur zentrifugiert. Der Überstand wurde entfernt und das Pellet in 5 ml Lysispuffer (155mM $NH_4Cl$, 10mM $KHCO_3$ und 1mM EDTA; pH = 7,2) suspendiert und 2 min bei 0°C inkubiert. Die Suspension wurde mit RPMI-Waschlösung auf 50 ml gebracht und vorsichtig mit einer Pasteurpipette aufgerührt. Lipidhaltige Verbindungen und Fette blieben dabei an der Pipette haften und konnten so leicht entfernt werden. Die Suspension wurde abermals für 5 min bei 300g und Raumtemperatur abzentrifugiert. Das Pellet wurde in 10 ml RPMI-Waschlösung resuspendiert und mit ca. $5 \times 10^7$ Maus-Myeloma Zellen, zB. NS-1 (aus vier 75 $cm^2$ Gewebekulturflaschen zu 50% konfluent) vermischt. Die Zellen wurden vorher dreimal mit 50 ml RPMI-Waschlösung gewaschen und bei 300g (5min und Raumtemperatur) zentrifugiert, um das gesamte Serum zu entfernen. Die Mischung aus Milz-und Myeloma Zellen wurde in 50 ml RPMI-Waschlösung resuspendiert und wie oben beschrieben zentrifugiert. Der Überstand wurde vorsichtig und vollständig entfernt, das Pellet sofort bei 37°C mit 1,5 ml thermostatisierter PEG-4000 Lösung versetzt und das Röhrchen vorsichtig 2 min lang an die Tischkante geklopft bis das Pellet und die PEG-4000 Lösung vermischt waren ("griesige Konsistenz"). Anschließend wurde 2 min bei 37°C unter leichtem Schütteln inkubiert und gleich darauf 20

26

ml RPMI-Waschlösung tropfenweise zugegeben, wobei man die Tropfen einzeln an der Gefäßwand herunterrinnen ließ. Weitere 30 ml RPMI-Waschlösung wurden vorsichtig zugegeben. Nach der Zentrifugation der Zellen (300g, 5min bei Raumtemperatur) würde der Überstand abgesaugt und das Pellet in 10 ml HAT-Thymozytenmedium vorsichtig mit einer Pasteurpipette resuspendiert. Diese Suspension wurde in aliquoten Teilen von 0,1 ml in Mikrotiterplatten (96 Näpfe/Platten) aufgeteilt und bei 37°C und 5% $CO_2$ inkubiert (Platten im Brutschrank nicht verschieben). Nach 3 Tagen wurden ca. 0,1 ml HAT-Medium zugegeben. Nach weiteren 3 Tagen wurden ca. 0,05 ml HAT-Medium zugegeben. 10 Tage nach der Fusion wurden 70% des Mediums entfernt und 0,2 ml HT-Medium nachgefüttert. 12 Tage nach der Fusion wurden die Platten mikroskopisch untersucht, das Medium für einen ELISA-und Neutralisationstest entnommen und ca. 0,3 ml 20%-FKS-DMEM pro Napf zugegeben. Positive Hybride wurden durch die oben erwähnten zwei Tests selektiert und die Hybride 1:3 bis 1:10 gespalten; d.h. der Inhalt eines Napfes wurde auf 3 bis 10 Näpfe der Mikrotiterplatten aufgeteilt. Nach dem Hochwachsen wurden die Hybride nochmals getestet und jene ausgewählt, die den höchsten Titer aufwiesen. Diese Hybride wurden durch "endpointdilution" zweimal kloniert, wobei unter dem Mikroskop sichergestellt wurde, daß die Hybridzellen von einer einzelnen Zelle abstammen. Die Klonierung wurde in Gegenwart von Thymuszellen und 20% FKS durchgeführt. Die einzelnen Klone wurden bis zur 50% - Konfluenz in 75 cm² Zellkulturflaschen hochgezüchtet. Die Zellen einer solchen Gewebekulturflasche wurden einer Maus, die 10 Tage vorher durch intraperitoneale Pristaninjektion (0,5 ml) vorbehandelt worden war, injiziert. Ascitesflüssigkeit wurde durch die Bauchdecke nach 2 bis 6 Wochen abgezogen.


ELISA:

Pro Napf einer Mikrotiterplatte wurden 50 μl einer Virusverdünnung (1-2 μg HRV2/ml) in Carbonatpuffer (15 mM $Na_2CO_3$/35 mM $NaHCO_3$) über Nacht bei Raumtemperatur inkubiert. Durch Zugabe von 100 μl PBS def., welches 1% BSA enthielt und durch einstündige Inkubation bei 37°C wurde abgesättigt. Die Mikrotiterplatte wurde auf Zellstoff ausgeklopft und die Näpfe mit 50 μl Hybridüberstand versetzt. Als Negativkontrolle wurde dabei reines HAT-Medium eingesetzt. Anschließend wurde eine Stunde bei 37°C inkubiert, erneut ausgeklopft und dreimal mit PBS-Waschlösung (PBS def. + 0,05% Tween 20( gewaschen. Dann wurden 50 μl einer 1/500 Verdünnung eines Peroxidase konjugierten Antiserums (RAM = "rabbit anti mouse" ) in PBS def. ( + 1% BSA und 2% Tween 20) pro Napf aufgebracht. Nach Inkubation (1 Stunde bei 37°C) wurden die Näpfe fünfmal mit PBS-Waschlösung, wie oben beschrieben, gewaschen. Zur Entwicklung des ELISAtests wurden pro Napf 50 μl Entwicklerlösung (1 ml 50mM Citratpuffer pH = 4, 10 μl ABTS-Lösung und 5 μl 30% Wasserstoffperoxid) zugesetzt. Ein positives Ergebnis war an der Grünfärbung zu erkennen.


**Neutralisationstest:**

Die Zellkulturüberstände von Hybridomkolonien wurden auf Anwesenheit von Virus neutralisierenden Antikörpern mittels Mikroneutralisation wie folgt getestet: jeweils 50 μl Hybridomaüberstand wurden pro Napf aufgetragen und mit je 50 μl MEM (Minimal-Essential-Medium; Flow Laboratories), welches 1000 PFU HRV2, 30 mM $MgCl_2$ und 2% HIFKS enthält, 1 Stunde bei 34°C in 5% $CO_2$ inkubiert. Jeder Napf wurde anschließend mit 3×10⁴ HeLa-Zellen (Stamm HeLa-Ohio, 03-0147, Flow Laboratories) versetzt, die Platten 48 Stunden bei 34°C in 5% $CO_2$ inkubiert und mit 0,1% Kristallviolett in 20% Ethanol angefärbt. Näpfe, die einen intakten Zellrasen aufwiesen, wurden als positiv angesehen. Unter den angeführten Bedingungen konnte bei einer 1/1000 Verdünnung der Ascitesflüssigkeit eine 50%ige Neutralisation festgestellt werden.
Aus der Gruppe von monoklonalen Antikörpern, die gegen das native HRV2 gerichtet sind, wurde ein monoklonaler Antikörper ausgesucht, der neben neutralisierenden Eigenschaften auch an die denaturierte Form seines Antigens - des viralen Kapsid-proteins VP2 - bindet, was mit Hilfe des "Western Blots" gezeigt werden konnte (Fig. 11). Ein solcher Antikörper wurde 8F5 benannt. Mit diesem monoklonalen Antikörper 8F5 hat man die Möglichkeit, nicht nur HRV2 zu neutralisieren oder Expressionsprodukte, die VP2 enthalten in "Western Blots" nachzuweisen, sondern man kann mit diesem Antikörper z.B. die immunogene Stelle des HRV2 auf VP2 näher charakterisieren, wie dies in Poliovirussystem für VP1 gezeigt werden konnte (Wychowski, C. et al., 1983, loc. cit.).
Ein monoklonaler Antikörper mit den genannten Eigenschaften, beispielsweise 8F5 wurde verwendet, um die antigenen Eigenschaften des exprimierten Proteins von pRH969/1 aus Klon 307 und 319 zu überprüfen.

Dieser Antikörper hat wie bereits erwähnt die für Expressionsstudien günstige Eigenschaft, VP2 (und damit auch 969/1) nicht nur im nativen, sondern auch im denaturierten Zustand zu erkennen. Diese Art von monoklonalen Antikörpern ist selten und wurde bis jetzt nur im Poliovirussystem gefunden (Wychowski, C. et al., 1983, loc. cit.). Die Filter mit den daraufgebundenen Proteinen wurden über Nacht bei Raumtemperatur in 50 ml "blocking-solution", das ist PBS (137mM NaCl, 2,7mM KCl, 8mM $Na_2HPO_4$, 1,5mM $KH_2PO_4$, 0,5mM $MgCl_2.6H_2O$, 1mM $CaCl_2.2H_2O$ mit 1% BSA, 1% Tween 20 und 10% Hitze inaktiviertem fötalen Kälberserum (HIFKS) gebadet. Anschließend erfolgte eine 3 stündige Inkubation in 4 ml "blocking-solution" mit dem Antikörper 8F5 (Maus Ascites-Überstand 1/200 in "blocking-solution" verdünnt) wobei das Filter zwischen Haushaltsfolie eingeschweißt (auf einer Wippe fixiert) inkubiert wurde. Danach wurde das Filter unter fließendem Leitungswasser gut gespült (ca. 20 min), dreimal je 15 min mit 50 ml PBS welches 1% Tween 20 enthält, gewaschen. Im letzten Schritt wurde das Filter in 50 ml "blocking solution" mit dem alkalische Phosphatase-konjugierten Anti-Maus IgG (ca. 1/3000 in "blocking solution" verdünnt) 3 Stunden bei Raumtemperatur inkubiert. Das Filter wurde wieder unter fließendem Leitungswasser gut gespült und dreimal wie oben beschrieben mit je 50 ml PBS, welches 1% Tween 20 enthält, gewaschen. Die Anfärbung erfolgte in 10 ml Phosphatasepuffer (100mM TrisHCl pH=9,5, 100mM NaCl, 5mM $MgCl_2$) in Anwesenheit der Farbstoffe Nitro-blue-Tetrazolium (NBT; 165 μg/ml) und 5-bromo-4-chloro-3-indolyl-Phosphat (BCIP; 82,5 μg/ml). Das alkalische Phosphatase-konjugierte Anti-Maus-IgG (H+L), sowie die Farbstoffe NBT und BCIP wurden dem Protoblot TM-System von Promega Biotec entnommen. Die Färbereaktion wurde ebenfalls durch Spülen unter fließendem Leitungswasser nach ca. 5 min abgebrochen. In Fig. 11 ist das Ergebnis der Expression von 969/1 im Maxizellsystem CSR603 zusammengefaßt. Die Spuren A1 bis A4 zeigen ein "Western-Blot"-Experiment nach Anfärben mit NBT und BCIP. In SpurA1 wurde als Kontrolle das gesamte denaturierte HRV2-Partikel (ca. 50 ng HRV2) mit aufgetrennt. Wie aus Fig. 11 hervorgeht reagiert der monoklonale Antikörper 8F5 ausschließlich mit VP2 (28,7 kd); die nur sehr schwach erkennbare höher-bzw. niedermolekulare Bande repräsentiert VPO (Vorläufer der Hüllenproteine) bzw. ein proteolytisches Abbauprodukt von VP2. In Spur A2 und Spur A4 wurden die Zellysate der mit dem Plasmid pRH969/1 (aus Klon 307 und 319 stammend) transformierten E.coli Stamm CSR603-Zellen aufgetrennt. Im beiden Spuren ist nach Anfärbung des "Western-Blots" das gewünschte spezifische Signal des Expressionsproduktes 969/1 als Bande bei ca. 40 kd (40,2 kd) zu erkennen. Als Negativkontrolle in Spur A3 wurde das Zellysat von E.coli Stamm CSR603-Zellen aufgetrennt, welche mit dem Plasmid aus Klon 224 transformiert worden waren. Dieses Plasmid weist wie oben beschrieben, eine invertierte Orientierung des Inserts 969/1 auf. Durch den damit verbundenen Verlust der spezifischen Antigenizität und der Tatsache, daß zu Beginn der invertiertkodierenden Region mehrere Terminationskodons vorhanden sind, kann am "Western-Blot" in Spur A3 kein Signal erhalten werden. Die Spur M zeigt die Coomassie-blue -Anfärbung der Markerproteine (siehe oben). Spur M zeigt die Coomassie-blue-Anfärbung der Markerproteine. Die Spuren B2 bis B4 stellen das Autoriadioprogramm von Spur A2 bis A4 dar (genaue Hinweise siehe Beispeil 2 loc. cit. ).

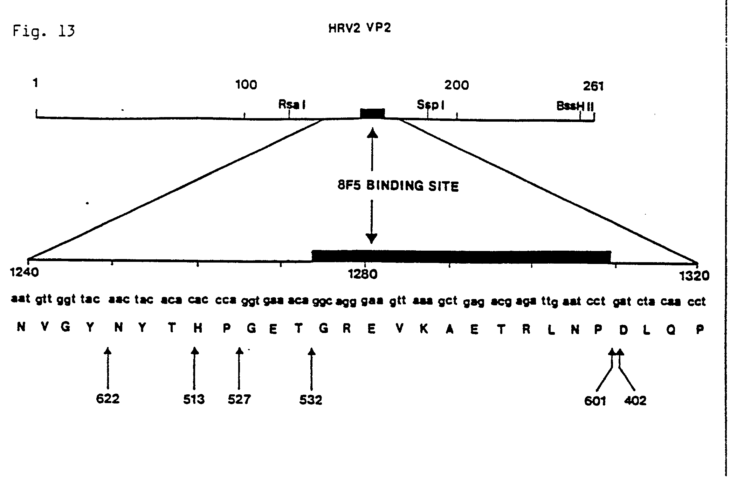

Im Wesentlichen der Strategie von Wychowsky et al. folgend, wurde ein Expressionsplasmid, das XhoII Fragment aus Fig. 9 enthält, beispielsweise das Plasmid pRH969/1 an der einzigen BssHII Schnittstelle linearisiert und mit der Exonuklease Bal-31 für 2, 4, 6, 8 und 10 Minuten behandelt. Die so verkürzten linearisierten Plasmide wurden rezirkularisiert und in kompetente E.coli Zellen transformiert. Die exprimierten Proteine wurden dann auf ihre Fähigkeit hin überprüft, den monoklonalen Antikörper 8F5 zu binden. Durch Vergleich der Sequenzen von den Plasmiden, die bindungsfähige Proteine exprimierten mit denen der Plasmide, die nicht-bindungsfähige Proteine exprimierten, konnte festgestellt werden, daß sich die immunogene Stelle des HRV2 auf VP2 für den monoklonalen Antikörper 8F5 zwischen den Aminosäuren befindet, die von den Nukleotiden 1274 bis 1309 des HRV2 kodiert werden (s. Fig. 9 und Fig. 13).

Erläuterungen von Abkürzungen ABTS:   2,2'-azino-di (3-ethylbenzthiazolinsulfonat)

AMP$^r$:   Ampicillin resistent

BCIP:   5-bromo-4-chloro-3-indolyl-phosphat

BME:   B-Mercaptoethanol

BPB:   Bromphenolblau

BSA:   Rinderserumalbumin

cccPlasmid:   "circular-covalently-closed"-Plasmid

DE-81-Papier:   Diethylaminoethyl-Zellulose-Papier

DMEM:   Dulbecco's Modified Eagle's Medium

dNTPs:   äquimolare Lösung aller 4 Desoxytriphosphate

DTT:   Dithiothreit

EDTA: Ethylendiaminotetraessigsäure

FKS: fötales Kälberserum

g: Erdbeschleunigung

HAT-Medium: Hypoxanthin-Thymidin-Medium

HIFKS: hitzeinaktiviertes, fötales Kälberserum

HRV2 bzw. 14: humanes Rhinovirus vom Typ2 bzw. 14

IAA: ß-Indolacrylsäure

kb: Kilobasen

kd: Kilodalton

Klenow(enzym): Klenowfragment der DNA-Polymerase I

LB-Medium: Das Luria-Bertani-Medium besteht aus 1% eines pankreatischen Verdaus von Milchcasein (Bactotrypton), 0,5% Hefeextrakt und 1% NaCl in destilliertem Wasser und wurde 20 min im Autoklaven auf 120°C erhitzt.

M: wässrige Lösung, die pro Liter ein Mol der betreffenden Substanz enthält.

MEM: Minimum-Essential-Medium

mM: wässrige Lösung, die pro Liter ein Millimol der betreffenden Substanz enthält.

ml: Milliliter

NBT: Nitro-blue-Tetrazolium

nm: Nanometer

$OD_{600}$ optische Dichte bei 600 nm

PBS: wässrige Puffermischung, die pro Liter 137 mMol NaCl, 2,7 mMol KCl, 8 mMol $Na_2HPO_4$, 1,5 mMol $KH_2PO_4$, 0,5 mMol $MgCl_2.6H_2O$ und 1 mMol $CaCl_2$ enthält und einen pH-Wert von 7,4 aufweist.

PBS def.: wie PBS nur ohne Ca-und Mg-Salze

PEG: Polyethylenglykol

PFU: "plaques forming units"

rATP: ribo ATP

RPMI-Medium: Roswell-Park-Memorial-Institute-Medium

SDS: Natriumdodecylsulfat

TE: wässrige Puffermischung, die pro Liter 10 mMol TrisHCl pH = 7,4 und 1 mMol EDTA enthält.

T4-PNK: T4-Polynucleotidkinase

Tris: Trishydroxymethylaminomethan

TWEEN 20: Polyoxyethylen(20)-sorbitanmonolaurat

U: Unit

uCi: Microcurie

ug: Mikrogramm

ul: Mikroliter

uM: wässrige Lösung, die pro Liter ein Mikromol der betreffenden Substanz enthält.

VP1,2,3,4: virales Hüllenprotein VP1,2,3,4.

VPO: virales Vorläuferprotein der Hüllenproteine VP2 und VP4

XC: Xylencyanol

## Ansprüche

1. DNA-Molekül, dadurch gekennzeichnet, daß es der gesamten oder Teilen der viralen RNA des Rhinovirusstammes HRV89 oder deren degenerierten Formen entspricht.

2. DNA-Molekül, dadurch gekennzeichnet, daß es eine DNA-Sequenz enthält, die für ein virales Protein mit der in Figur 4 gezeigten Aminosäuresequenz 1 bis 2164, für Teile oder für Allele hiervon, insbesondere für das virale Protein VP1, VP2, VP3, VP4, P2A, P2B, P2C, P3A, P3B oder P3C, für Teile oder für Allele hiervon oder für beliebige Kombinationen von mindestens 2 miteinander verknüpften viralen Proteinen, mit den Eigenschaften mindestens eines oder Teilen der viralen Proteine des Rhinovirusstammes HRV89 codiert, sowie deren degenerierte Formen.

3. DNA-Molekül, dadurch gekennzeichnet, daß es unter stringenten Bedingungen, die es erlauben, eine Homologie zu erkennen, die größer als 85% ist, mit einem DNA-Molekül nach einem der vorhergehenden Ansprüche hybridisiert und für ein virales Protein codiert, das die Eigenschaften mindestens eines oder Teilen der viralen Proteine des Rhinovirusstammes HRV89 aufweist.

4. Replizierbarer Expressionsvektor, dadurch gekennzeichnet, daß er in einem transformierten Wirtsorganismus oder einer transformierten Zellkultur eine DNA-Sequenz, die für ein virales Protein mit der in Figur 4 oder Figur 14 gezeigten Aminosäuresequenz, für Teile oder Allele hiervon, mit den Eigenschaften mindestens eines oder Teilen der viralen Proteine der Rhinovirusstämme HRV89 oder HRV2 codiert, vorzugsweise eine DNA-Sequenz nach einem der Ansprüche 1 bis 3 sowie deren degenerierte Formen exprimieren kann.

5. Replizierbarer Expressionsvektor nach Anspruch 4, dadurch gekennzeichnet, daß es der Vektor pKK89/2A, pRH969/1 oder pRH969/2 ist.

6. Transformierter Wirtsorganismus oder transformierte Zellkultur, dadurch gekennzeichnet, daß er oder sie ein durch eine DNA-Sequenz codiertes virales Protein mit der in Figur 4 oder Figur 14 gezeigten Aminosäuresequenz, ein Allel oder Teil der viralen Proteine der Rhinovirusstämme HRV89 oder HRV2, vorzugsweise eine DNA-Sequenz nach einem der Ansprüche 1 bis 3, sowie deren degenerierte Formen exprimieren kann.

7. Transformierter Wirtsorganismus oder transformierte Zellkultur nach Anspruch 6, dadurch gekennzeichnet, daß die DNA-Sequenz in einem replizierbaren Expressionsvektor nach einem der Ansprüche 4 oder 5 enthalten ist, wobei E.coli als transformierter Wirtsorganismus bevorzugt ist.

8. Polypeptid, dadurch gekennzeichnet, daß es durch ein DNA-Molekül nach einem der Ansprüche 1 bis 3 oder durch eine in den replizierbaren Expressionsvektoren nach einem der Ansprüche 4 oder 5 enthaltene DNA-Sequenz codiert wird und die Eigenschaft mindestens eines der in Figur 4 oder Figur 14 aufgeführten viralen Proteine aufweist.

9. Polypeptid, dadurch gekennzeichnet, daß es mindestens einem der in Figur 4 unter der Aminosäuresequenz 1 bis 2164 aufgeführten viralen Proteine, Teilen oder Allelen hiervon entspricht und frei von anderen Proteinen ist, vorzugsweise die Aminosäuresequenz des viralen Proteins VP1, VP2, VP3, P2A, P2B, P2C, P3A, P3B oder P3C oder Teile hiervon enthält und frei von anderen Proteinen ist oder daß mindestens zwei der genannten viralen Proteine in beliebiger Kombination miteinander verknüpft sind.

10. Polypeptid, dadurch gekennzeichnet, daß es ein Derivat des Polypeptids mit der in Figur 4 gezeigten Aminosäuresequenz 1 bis 2164 oder dessen Teilen ist, mit den Eigenschaften mindestens eines oder Teilen der viralen Proteine des Rhinovirusstammes HRV89.

11. Verfahren zur Herstellung eines Polypeptids nach einem der Ansprüche 8 bis 10, dadurch gekennzeichnet, daß ein geeigneter Wirtsorganismus oder eine geeignete Zellkultur, vorzugsweise ein Wirtsorganismus oder eine Zellkultur nach einem der Ansprüche 6 oder 7 mit genetischen Informationen codierend für ein Polypeptid nach einem der Ansprüche 8 bis 10, vorzugsweise mit genetischen Informationen, die in einem replizierbaren Expressionsvektor nach einem der Ansprüche 4 oder 5 enthalten sind, transformiert wird, daß der so transformierte Wirtsorganismus oder die so transformierte Zellkultur unter geeigneten Bedingungen kulitviert wird und daß das exprimierte Polypeptid isoliert und gereinigt wird.

12. Hybrid-Zellinie, dadurch gekennzeichnet daß sie monoklonale Antikörper gegen eines der Polypeptide nach einem der Ansprüche 8 bis 10 sezerniert.

13. Monoklonale Antikörper dadurch gekennzeichnet, daß sie spezifische die Wirkung der Polypeptide nach einem der Ansprüch 8 bis 10 ganz oder teilweise neutralisieren und/oder spezifisch an eines der besagten Polypeptide, vorzugsweise auch an die denaturierten Formen ihrer Antigene binden.

14. Verwendung der monoklonalen Antikörper nach Anspruch 13 zur qualitativen und/oder quantitativen Bestimmung eines der Polypeptide nach einem der Ansprüche 8 bis 10, zur Reinigung eines der Polypeptide nach einem der Ansprüche 8 bis 10 und/oder zu therapeutischen oder diagnostischen Zwecken.

15. Test Kit zur Bestimmung von Polypeptiden nach einem der Ansprüche 8 bis 10, dadurch gekennzeichnet, daß er monoklonale Antikörper nach Anspruch 13 enthält.

16. Verfahren zur Herstellung von monoklonalen Antikörpern nach Anspruch 13, dadurch gekennzeichnet, daß Wirtstiere mit einem der Polypeptide nach einem der Ansprüche 8 bis 10 immunisiert, B-Lymphozyten dieser Wirtstiere mit Myelomzellen fusioniert, die die monoklonalen Antikörper ausscheidenden Hybrid-Zellinien subkloniert und in vitro oder in vivo kultiviert werden.

17. Verwendung eines Polypeptids nach den Ansprüchen 8 bis 10 zur therapeutischen Behandlung, insbesondere zur therapeutischen Behandlung rhinoviraler Infektionen oder zur Prophylaxe rhinoviraler Infektionen oder zur Stimulierung des Immunsystems oder zur Bindung und/oder Blockierung der zellulären Rezeptoren für die Rhinoviren Stamm HRV89.

18. Arzneimittel, geeignet für die Verwendung der Polypeptide nach einem der Ansprüche 8 bis 10 gemäß Anspruch 17, dadurch gekennzeichnet, daß es neben pharmazeutisch inerten Hilfs-und/oder Trägerstoffen eine wirksame Menge mindestens eines der Polypeptide nach einem der Ansprüche 8 bis 10 enthält.

Fig. 1

Fig. 2

Fig. 3

Fig. 4 a    0 261 403

```
TTAAAACTGGGAGTGGGTTGTTCCCACTCACTCCACCCATGCGGTGTTGTACTCTGTTATTACGGTAACTTTGTACGCCAGTTTTTCCCACCCTTCCCCA
        10        20        30        40        50        60        70        80        90       100


TAATGTAACTTAGAAGTTTGTACAATATGACCAATAGGTGACAATCATCCAGACTGTCAAAGGTCAAGCACTTCTGTTTCCCCGGTCAATGAGGATATGC
       110       120       130       140       150       160       170       180       190       200


TTTACCCAAGGCAAAAACCTTAGAGATCGTTATCCCCACACTGCCTACACAGAGCCCAGTACCATTTTTGATATAATTGGGTTGGTCGCTCCCTGCAAAC
       210       220       230       240       250       260       270       280       290       300


CCAGCAGTAGACCTGGCAGATGAGGCTGGACATTCCCCACTGGCGACAGTGGTCCAGCCTGCGTGGCTGCCTGCTCACCCTTCTTGGGTGAGAAGCCTAA
       310       320       330       340       350       360       370       380       390       400


TTATTGACAAGGTGTGAAGAGCCGCGTGTGCTCAGTGTGCTTCCTCCGGCCCCTGAATGTGGCTAACCTTAACCCTGCAGCCGTTGCCCATAATCCAATG
       410       420       430       440       450       460       470       480       490       500


GGTTTGCGGTCGTAATGCGTAAGTGCGGGATGGGACCAACTACTTTGGGTGTCCGTGTTTCCTGTTTTTCTTTTGATTGCATTTTATGGTGACAATTTAT
       510       520       530       540       550       560       570       580       590       600
                      VP4
                      M  G  A  Q  V  S  R  Q  N  V  G  T  H  S  T  Q  N  S  V  S  N  G  S  S  L  N  Y
AGTGTATAGATTGTCATCATGGGTGCACAAGTATCTAGACAAAATGTTGGGACACACTCCACACAAAATTCAGTGAGCAATGGATCTAGCTTAAATTATT
       610       620       630       640       650       660       670       680       690       700


 F  N  I  N  Y  F  K  D  A  A  S  S  G  A  S  R  L  D  F  S  Q  D  P  S  K  F  T  D  P  V  K  D  V  L
TCAACATCAATTATTTTAAAGACGCAGCTTCAAGTGGTGCTTCTAGATTGGATTTTTCTCAAGACCCTAGTAAATTTACTGATCCTGTTAAAGATGTGTT
       710       720       730       740       750       760       770       780       790       800
                       VP2
  E  K  G  I  P  T  L  Q  S  P  T  V  E  A  C  G  Y  S  D  R  L  I  Q  I  T  R  G  D  S  T  I  T  S
AGAAAAGGGTATTCCAACACTTCAATCACCAACAGTTGAAGCTTGTGGTTATTCAGACAGACTAATACAGATAACCCGAGGAGATTCCACTATAACATCC
       810       820       830       840       850       860       870       880       890       900


  Q  D  T  A  N  A  V  V  A  Y  G  V  W  P  S  Y  L  T  P  D  D  A  T  A  I  D  K  P  T  Q  P  D  T
CAAGATACTGCAAATGCAGTTGTTGCTTATGGTGTGTGGCCATCATACTTGACTCCAGATGATGCGACTGCTATTGACAAACCCACACAACCTGATACAT
       910       920       930       940       950       960       970       980       990      1000


  S  S  N  R  F  Y  T  L  D  S  R  S  W  T  S  A  S  S  G  W  W  W  K  L  P  D  A  L  K  N  M  G  I  F
CATCCAACAGATTCTACACCTTGGACAGTCGTTCTTGGACATCTGCCTCATCTGGATGGTGGTGGAAATTGCCTGATGCCCTTAAAAACATGGGTATATT
      1010      1020      1030      1040      1050      1060      1070      1080      1090      1100


  G  E  N  M  F  Y  H  F  L  G  R  S  G  Y  T  I  H  V  Q  C  N  S  S  K  F  H  Q  G  L  L  I  V  A
TGGTGAAAATATGTTTTACCATTTTCTAGGGAGATCTGGATACACAATACATGTACAATGTAATTCTAGCAAGTTTCATCAGGGTTTATTAATAGTTGCC
      1110      1120      1130      1140      1150      1160      1170      1180      1190      1200


  A  I  P  E  H  Q  L  A  S  A  T  S  G  N  V  S  V  G  Y  N  H  T  H  P  G  E  Q  G  R  E  V  V  P
GCCATCCCAGAACATCAATTGGCATCTGCAACAAGTGGAAATGTATCAGTCGGGTACAATCACACCCACCCAGGTGAGCAAGGTAGAGAAGTAGTACCAT
      1210      1220      1230      1240      1250      1260      1270      1280      1290      1300


  S  R  T  S  S  D  N  K  R  P  S  D  D  S  W  L  N  F  D  G  T  L  L  G  N  L  P  I  Y  P  H  Q  Y  I
CACGGACATCTAGTGATAATAAAAGACCTAGTGATGACAGTTGGTTAAATTTTGATGGAACATTACTTGGTAACTTACCTATTTATCCCCACCAATACAT
      1310      1320      1330      1340      1350      1360      1370      1380      1390      1400


  N  L  R  T  N  N  S  A  T  L  I  L  P  Y  V  N  A  V  P  M  D  S  M  L  R  H  N  N  W  S  L  V  I
TAATCTAAGGACTAACAATTCAGCTACCCTTATTTTACCTTATGTCAATGCTGTACCAATGGACTCTATGCTTAGACATAATAATTGGAGCTTGGTTATA
      1410      1420      1430      1440      1450      1460      1470      1480      1490      1500
```

Fig. 4 b        0 261 403

```
I  P  I  C  P  L  Q  V  Q  P  G  G  T  Q  S  I  P  I  T  V  S  I  S  P  M  F  S  E  F  S  G  P  R
ATCCCAATATGCCCTCTTCAGGTCCAACCTGGGGGGACACAATCCATACCTATAACAGTATCAATTAGCCCTATGTTTTCAGAATTTTCAGGGCCAAGAA
```

```
                                VP3
S  K  V  V  F  S  T  T  Q  G  L  P  V  M  L  I  P  G  S  G  Q  F  L  I  T  D  D  T  Q  S  P  S  A  F
GTAAGGTTGTGTTTAGTACCACTCAGGGTTTACCAGTTATGTTAACACCTGGATCTGGGCAATTCTTAACAACTGATGATACTCAATCCCCATCAGCGTT
```

```
P  Y  F  H  P  T  K  E  I  F  I  P  G  Q  V  R  N  L  I  E  M  C  Q  V  D  T  L  I  P  V  N  N  T
TCCATACTTCCACCCGACCAAGGAAATATTTATACCTGGACAAGTTAGGAATTTAATTGAAATGTGCCAAGTTGACACACTCATTCCTGTTAACAATACA
```

```
Q  E  N  V  R  S  V  N  M  Y  T  V  D  L  R  T  Q  V  D  L  A  K  E  V  F  S  I  P  V  D  I  A  S
CAGGAAAATGTAAGATCTGTGAATATGTACACTGTTGATTTACGCACACAAGTTGATTTAGCTAAAGAAGTCTTTTCTATACCAGTAGATATTGCCTCAC
```

```
Q  P  L  A  T  T  L  I  G  E  L  A  S  Y  Y  T  H  W  T  G  S  L  R  F  S  F  M  F  C  G  S  A  S  S
AACCTTTAGCCACTACTCTCATAGGAGAACTTGCAAGCTATTACACACACTGGACTGGTAGTCTGCGCTTTAGCTTTATGTTTTGTGGTTCTGCTAGCTC
```

```
T  L  K  L  L  I  A  Y  T  P  P  G  V  G  K  P  K  S  R  R  E  A  M  L  G  T  H  L  V  W  D  V  G
TACTTTGAAACTATTAATTGCATACACTCCTCCTGGTGTTGGAAAACCTAAATCCAGGAGAGAAGCCATGCTTGGTACACATTTAGTGTGGGATGTGGGG
```

```
L  Q  S  T  A  S  L  V  V  P  W  V  S  A  S  H  F  R  F  T  T  P  D  T  Y  S  S  A  G  Y  I  T  C
TTGCAGTCCACCGCCTCACTAGTTGTACCATGGGTTAGTGCTAGCCATTTTAGATTCACTACACCTGACACATATTCCTCAGCTGGTTATATTACATGCT
```

```
W  Y  Q  T  N  F  V  V  P  D  S  T  P  D  N  A  K  M  V  C  M  V  S  A  C  K  D  F  C  L  R  L  A  R
GGTACCAGACCAACTTTGTAGTACCTGATAGTACTCCAGATAACGCCAAAATGGTGTGCATGGTTTCTGCATGCAAAGATTTTTGCTTAAGATTAGCCAG
```

```
                        VP1
D  T  N  L  H  T  Q  E  G  V  L  T  Q  N  P  V  E  N  Y  I  D  S  V  L  N  E  V  L  V  V  P  N  I
AGATACTAACCTACACACACAAGAAGGAGTACTCACACAAAACCCAGTTGAAAATTATATAGATAGTGTATTAAATGAAGTTCTTGTGGTGCCAAATATC
```

```
Q  P  S  T  S  V  S  S  H  A  A  P  A  L  D  A  A  E  T  G  H  T  S  S  V  Q  P  E  D  M  I  E  T
CAACCTAGCACATCTGTGTCAAGTCATGCAGCGCCTGCATTGGATGCTGCGGAAACCGGACACACCAGCTCTGTTCAACCTGAAGATATGATTGAAACTA
```

```
R  Y  V  I  T  D  Q  T  R  D  E  T  S  I  E  S  F  L  G  R  S  G  C  I  A  M  I  E  F  N  T  S  S  D
GATATGTTATAACTGATCAAACAAGGGATGAAACAAGTATTGAGAGTTTCTTAGGTAGGTCAGGGTGTATCGCTATGATAGAATTTAATACAAGTAGTGA
```

```
K  T  E  H  D  K  I  G  K  G  F  K  T  W  K  V  S  L  Q  E  M  A  Q  I  R  R  K  Y  E  L  F  T  Y
TAAAACTGAACATGATAAAATTGGTAAAGGATTCAAAACATGGAAGGTTAGTCTTCAAGAAATGGCACAAATCAGAAGAAAATATGAATTATTCACATAT
```

```
T  R  F  D  S  E  I  T  I  V  T  A  A  A  A  Q  G  N  D  S  G  H  I  V  L  Q  F  M  Y  V  P  P  G
ACAAGATTTGATTCAGAGATAACAATAGTCACTGCAGCCGCAGCTCAAGGAAATGATAGTGGACATATAGTATTGCAATTTATGTATGTACCCCCAGGAG
```

```
A  P  V  P  E  K  R  D  D  Y  T  W  Q  S  G  T  N  A  S  V  F  W  Q  E  G  Q  P  Y  P  R  F  T  I  P
CACCTGTCCCCGAAAAACGTGATGATTACACATGGCAATCAGGAACAAATGCATCTGTGTTCTGGCAAGAAGGACAACCATACCCCAGATTCACAATCCC
```

```
F  M  S  I  A  S  A  Y  Y  M  F  Y  D  G  Y  D  G  D  S  A  A  S  K  Y  G  S  V  V  T  N  D  M  G
TTTTATGAGCATTGCATCAGCCTATTACATGTTTTATGATGGTTATGATGGTGATAGTGCAGCATCAAAATACGGTTCTGTAGTCACTAATGATATGGGA
```

Fig. 4 c

```
      T I C V R I V T S N Q K H D S N I V C R I Y H K A K H I K A W C P
ACCATATGTGTTAGAATAGTGACATCCAACCAAAAACACGATTCAAATATTGTGTGCCGCATTTACCACAAGGCCAAACATATAAAAGCATGGTGTCCTC
     3010      3020      3030      3040      3050      3060      3070      3080      3090      3100

      R P P R A V A Y Q H T H S T N Y I P S N G E A T T Q I K T R P D V F
GCCCACCAAGGGCTGTTGCCTATCAACACACACACTCAACCAATTACATACCATCCAATGGTGAGGCCACAACTCAGATTAAAACCAGACCTGATGTTTT
     3110      3120      3130      3140      3150      3160      3170      3180      3190      3200

                                         ↓   P2-A
      T V T N V G P S S M F▽V H V G N L I Y R N L H L F N S D L D D S I
TACCGTTACAAACGTCGGACCATCTAGTATGTTTGTACATGTGGGTAACTTAATCTATAGAAATCTTCATCTCTTTAATTCTGATCTTGATGATTCTATT
     3210      3220      3230      3240      3250      3260      3270      3280      3290      3300

      L V S Y S S D L I I Y R T N T E G N D V I P N C D C T E C T Y Y C
CTTGTATCATACTCCAGTGATCTAATCATATATCGAACAAACACTGAAGGTAATGATGTGATCCCTAATTGTGATTGCACTGAATGTACATATTACTGCC
     3310      3320      3330      3340      3350      3360      3370      3380      3390      3400

      H H K D R Y F P I R V T A H D W Y E I Q E S E Y Y P K H I Q Y N L L
ACCACAAAGATAGGTATTTTCCTATCAGAGTTACTGCACATGATTGGTATGAGATTCAAGAATCAGAATATTACCCAAAACATATCCAATATAATCTCCT
     3410      3420      3430      3440      3450      3460      3470      3480      3490      3500

      I G E G P C E P G D C G G K L L C K H G V I G M I T A G G E G H V
GATTGGAGAGGGTCCTTGTGAACCAGGAGATTGTGGAGGAAAACTATTGTGTAAACATGGTGTTATAGGTATGATTACAGCTGGAGGTGAAGGTCACGTT
     3510      3520      3530      3540      3550      3560      3570      3580      3590      3600

                                   ↓   P2-B
      A F I D L R K F Q C A E E Q▽G L S D Y V E H L G Q V F G V G F V D
GCTTTTATTGACCTGAGAAAATTCCAGTGTGCTGAGGAGCAAGGGTTATCTGATTATGTGGAACATCTTGGTCAAGTCTTTGGTGTAGGCTTCGTAGACA
     3610      3620      3630      3640      3650      3660      3670      3680      3690      3700

      S I K Q Q V N F I N P T S K I G S K V I K W L L R I V S A M I I M V
GCATCAAACAACAGGTAAACTTTATCAACCCCACTAGTAAAAATTGGTTCAAAAGTGATTAAATGGTTGTTGAGGATAGTTTCAGCTATGATAATAATGGT
     3710      3720      3730      3740      3750      3760      3770      3780      3790      3800

      R N S S D P Q T V I A T L T L L G C S G S P W R F L K E K L C A W
AAGGAATAGTTCTGATCCACAAACTGTAATTGCCACTCTCACCCTTCTAGGTTGTTCAGGCTCACCATGGAGGTTTCTTAAAGAGAAACTCTGTGCGTGG
     3810      3820      3830      3840      3850      3860      3870      3880      3890      3900

                                ↓   P2-C
      L Q L S Y V H K Q▽S D S W L K K F T E A C N A A R G L E W I G Q K
CTCCAGCTTAGCTATGTACATAAGCAGTCTGATTCATGGCTCAAGAAATTTACTGAAGCGTGTAACGCAGCACGTGGGCTAGAGTGGATTGGACAAAAGA
     3910      3920      3930      3940      3950      3960      3970      3980      3990      4000

      I S K F I D W I K S M L P Q A Q L K I D Y L T K L K Q L N L L E K Q
TATCTAAATTTATAGATTGGATAAAGAGTATGTTACCACAGGCTCAATTGAAAATTGATTACCTAACCAAATTAAAACAACTTAATCTCTTAGAGAAACA
     4010      4020      4030      4040      4050      4060      4070      4080      4090      4100

      I E T I R L A P A S V Q E K I F I E I N T L H D L S L K F L P L Y
AATAGAAACAATTAGACTTGCACCTGCTAGTGTTCAGGAGAAAATTTTCATTGAAATAAACACCCTTCATGATTTATCCTTAAAATTCTTACCACTGTAT
     4110      4120      4130      4140      4150      4160      4170      4180      4190      4200

      A S E A R R I K N L Y I K C S N V I K G G K R N E P V A V L I H G
GCATCTGAAGCACGTAGAATTAAGAATTTATATATCAAATGCAGTAATGTTATTAAAGGGGGGAAAGAGGAATGAACCAGTTGCAGTTCTAATACATGGTT
     4210      4220      4230      4240      4250      4260      4270      4280      4290      4300

      S P G T G K S L A T S V L A R M L T V E T D I Y S L P P D P K Y F D
CTCCTGGTACTGGAAAATCTCTTGCCACTTCTGTTCTTGCTCGAATGCTAACTGTTGAGACTGATATATATTCTTTGCCCCCAGATCCTAAATATTTTGA
     4310      4320      4330    . 4340      4350      4360      4370      4380      4390      4400

      G Y D Q Q S V V I M D D I M Q N P S G E D M T L F C Q M V S S V P
TGGGTATGATCAACAGAGTGTTGTTATCATGGATGATATCATGCAAAATCCTAGTGGTGAAGACATGACTTTGTTTTGCCAAATGGTATCGAGTGTCCCT
     4410      4420      4430      4440      4450      4460      4470      4480      4490      4500
```

Fig. 4 d

```
        F I P P M A D L P D K G K P F T S K F V L A S T N H T L L T P P T
        TTCATACCTCCTATGGCAGATCTTCCAGATAAAGGAAAACCATTTACATCCAAGTTTGTACTTGCAAGCACTAATCACACTCTACTAACACCACCAACAG
          4510      4520      4530      4540      4550      4560      4570      4580      4590      4600

        V S S L P A M A R R F Y F D L D I Q V K K E Y L L D G K L D I A K S
        TATCTTCATTACCAGCAATGGCAAGAAGGTTTTACTTTGATCTAGACATTCAAGTTAAGAAAGAGTATCTTTTAGATGGCAAACTAGATATAGCAAAAAG
          4610      4620      4630      4640      4650      4660      4670      4680      4690      4700

         F R P C D V N I K I G N A K C C P F I C G K A V E F K D R N S C T
         CTTTCGACCATGTGATGTTAATATTAAAATAGGCAATGCTAAGTGCTGTCCATTTATCTGTGGAAAAGCTGTAGAGTTTAAAGATAGAAATTCATGTACA
           4710      4720      4730      4740      4750      4760      4770      4780      4790      4800

                                                                                    ↓     P3-A
        T L S L S Q L Y S H I K E E D R R R S S A A Q A M E A I F Q▽G I D
        ACCTTGTCTTTATCTCAATTGTATAGTCATATAAAGGAAGAAGATAGGGAAGAAGCAGTGCAGCACAAGCAATGGAGGCTATATTTCAAGGTATAGACC
          4810      4820      4830      4840      4850      4860      4870      4880      4890      4900

         L Q S P P P P A I A D L L R S V K T P E I I K Y C Q D N N W I V P A
         TCCAATCTCCTCCACCTCCAGCCATAGCTGACCTCCTTAGGTCTGTGAAAACACCAGAGATCATTAAGTATTGCCAAGATAATAATTGGATTGTTCCAGC
           4910      4920      4930      4940      4950      4960      4970      4980      4990      5000

          E C S I E R D L G I A N M T I G I I A N V V S I V G V I Y I I Y K
          AGAGTGTTCTATTGAAAGAGATTTAGGGATAGCAAATATGACTATAGGTATAATAGCTAATGTGGTCTCTATAGTAGGTGTTATCTATATAATTTATAAA
            5010      5020      5030      5040      5050      5060      5070      5080      5090      5100

                      ↓     VPg                                                   ↓     PROTEASE
          L F C T L Q▽G P Y S G E P K P K S R A P E R R V V T Q▽G P E E E F
          TTGTTCTGTACACTTCAGGGTCCATACTCAGGGGAACCTAAACCCAAAAGCAGAGCTCCAGAGAGAAGAGTAGTTACTCAGGGCCCAGAGGAAGAGTTTG
            5110      5120      5130      5140      5150      5160      5170      5180      5190      5200

         G R S L L K H N C C V V T T D K G K F T G L G I Y D Q V M V L P T H
         GTCGCTCACTACTCAAACATAATTGCTGTGTTGTGACAACCGACAAAGGCAAATTCACAGGTCTTGGCATATATGACCAAGTCATGGTACTTCCAACACA
           5210      5220      5230      5240      5250      5260      5270      5280      5290      5300

          S D P G S E I L V D G V K V K V S D S Y D L H N H E G V K L E I T
          TTCTGACCCAGGCTCTGAGATCTTGGTAGATGGAGTAAAAGTTAAGGTCTCTGATTCCTATGATTTGCATAACCATGAGGGTGTTAAGCTAGAGATCACA
            5310      5320      5330      5340      5350      5360      5370      5380      5390      5400

          V V K L I R N E K F K D I R K Y L P S R E D D Y P A C N L A L L A
          GTTGTGAAATTAATTAGAAATGAGAAGTTTAAAGACATCAGAAAATATTTACCCTCACGTGAAGATGACTATCCTGCTTGTAACCTTGCCTTACTAGCTA
            5410      5420      5430      5440      5450      5460      5470      5480      5490      5500

          N Q D E P T I I S V G D A V S Y G N I L L S G T N T A R M I K Y H Y
          ATCAAGATGAGCCAACAATAATAAGTGTTGGTGATGCAGTATCTTATGGTAACATCTTATTGAGTGGTACCAATACTGCACGAATGATCAAGTACCATTA
            5510      5520      5530      5540      5550      5560      5570      5580      5590      5600

           P T K A G Y C G G V L Y K V G S I L G I H V G G N G R D G F S A M
           CCCGACAAAAGCTGGATATTGTGGGGGTGTTTTGTACAAGGTTGGCTCTATTCTTGGTATACATGTTGGTGGCAATGGTAGAGATGGATTTTCTGCAATG
             5610      5620      5630      5640      5650      5660      5670      5680      5690      5700

                     ↓     POLYMERASE
           L L K S Y F G E T Q▽G L I T K E L P V S V K N L P S V H V S S K T
           CTTCTCAAATCTTATTTTGGTGAAACCCAGGGTTTAATCACTAAAGAACTTCCTGTATCTGTAAAGAACTTACCATCCGTACATGTTTCATCTAAAACCC
             5710      5720      5730      5740      5750      5760      5770      5780      5790      5800

          R L Q P S V F H D V F P G T K E P A V L S S N D P R L E T D F D S A
          GACTACAACCTAGTGTTTTTCATGATGTTTTCCCTGGAACAAAAGAGCCTGCAGTTCTTAGTAGTAATGATCCAAGACTAGAAACTGACTTTGACTCAGC
            5810      5820      5830      5840      5850      5860      5870      5880      5890      5900

           L F S K Y K G N P A C Q V T P H M K I A V A H Y A A Q L S T L D I
           ACTTTTCTCCAAATATAAAGGTAATCCTGCTTGTCAAGTGACCCCACACATGAAAATTGCTGTAGCACATTATGCAGCACAGTTATCTACACTAGACATA
             5910      5920      5930      5940      5950      5960      5970      5980      5990      6000
```

Fig. 4  e                    0 261 403                    : : 20·08·87

```
  N P Q P L S L E E S V F G I E G L E A L D L N T S A G F P Y V S L
AATCCTCAACCCCTTTCATTGGAAGAGAGTGTGTTTGGTATTGAGGGATTAGAAGCTTTGGATTTAAATACTAGTGCAGGATTTCCTTATGTTTCACTGG
    6010      6020      6030      6040      6050      6060      6070      6080      6090      6100

  G I K K K D L I D K K T K D I T K L R K A I D E Y G I D L P M V T F
GAATAAAGAAGAAAGATCTTATAGATAAAAAGACCAAAGACATCACAAAACTTAGGAAAGCAATTGATGAATATGGTATTGATTTGCCTATGGTTACTTT
    6110      6120      6130      6140      6150      6160      6170      6180      6190      6200

   L K D E L R K K E K I K D G K T R V I E A N S V N D T V L F R S V
TCTGAAAGATGAACTTAGAAAGAAGGAAAAAATAAAAGATGGAAAGACTAGAGTCATAGAAGCTAATAGTGTGAATGATACTGTGTTATTCAGAAGTGTA
    6210      6220      6230      6240      6250      6260      6270      6280      6290      6300

   F G N L F S A F H K N P G I V T G S A V G C D P E V F W S T I P L
TTTGGAAATCTTTTCTCTGCTTTCCACAAAAACCCAGGTATAGTCACTGGTTCAGCAGTAGGGTGTGACCCTGAAGTATTCTGGTCAACTATACCTCTCA
    6310      6320      6330      6340      6350      6360      6370      6380      6390      6400

   M L D G E C L M A F D Y S N Y D G S L H P V W F K C L S M L L E D I
TGCTAGATGGAGAATGTTTAATGGCTTTTGATTATTCAAACTATGATGGTAGCCTACATCCCGTTTGGTTTAAATGTCTTAGTATGCTCTTAGAAGACAT
    6410      6420      6430      6440      6450      6460      6470      6480      6490      6500

    G F S S Q L I N Q I C N S K H I Y K S K Y Y E V E G G M P S G C A
AGGTTTCTCCTCTCAACTTATTAACCAGATCTGTAACTCTAAACATATATACAAATCTAAGTATTATGAAGTGGAAGGAGGTATGCCATCTGGATGTGCT
    6510      6520      6530      6540      6550      6560      6570      6580      6590      6600

   G T S I F N T I I N N I I I R T L V L D A Y K N I D L D K L K I L
GGTACTAGTATTTTTAATACAATAATCAACAATATTATCATTAGAACTTTGGTACTAGATGCTTATAAGAACATAGATCTAGATAAACTGAAAATCTTAG
    6610      6620      6630      6640      6650      6660      6670      6680      6690      6700

   A Y G D D V I F S Y N F K L D M A V L A K E G E K Y G L T I T P A D
CATATGGGGATGATGTCATCTTTTCTTATAATTTTAAACTTGACATGGCAGTTCTTGCCAAAGAAGGAGAAAAATATGGACTAACAATCACCCCTGCTGA
    6710      6720      6730      6740      6750      6760      6770      6780      6790      6800

   K S D V F Q E L T Y K N V T F L K R G F R A D E R H S F L I H P T
TAAGTCTGATGTTTCCAAGAATTCACCTATAAAAATGTAACTTTTCTTAAAAGAGGATTCAGAGCTGATGAGCGCCACTCTTTCCTTATACACCCTACC
    6810      6820      6830      6840      6850      6860      6870      6880      6890      6900

   F P V A E I H D S I R W T K N P S C M Q E H V L S L C H L M W H N
TTTCCTGTGGCTGAGATTCATGACTCCATCAGATGGACCAAAAACCCTTCATGTATGCAGGAACACGTGCTATCTTTGTGTCATTTAATGTGGCATAATG
    6910      6920      6930      6940      6950      6960      6970      6980      6990      7000

   G R H A Y Q E F I K G I R S V S A G R A L Y I P A Y E V L E H E W Y
GTAGACATGCATACCAGGAATTCATTAAAGGTATACGCAGTGTATCTGCCGGTCGGGCACTGTATATACCAGCTTATGAAGTTCTTGAACATGAATGGTA
    7010      7020      7030      7040      7050      7060      7070      7080      7090      7100

   E K F *
TGAAAAATTTTAGATATAAAACTGTTAAATATAGCTAGTTTATTAGTTTTAT poly (A)
    7110      7120      7130      7140      7150
```

Fig. 5

0 261 403

20·03·87

|  | HRV89/HRV2 | HRV89/HRV14 | 89/POLIOIA |
|---|---|---|---|
| VP4 | 94 | 54 | 59 |
| VP2 | 73 | 57 | 50 |
| VP3 | 66 | 51 | 47 |
| VP1 | 62 | 38 | 37 |
| P2-A | 85 | 36 | 36 |
| P2-B | 64 | 45 | 44 |
| P2-C | 72 | 49 | 50 |
| P3-A | 65 | 40 | 32 |
| VPg | 86 | 57 | 36 |
| Protease | 75 | 52 | 36 |
| Polymerase | 72 | 58 | 57 |

Fig. 6

VP1    N P V E N Y I D . V L N E V L V V P ...

VP2    S P T V E A . G Y S D . L I Q ...

VP3    G L P V M L T P G . . . F L T ...

# Fig. 7

0 261 403

a) 5'-AGCTTAAAGATGAGCT-3'
b) 5'-CATCTTTA-3'

pER103  Ap$^r$  Tc$^r$  p/o  ori

a) HindIII
b) Phosphatase

Kinase

a) Ligase
b) SacI
c) Ligase

5'...AGGAGGTTTAAGCTTAAAGATGAGCTCATCTTTAAGCTT...3'
3'...TCCTCCAAATTCGAATTTCTACTCGAGTAGAAATTCGAA...5'

p/o  RBS  HindIII  SacI  HindIII

pRH100  Ap$^r$  Tc$^r$  ori

H: HindIII, S: SacI
Ap$^r$: Ampicillin resistance gene
Tc$^r$: Tetracyclin resistance gene
ori: origin of replication
RBS: ribosome binding site
p/o: tryptophan promoter/operator

Fig. 8

```
                          Eco RI
                         ━━━━━━━━
                            |
.......cgtcttcaagaaTTCACGCTGATCGCTAAAACATTGTGCAAAAAGAGGGTTGACTTT
                            ↑
                          4363              ◄---------- trp-Promotor -----

                    ◄- - - - - - - - - - - ►
                     GAATTCACGCTGATCGC
                       Sequenzprimer
```

```
              Hpa I                                        Hind III
             ━━━━━━                                       ━━━━━━━━━━
                |                                              |
GCCTTCGCGAACCAGTTAACTAGTACACAAGTTCACGGCAACGGTAAGGAGGTTTAAGCTTAAAG

-------- trp-Promotor/Operator ------------►◄---- RBS ----►
```

```
    Sac I      Hind III
   ━━━━━━━     ━━━━━━━━━
      |           |
ATGAGCTCATCTTTaagctttaatgcggtag..........

Startkodon    29
              ↑
```

Fig. 9

|XhoII

N Y F K D A A S N G A S K L E F T G D P S K F T D P V K D V L E K
AATTATTTCAAAGATGCTGCTTCAAATGGTGCATCAAAACTGGAATTCACACAAGATCCTAGTAAATTTACTGACCCAGTTAAGGATGTTTTGGAAAAGG
710    720    730    740    750    760    770    780    790    800

VP2
G I P T L Q S P T V E A C G Y S D R I I Q I T R G D S T I T S Q D V
GAATACCAACACTACAGTCCCCCACAGTGGAGGCTTGTGGATACTCTGATAGGATTATACAGATTACCAGAGGAGATTCAACCATAACCTCACAAGATGT
810    820    830    840    850    860    870    880    890    900

|Ball

A N A I V A Y G V W P H Y L S S K D A S A I D K P S Q P D T S S N
GGCTAATGCTATCGTTGCGTATGGTGTTTGGCCACATTATCTATCCTCCAAGGATGCCTCTGCAATTGATAAACCCTCTCAACCAGATACATCTTCTAAT
910    920    930    940    950    960    970    980    990    1000

R F Y T L R S V T W S S S S K G W W W K L P D A L K D M G I F G E
AGATTTTATACTCTAAGGAGTGTGACCTGGAGCAGTTCCTCAAAGGGTTGGTGGTGGAAACTACCTGATGCACTCAAGGACATGGGTATTTTTGGTGAAA
1010    1020    1030    1040    1050    1060    1070    1080    1090    1100

N M F Y H Y L G R S G Y T I H V Q C N A S K F H Q G T L I V A L I P
ACATGTTTTATCATTACCTGGGTAGGAGTGGATACACAATACATGTGCAGTGTAATGCTAGTAAATTTCACCAGGGTACACTAATTGTTGCTCTGATACC
1110    1120    1130    1140    1150    1160    1170    1180    1190    1200

E H Q I A S A L H G N V N V G Y N Y T H P G E T G R E V K A E T R
TGAGCATCAGATTGCAAGTGCCTTACATGGCAATGTGAATGTTGGTTACAACTACACACACCCAGGTGAAACAGGCAGGGAAGTTAAAGCTGAGACGAGA
1210    1220    1230    1240    1250    1260    1270    1280    1290    1300

L N P D L Q P T E E Y W L N F D G T L L G N I T I F P H Q F I N L
TTGAATCCTGATCTACAACCTACTGAAGAGTATTGGCTAAACTTTGATGGGACACTCCTTGGAAATATTACCATATTCCCTCATCAATTTATCAACTTGA
1310    1320    1330    1340    1350    1360    1370    1380    1390    1400

R S N N S A T I I A P Y V N A V P M D S M R S H N N W S L V I I P I
GGAGTAATAATTCTGCCACAATAATTGCCCCTTATGTCAATGCAGTTCCTATGGATTCAATGCGGAGCCACAATAATTGGAGTTTGGTAATAATACCAAT
1410    1420    1430    1440    1450    1460    1470    1480    1490    1500
                                                                    |BssHII

C P L E T S S A I N T I P I T I S I S P M C A E F S G A R A K R Q
ATGTCCCCTTGAGACATCAAGTGCAATTAACACAATACCTATTACAATATCTATAAGCCCCATGTGTGCAGAGTTTTCCGGCGCGCGTGCCAAGCGTCAA
1510    1520    1530    1540    1550    1560    1570    1580    1590    1600

VP3
G L P V F I T P G S G Q F L T T D D F G S P C A L P W Y H P T K E
GGATTACCAGTTTTCATCACACCAGGTTCAGGACAGTTTTTGACAACAGATGATTTCCAATCCCCATGTGCACTTCCCTGGTATCACCCAACTAAGGAAA
1610    1620    1630    1640    1650    1660    1670    1680    1690    1700

                                        |Acc I
I S I P G E V K N L V E I C Q V D S L V F I N N T D T Y I N S E N M
TTTCTATTCCAGGTGAGGTTAAAAATTTGGTTGAAATTTGTCAAGTAGACAGCCTAGTACCAATAAATAACACTGACACCTACATCAATAGTGAAAATAT
1710    1720    1730    1740    1750    1760    1770    1780    1790    1800

                        |XhoII
Y S V V L Q S S I N A P D K I F S I R T D V A S Q P L A T T T L I G
GTATTCTGTTGTATTGCAATCATCAATTAATGCACCAGATAAGATCTTTTCTATTCGAACAGATGTTGCTTCCCAACCTTTAGCTACTACTTTGATTGGT
1810    1820    1830    1840    1850    1860    1870    1880    1890    1900

E I S S Y F T H W T G S L R F S F M F C G T A N T T V K L L L A Y
GAGATATCTAGCTATTTCACCCACTGGACAGGGAGTCTCCGTTTCAGCTTCATGTTTTGTGGTACTGCCAACACTACTGTTAAGCTTTTGTTGGCATACA
1910    1920    1930    1940    1950    1960    1970    1980    1990    2000

Fig. 10

0 261 403

Fig. 11

0 261 403

Fig. 12

```
    S  P  T  V  E  A  C  G  Y  S  D  R  I  I  Q  I  T  R  G  D  S  T  I  T  S  Q  D  V  A  N  A  I  V
TCCCCCACAGTGGAGGCTTGTGGATACTCTGATAGGATTATACAGATTACCAGAGGAGATTCAACCATAACCTCACAAGATGTGGCTAATGCTATCGTTG
        10        20        30        40        50        60        70        80        90        100

    A  Y  G  V  W  P  H  Y  L  S  S  K  D  A  S  A  I  D  K  P  S  G  P  D  T  S  S  N  R  F  Y  T  L  R
CGTATGGTGTTTGGCCACATTATCTATCCTCCAAGGATGCCTCTGCAATTGATAAACCCTCTCAACCAGATACATCTTCTAATAGATTTTATACTCTAAG
       110       120       130       140       150       160       170       180       190       200

    S  V  T  W  S  S  S  S  K  G  W  W  W  K  L  P  D  A  L  K  D  M  G  I  F  G  E  N  M  F  Y  H  Y
GAGTGTGACCTGGAGCAGTTCCTCAAAGGGTTGGTGGTGGAAACTACCTGATGCACTCAAGGACATGGGTATTTTTGGTGAAAACATGTTTTATCATTAC
       210       220       230       240       250       260       270       280       290       300

    L  G  R  S  G  Y  T  I  H  V  Q  C  N  A  S  K  F  H  Q  G  T  L  I  V  A  L  I  P  E  H  Q  I  A
CTGGGTAGGAGTGGATACACAATACATGTGCAGTGTAATGCTAGTAAATTTCACCAGGGTACACTAATTGTTGCTCTGATACCTGAGCATCAGATTGCAA
       310       320       330       340       350       360       370       380       390       400

    S  A  L  H  G  N  V  N  V  G  Y  N  Y  T  H  P  G  E  T  G  R  E  V  K  A  E  T  R  L  N  P  D  L  Q
GTGCCTTACATGGCAATGTGAATGTTGGTTACAACTACACACACCCAGGTGAAACAGGCAGGGAAGTTAAAGCTGAGACGAGATTGAATCCTGATCTACA
       410       420       430       440       450       460       470       480       490       500

    P  T  E  E  Y  W  L  N  F  D  G  T  L  L  G  N  I  T  I  F  P  H  Q  F  I  N  L  R  S  N  N  S  A
ACCTACTGAAGAGTATTGGCTAAACTTTGATGGGACACTCCTTGGAAATATTACCATATTCCCTCATCAATTTATCAACTTGAGGAGTAATAATTCTGCC
       510       520       530       540       550       560       570       580       590       600

    T  I  I  A  P  Y  V  N  A  V  P  M  D  S  M  R  S  H  N  N  W  S  L  V  I  I  P  I  C  P  L  E  T
ACAATAATTGCCCCTTATGTCAATGCAGTTCCTATGGATTCAATGCGGAGCCACAATAATTGGAGTTTGGTAATAATACCAATATGTCCCCTTGAGACAT
       610       620       630       640       650       660       670       680       690       700

    S  S  A  I  N  T  I  P  I  T  I  S  I  S  P  M  C  A  E  F  S  G  A  R  A  K  R  Q
CAAGTGCAATTAACACAATACCTATTACAATATCTATAAGCCCCATGTGTGCAGAGTTTTCCGGCGCGCGTGCCAAGCGTCAATAG
       710       720       730       740       750       760       770       780       790       800
```

Fig. 13

HRV2 VP2

1                100                 200       261

Rsa I           Ssp I           BssH II

8F5 BINDING SITE

1240                   1280                            1320

aat gtt ggt tac aac tac aca cac cca ggt gaa aca ggc agg gaa gtt aaa gct gag acg aga ttg aat cct gat cta caa cct

N V G Y N Y T H P G E T G R E V K A E T R L N P D L Q P

622        513  527      532                    601  402

```
TTAAAACTGGATCCAGGTTGTTCCCACCTGGATTTCCCACAGGGAGTGGTACTCTGTTATTACGGTAACTTTGTACGCCAGTTTTATCTCCCTTCCCCCA
         10        20        30        40        50        60        70        80        90       100

TGTAACTTAGAAGTTTTTCACAAAGACCAATAGCCGGTAATCAGCCAGATTACTGAAGGTCAAGCACTTCTGTTTCCCCGGTCAATGTTGATATGCTCCA
        110       120       130       140       150       160       170       180       190       200

ACAGGGCAAAAACAACTGCGATCGTTAACCGCAAAGCGCCTACGCAAAGCTTAGTAGCATCTTTGAAATCGTTTGGCTGGTCGATCCGCCATTTCCCCTG
        210       220       230       240       250       260       270       280       290       300

GTAGACCTGGCAGATGAGGCTAGAAATACCCCACTGGCGACAGTGTTCTAGCCTGCGTGGCTGCCTGCACACCCTATGGGTGTGAAGCCAAACAATGGAC
        310       320       330       340       350       360       370       380       390       400

AAGGTGTGAAGAGCCCCGTGTGCTCGCTTTGAGTCCTCCGGCCCCTGAATGTGGCTAACCTTAACCCTGCAGCTAGAGCACGTAACCCAATGTGTATCTA
        410       420       430       440       450       460       470       480       490       500

GTCGTAATGAGCAATTGCGGGATGGGACCAACTACTTTGGGTGTCCGTGTTTCACTTTTTCCTTTATATTTGCTTATGGTGACAATATATACAATATATA
        510       520       530       540       550       560       570       580       590       600
                VP4
                   M  G  A  Q  V  S  R  Q  N  V  G  T  H  S  T  Q  N  S  V  S  N  G  S  S  L  N  Y  F  N  I
TATTGGCACCATGGGTGCACAGGTTTCAAGACAAAATGTTGGAACTCACTCCACGCAAAACTCTGTATCAAATGGGTCTAGTTTAAATTATTTTAACATC
        610       620       630       640       650       660       670       680       690       700

 N  Y  F  K  D  A  A  S  N  G  A  S  K  L  E  F  T  Q  D  P  S  K  F  T  D  P  V  K  D  V  L  E  K
AATTATTTCAAAGATGCTGCTTCAAATGGTGCATCAAAACTGGAATTCACACAAGATCCTAGTAAATTTACTGACCCAGTTAAGGATGTTTTGGAAAAGG
       .710       720       730       740       750       760       770        780       790       800
                              ↓    VP2
 G  I  P  T  L  Q  S  P  T  V  E  A  C  G  Y  S  D  R  I  I  Q  I  T  R  G  D  S  T  I  T  S  Q  D  V
GAATACCAACACTACAGTCCCCCACAGTGGAGGCTTGTGGATACTCTGATAGGATTATACAGATTACCAGAGGAGATTCAACCATAACCTCACAAGATGT
        810       820       830       840       850       860       870       880       890       900

 A  N  A  I  V  A  Y  G  V  W  P  H  Y  L  S  S  K  D  A  S  A  I  D  K  P  S  Q  P  D  T  S  S  N
GGCTAATGCTATCGTTGCGTATGGTGTTTGGCCACATTATCTATCCTCCAAGGATGCCTCTGCAATTGATAAACCCTCTCAACCAGATACATCTTCTAAT
        910       920       930       940       950       960     . 970       980       990      1000

 R  F  Y  T  L  R  S  V  T  W  S  S  S  S  K  G  W  W  W  K  L  P  D  A  L  K  D  M  G  I  F  G  E
AGATTTTATACTCTAAGGAGTGTGACCTGGAGCAGTTCCTCAAAGGGTTGGTGGTGGAAACTACCTGATGCACTCAAGGACATGGGTATTTTTGGTGAAA
       1010      1020      1030      1040      1050      1060      1070      1080      1090      1100

 N  M  F  Y  H  Y  L  G  R  S  G  Y  T  I  H  V  Q  C  N  A  S  K  F  H  Q  G  T  L  I  V  A  L  I  P
ACATGTTTTATCATTACCTGGGTAGGAGTGGATACACAATACATGTGCAGTGTAATGCTAGTAAATTTCACCAGGGTACACTAATTGTTGCTCTGATACC
       1110      1120      1130      1140      1150      1160      1170      1180      1190      1200

 E  H  Q  I  A  S  A  L  H  G  N  V  N  V  G  Y  N  Y  T  H  P  G  E  T  G  R  E  V  K  A  E  T  R
TGAGCATCAGATTGCAAGTGCCTTACATGGCAATGTGAATGTTGGTTACAACTACACACACCCAGGTGAAACAGGCAGGGAAGTTAAAGCTGAGACGAGA
       1210      1220      1230      1240      1250      1260      1270      1280      1290      1300

 L  N  P  D  L  Q  P  T  E  E  Y  W  L  N  F  D  G  T  L  L  G  N  I  T  I  F  P  H  Q  F  I  N  L
TTGAATCCTGATCTACAACCTACTGAAGAGTATTGGCTAAACTTTGATGGGACACTCCTTGGAAATATTACCATATTCCCTCATCAATTTATCAACTTGA
       1310      1320      1330      1340      1350      1360      1370      1380      1390      1400
```

Fig. 14b

```
    R S N N S A T I I A P Y V N A V P M D S M R S H N N W S L V I I P I
GGAGTAATAATTCTGCCACAATAATTGCCCCTTATGTCAATGCAGTTCCTATGGATTCAATGCGGAGCCACAATAATTGGAGTTTGGTAATAATACCAAT
    1410      1420      1430      1440      1450      1460      1470      1480      1490      1500

    C P L E T S S A I N T I P I T I S I S P M C A E F S G A R A K R Q
ATGTCCCCTTGAGACATCAAGTGCAATTAACACAATACCTATTACAATATCTATAAGCCCCATGTGTGCAGAGTTTTCCGGCGCGCGTGCCAAGCGTCAA
    1510      1520      1530      1540      1550      1560      1570      1580      1590      1600
   ↓  VP3
    G L P V F I T P G S G Q F L T T D D F Q S P C A L P W Y H P T K E
GGATTACCAGTTTTCATCACACCAGGTTCAGGACAGTTTTTTGACAACAGATGATTTCCAATCCCCATGTGCACTTCCCTGGTATCACCCAACTAAGGAAA
    1610      1620      1630      1640      1650      1660      1670      1680      1690      1700

    I S I P G E V K N L V E I C Q V D S L V P I N N T D T Y I N S E N M
TTTCTATTCCAGGTGAGGTTAAAAATTTGGTTGAAATTTGTCAAGTAGACAGCCTAGTACCAATAAATAACACTGACACCTACATCAATAGTGAAAATAT
    1710      1720      1730      1740      1750      1760      1770      1780      1790      1800

    Y S V V L Q S S I N A P D K I F S I R T D V A S Q P L A T T L I G
GTATTCTGTTGTATTGCAATCATCAATTAATGCACCAGATAAGATCTTCTCTATTCGAACAGATGTTGCTTCCCAACCTTTAGCTACTACTTTGATTGGT
    1810      1820      1830      1840      1850      1860      1870      1880      1890      1900

    E I S S Y F T H W T G S L R F S F M F C G T A N T T V K L L L A Y
GAGATATCTAGCTATTTCACCCACTGGACAGGGAGTCTCCGTTTCAGCTTCATGTTTTGTGGTACTGCCAACACTACTGTTAAGCTTTTGTTGGCATACA
    1910      1920      1930      1940      1950      1960      1970      1980      1990      2000

    T P P G I A E P T T R K D A M L G T H V I W D V G L Q S T I S M V V
CACCACCTGGTATCGCAGAACCCACCACAAGAAAGGATGCAATGCTAGGCACTCATGTTATATGGGATGTGGGGTTGCAGTCTACAATATCAATGGTAGT
    2010      2020      2030      2040      2050      2060      2070      2080      2090      2100

    P W I S A S H Y R N T S P G R S T S G Y I T C W Y Q T R L V I P P
GCCATGGATTAGCGCTAGTCATTATAGAAACACATCACCAGGTAGATCTACATCTGGGTACATAACATGCTGGTATCAGACTAGATTAGTCATTCCACCT
    2110      2120      2130      2140      2150      2160      2170      2180      2190      2200

    Q T P P T A R L L C F V S G C K D F C L R M A R D T N L H L Q S G
CAGACCCCACCAACAGCTAGATTGTTATGTTTTGTATCTGGGTGCAAAGACTTTTGCTTGCGCATGGCACGAGATACTAACCTACACCTGCAAAGTGGTG
    2210      2220      2230      2240      2250      2260      2270      2280      2290      2300
   ↓  VP1
    A I A Q N P V E N Y I D E V L N E V L V V P N I N S S N P T T S N S
CAATAGCACAGAACCCTGTTGAGAATTATATAGATGAAGTTCTTAATGAAGTTTTTAGTTGTCCCAAATATTAATAGTAGTAACCCCACAACATCAAATTC
    2310      2320      2330      2340      2350      2360      2370      2380      2390      2400

    A P A L D A A E T G H T S S V Q P E D V I E T R Y V Q T S Q T R D
TGCCCCAGCATTAGATGCTGCAGAAACAGGGCACACTAGTAGTGTTCAACCAGAGGATGTCATTGAAACTAGGTATGTGCAGACATCACAAACAAGAGAT
    2410      2420      2430      2440      2450      2460      2470      2480      2490      2500

    E M S L E S F L G R S G C I H E S K L E V T L A N Y N K E N F T V
GAAATGAGTTTAGAGAGTTTTCTTGGCAGATCAGGATGCATACATGAATCTAAATTAGAGGTTACACTTGCAAATTATAACAAGGAGAATTTTACAGTGT
    2510      2520      2530      2540      2550      2560      2570      2580      2590      2600

    W A I N L Q E M A Q I R R K F E L F T Y T R F D S E I T L V P C I S
GGGCTATTAATCTACAAGAAATGGCTCAAATTAGAAGGAAATTTGAATTGTTCACCTATACTAGGTTTGATTCTGAAATAACCCTAGTTCCATGCATTTC
    2610      2620      2630      2640      2650      2660      2670      2680      2690      2700

    A L S Q D I G H I T M Q Y M Y V P P G A P V P N S R D D Y A W Q S
CGCCCTTAGTCAGGACATTGGACACATCACAATGCAATACATGTATGTTCCACCAGGTGCACCGGTGCCCAATAGTAGGGACGATTATGCATGGCAGTCT
    2710      2720      2730      2740      2750      2760      2770      2780      2790      2800

    G T N A S V F W Q H G Q A Y P R F S L P F L S V A S A Y Y M F Y D
GGCACTAATGCCTCTGTTTTCTGGCAACATGGACAGGCTTATCCAAGATTTTCCTTACCTTTCCTAAGTGTGGCATCTGCTTATTACATGTTTTATGATG
    2810      2820      2830      2840      2850      2860      2870      2880      2890      2900
```

Fig. 14c

```
      G  Y  D  E  Q  D  Q  N  Y  G  T  A  N  T  N  N  M  G  S  L  C  S  R  I  V  T  E  K  H  I  H  K  V  H
GGTATGATGAACAAGATCAAAACTATGGTACAGCAAACACAAATAACATGGGGTCACTATGCTCTAGGATAGTAACAGAGAAACACATTCATAAAGTACA
     2910    2920    2930    2940    2950    2960    2970    2980    2990    3000

      I  M  T  R  I  Y  H  K  A  K  H  V  K  A  W  C  P  R  P  P  R  A  L  E  Y  T  R  A  H  R  T  N  F
TATAATGACAAGAATCTATCACAAGGCTAAACATGTCAAGGCATGGTGTCCACGCCCACCCAGAGCGCTTGAGTATACTCGTGCTCATCGCACTAATTTT
     3010    3020    3030    3040    3050    3060    3070    3080    3090    3100

                                                                              ▽ ?
      K  I  E  D  R  S  I  Q  T  A  I  V  T  R  P  I  I  T  T  A  G  P  S  D  M  Y  V  H  V  G  N  L  I
AAAATTGAGGATAGGAGTATTCAGACAGCAATTGTGACCAGACCAATTATCACTACAGCTGGCCCCAGTGACATGTATGTTCATGTAGGTAACCTTATTT
     3110    3120    3130    3140    3150    3160    3170    3180    3190    3200

                                         ▽ ? P2-A
      Y  R  N  L  H  L  F  N  S  E  M  H  E  S  I  L  V  S  Y  S  S  D  L  I  I  Y  R  T  N  T  V  G  D  D
ATAGAAATCTTCATCTTTTCAACTCTGAGATGCATGAATCTATTTTGGTATCTTATTCATCAGATTTAATCATTTACCGAACAAACACTGTAGGTGATGA
     3210    3220    3230    3240    3250    3260    3270    3280    3290    3300

      Y  I  P  S  C  D  C  T  Q  A  T  Y  Y  C  K  H  K  N  R  Y  F  P  I  T  V  T  S  H  D  W  Y  E  I
TTACATTCCCTCTTGTGATTGTACCCAAGCTACTTATTATTGCAAACATAAAAATAGATACTTCCCAATTACAGTTACAAGCCATGACTGGTATGAAATA
     3310    3320    3330    3340    3350    3360    3370    3380    3390    3400

      Q  E  S  E  Y  Y  P  K  H  I  Q  Y  N  L  L  I  G  E  G  P  C  E  P  G  D  C  G  G  K  L  L  C  K
CAGGAAAGTGAGTACTATCCCAAACACATACAGTACAATTTGTTGATTGGTGAGGGCCCTTGTGAACCAGGTGACTGTGGTGGAAAGTTGCTATGCAAAC
     3410    3420    3430    3440    3450    3460    3470    3480    3490    3500

                                                                     ▽ P2-B
      H  G  V  I  G  I  V  T  A  G  G  D  N  H  V  A  F  I  D  L  R  H  F  H  C  A  E  E  Q  G  V  T  D  Y
ATGGTGTCATAGGTATAGTAACAGCTGGTGGTGATAATCATGTGGCTTTTATTGACCTTAGACACTTCCATTGTGCTGAAGAACAAGGGGTTACAGATTA
     3510    3520    3530    3540    3550    3560    3570    3580    3590    3600

      I  H  M  L  G  E  A  F  G  N  G  F  V  D  S  V  K  E  H  I  H  A  I  N  P  V  G  N  I  S  K  K  I
TATACATATGCTAGGAGAAGCATTTGGAAATGGATTTGTGGATAGTGTAAAAGAACATATACATGCCATAAACCCAGTAGGAAATATCAGCAAGAAAATT
     3610    3620    3630    3640    3650    3660    3670    3680    3690    3700

      I  K  W  M  L  R  I  I  S  A  M  V  I  I  I  R  N  S  S  D  P  Q  T  I  L  A  T  L  T  L  I  G  C
ATTAAATGGATGTTGAGAATAATATCAGCAATGGTCATAATAATTAGAAACTCTTCTGACCCCCAAACTATATTAGCAACACTCACACTGATTGGGTGTT
     3710    3720    3730    3740    3750    3760    3770    3780    3790    3800

                                                           ▽ P2-C
      S  G  S  P  W  R  F  L  K  E  K  F  C  K  W  T  Q  L  N  Y  I  H  K  E  S  D  S  W  L  K  K  F  T  E
CTGGATCACCCTGGAGATTTTTAAAGGAAAAATTCTGTAAATGGACACAGCTTAATTATATACACAAAGAATCAGATTCATGGTTAAAGAAATTTACTGA
     3810    3820    3830    3840    3850    3860    3870    3880    3890    3900

      A  C  N  A  A  R  G  L  E  W  I  G  N  K  I  S  K  F  I  E  W  M  K  S  M  L  P  Q  A  Q  L  K  V
AGCATGCAATGCAGCTAGAGGGCTTGAATGGATAGGGAATAAGATATCTAAATTTATTGAATGGATGAAGTCGATGCTCCCGCAAGCTCAATTGAAGGTT
     3910    3920    3930    3940    3950    3960    3970    3980    3990    4000

      K  Y  L  N  E  L  K  K  L  N  L  Y  E  K  Q  V  E  S  L  R  V  A  D  M  K  T  Q  E  K  I  K  M  E
AAGTACTTAAACGAGCTTAAAAAAACTCAACCTATACGAAAAGCAAGTTGAGAGCTTGCGGGTGGCTGACATGAAAACACAAGAAAAAATTAAAATGGAAA
     4010    4020    4030    4040    4050    4060    4070    4080    4090    4100

      I  D  T  L  H  D  L  S  R  K  F  L  P  L  Y  A  S  E  A  K  R  I  K  T  L  Y  I  K  C  D  N  I  I  K
TAGACACTTTACATGATTTGTCACGTAAATTTCTACCTTTGTATGCAAGTGAGGCAAAAAGGATAAAAACCCTATACATTAAATGTGATAATATCATCAA
     4110    4120    4130    4140    4150    4160    4170    4180    4190    4200

      Q  K  K  R  C  E  P  V  A  I  V  I  H  G  P  P  G  A  G  K  S  I  T  T  N  F  L  A  K  M  I  T  N
GCAGAAGAAAAGATGTGAACCAGTAGCTATAGTTATTCATGGACCACCTGGTGCTGGCAAATCTATAACAACAAATTTCCTGGCCAAAATGATAACTAAT
     4210    4220    4230    4240    4250    4260    4270    4280    4290    4300

      D  S  D  I  Y  S  L  P  P  D  P  K  Y  F  D  G  Y  D  Q  Q  S  V  V  I  M  D  D  I  M  Q  N  P  A
GATAGTGACATATACTCTCTACCTCCTGATCCAAAATATTTTGATGGTTATGACCAACAGAGTGTAGTAATAATGGATGACATTATGCAGAATCCAGCCG
     4310    4320    4330    4340    4350    4360    4370    4380    4390    4400
```

```
        G  D  D  M  T  L  F  C  Q  M  V  S  S  V  T  F  I  P  P  M  A  D  L  P  D  K  G  K  A  F  D  S  R  F
     GGGATGACATGACACTGTTCTGCCAAATGGTTTCTAGTGTTACATTTATACCACCAATGGCTGATCTACCAGATAAAGGCAAGGCTTTTGATTCTAGGTT
        4410      4420      4430      4440      4450      4460      4470      4480      4490      4500


        V  L  C  S  T  N  H  S  L  L  T  P  P  T  I  T  S  L  P  A  M  N  R  R  F  F  L  D  L  D  I  I  V
     TGTATTATGCAGCACAAATCATTCCCTTCTAACACCCCCGACAATAACTTCACTACCTGCAATGAATAGAAGATTTTTCCTAGATTTAGATATAATAGTA
        4510      4520      4530      4540      4550      4560      4570      4580      4590      4600


        H  D  N  F  K  D  P  Q  G  K  L  N  V  A  A  A  F  R  P  C  D  V  D  N  R  I  G  N  A  R  C  C  P
     CATGATAACTTCAAAGATCCACAGGGCAAACTTAATGTGGCAGCAGCGTTTCGACCATGTGATGTAGATAATAGAATAGGAAATGCACGTTGTTGTCCAT
        4610      4620      4630      4640      4650      4660      4670      4680      4690      4700


        F  V  C  G  K  A  V  S  F  K  D  R  N  S  C  N  K  Y  S  L  A  Q  V  Y  N  I  M  I  E  E  D  R  R  R
     TTGTGTGTGGAAAAGCAGTTTCTTTCAAAGATCGTAACTCTTGCAACAAATACAGCCTTGCGCAGGTGTACAACATAATGATTGAAGAAGACAGACGGAG
        4710      4720      4730      4740      4750      4760      4770      4780      4790      4800

                                            ↓ P3-A
        R  Q  V  V  D  V  M  T  A  I  F  Q  G  P  I  D  M  K  N  P  P  P  P  A  I  T  D  L  L  Q  S  V  R
     AAGACAAGTGGTTGATGTCATGACAGCTATATTCCAAGGGCCAATTGATATGAAAAACCCACCACCACCTGCTATTACTGACTTGCTCCAGTCTGTTAGA
        4810      4820      4830      4840      4850      4860      4870      4880      4890      4900


        T  P  E  V  I  K  Y  C  E  G  N  R  W  I  I  P  A  E  C  K  I  E  K  E  L  N  L  A  N  T  I  I  T
     ACCCCTGAAGTTATTAAGTATTGTGAGGGTAATAGATGGATAATTCCAGCAGAATGCAAGATAGAAAAGGAGTTGAACTTGGCTAACACAATCATAACAA
        4910      4920      4930      4940      4950      4960      4970      4980      4990      5000

                                                                        ↓ VPg
        I  I  A  N  V  I  G  M  A  R  I  I  Y  V  I  Y  K  L  F  C  T  L  Q  G  P  Y  S  G  E  P  K  P  K  T
     TCATTGCAAATGTTATTGGTATGGCGAGAATAATATATGTTATTTACAAACTTTTTTTGCACATTACAGGGACCATATTCAGGAGAACCAAAGCCCAAGAC
        5010      5020      5030      5040      5050      5060      5070      5080      5090      5100

                          ↓ PROTEASE
        K  I  P  E  R  R  V  V  T  Q  G  P  E  E  E  F  G  M  S  L  I  K  H  N  S  C  V  I  T  T  E  N  G
     TAAAAATCCCAGAAAGGCGTGTAGTAACACAGGGACCAGAGGAGGAATTTGGGATGTCTTTAATTAAACATAACTCATGTGTTATTACAACAGAAAATGGG
        5110      5120      5130      5140      5150      5160      5170      5180      5190      5200


        K  F  T  G  L  G  V  Y  D  R  F  V  V  V  P  T  H  A  D  P  G  K  E  I  Q  V  D  G  I  T  T  K  V
     AAATTCACAGGTCTTGGAGTATACGACAGATTTGTGGTCGTACCAACACATGCAGATCCTGGAAAGGAAATTCAGGTTGATGGTATAACTACAAAAGTCA
        5210      5220      5230      5240      5250      5260      5270      5280      5290      5300


        I  D  S  Y  D  L  Y  N  K  N  G  I  K  L  E  I  T  V  L  K  L  D  R  N  E  K  F  R  D  I  R  R  Y  I
     TTGACTCATATGACCTATACAACAAGAATGGGATAAAGCTAGAAATAACAGTACTTAAATTAGATAGAAATGAAAAATTTAGAGATATCAGGAGATATAT
        5310      5320      5330      5340      5350      5360      5370      5380      5390      5400


        F  N  N  E  D  D  Y  P  N  C  N  L  A  L  L  A  N  Q  P  E  P  T  I  I  N  V  G  D  V  V  S  Y  G
     ACCTAACAATGAAGATGATTACCCCAATTGCAACTTAGCACTGCTAGCAAACCAGCCTGAACCAACTATAATCAATGTTGGAGATGTTGTATCCTATGGC
        5410      5420      5430      5440      5450      5460      5470      5480      5490      5500


        N  I  L  L  S  G  N  Q  T  A  R  M  L  K  Y  S  Y  P  T  K  S  G  Y  C  G  G  V  L  Y  K  I  G  Q
     AATATACTGCTCAGTGGCAACCAAACGGCTAGAATGCTTAAATACAGTTACCCAACTAAATCTGGTTACTGTGGAGGTGTCTTATACAAAATTGGGCAAG
        5510      5520      5530      5540      5550      5560      5570      5580      5590      5600

                                                                    ↓ POLYMERASE
        V  L  G  I  H  V  G  G  N  G  R  D  G  F  S  A  M  L  L  R  S  Y  F  T  D  V  Q  G  Q  I  T  L  S  K
     TGCTTGGAATACATGTTGGGGGCAATGGTAGGGATGGTTTCTCAGCTATGTTACTCAGATCCTATTTCACTGATGTTCAGGGCCAAATAACGTTATCAAA
        5610      5620      5630      5640      5650      5660      5670      5680      5690      5700


        K  T  S  E  C  N  L  P  S  I  H  T  P  C  K  T  K  L  Q  P  S  V  F  Y  D  V  F  P  G  S  K  E  P
     GAAGACCAGTGAATGTAACCTACCCAGTATACACACCCCATGCAAAACCAAATTGCAGCCTAGTGTTTTCTATGATGTATTCCCTGGTTCAAAAGAACCA
        5710      5720      5730      5740      5750      5760      5770      5780      5790      5800


        A  V  L  S  E  K  D  A  R  L  Q  V  D  F  N  E  A  L  F  S  K  Y  K  G  N  T  D  C  S  I  N  D  H
     GCTGTGTTGTCTGAAAAAGATGCCCGGTTACAAGTTGATTTCAATGAAGCACTATTTTCTAAATACAAAGGGAATACAGATTGCTCCATTAATGACCACA
        5810      5820      5830      5840      5850      5860      5870      5880      5890      5900
```

Fig. 14e

```
 I  R  I  A  S  S  H  Y  A  A  Q  L  I  T  L  D  I  D  P  K  P  I  T  L  E  D  S  V  F  G  T  D  G  L
TAAGAATTGCATCATCACATTATGCAGCACAACTCATTACCTTAGATATTGACCCAAAACCTATTACACTTGAGGACAGTGTCTTTGGCACTGATGGATT
     5910      5920      5930      5940      5950      5960      5970      5980      5990      6000

    E  A  L  D  L  N  T  S  A  G  F  P  Y  I  A  M  G  V  K  K  R  D  L  I  N  N  K  T  K  D  I  S  K
AGAGGCTCTTGATTTGAACACTAGCGCAGGATTTCCATATATTGCAATGGGAGTTAAAAAGAGAGATTTAATAAACAACAAGACCAAGGATATAAGCAAA
     6010      6020      6030      6040      6050      6060      6070      6080      6090      6100

    L  K  E  A  I  D  K  Y  G  V  D  L  P  M  V  T  F  L  K  D  E  L  R  K  H  E  K  V  I  K  G  K  T
CTTAAAGAAGCAATTGACAAATACGGAGTTGACTTACCTATGGTCACCTTCTTGAAAGATGAACTCAGAAAGCATGAAAAGGTAATTAAAGGTAAAACTA
     6110      6120      6130      6140      6150      6160      6170      6180      6190      6200

    R  V  I  E  A  S  S  V  N  D  T  L  L  F  R  T  T  F  G  N  L  F  S  K  F  H  L  N  P  G  I  V  T  G
GAGTTATTGAAGCTAGTAGTGTGAATGATACCCTATTATTTAGAACAACTTTTGGCAACCTCTTTTCAAAGTTCCACTTGAATCCTGGAATTGTTACTGG
     6210      6220      6230      6240      6250      6260      6270      6280      6290      6300

       S  A  V  G  C  D  P  E  V  F  W  S  K  I  P  A  M  L  D  D  K  C  I  M  A  F  D  Y  T  N  Y  D  G
ATCAGCAGTTGGATGTGATCCAGAGGTGTTTTGGTCAAAAATACCAGCAATGTTGGATGATAAATGTATTATGGCTTTTGATTATACAAATTATGATGGT
     6310      6320      6330      6340      6350      6360      6370      6380      6390      6400

    S  I  H  P  I  W  F  E  A  L  K  Q  V  L  V  D  L  S  F  N  P  T  L  I  D  R  L  C  K  S  K  H  I
AGTATACACCCTATTTGGTTTGAAGCTCTTAAACAGGTACTGGTAGATCTATCATTTAATCCAACATTAATAGATAGACTATGCAAGTCTAAACACATCT
     6410      6420      6430      6440      6450      6460      6470      6480      6490      6500

    F  K  N  T  Y  Y  E  V  E  G  G  V  P  S  G  C  S  G  T  S  I  F  N  T  M  I  N  N  I  I  I  R  T  L
TCAAAAATACATACTATGAAGTGGAGGGAGGTGTACCATCTGGGTGTTCAGGTACTAGTATTTTTAACACTATGATCAATAATATTATCATAAGGACCTT
     6510      6520      6530      6540      6550      6560      6570      6580      6590      6600

    V  L  D  A  Y  K  N  I  D  L  D  K  L  K  I  I  A  Y  G  D  D  V  I  F  S  Y  I  H  E  L  D  M  E
AGTGTTAGATGCATACAAGAATATAGATCTAGATAAGCTTAAGATAATTGCCTATGGTGATGATGTCATATTCTCATACATACATGAACTGGACATGGAG
     6610      6620      6630      6640      6650      6660      6670      6680      6690      6700

    A  I  A  I  E  G  V  K  Y  G  L  T  I  T  P  A  D  K  S  N  T  F  V  K  L  D  Y  S  N  V  T  F  L
GCTATAGCAATAGAGGGTGTTAAATATGGTTTGACTATAACTCCTGCTGATAAATCTAACACATTTGTAAAATTAGACTATAGCAATGTTACTTTTTTAA
     6710      6720      6730      6740      6750      6760      6770      6780      6790      6800

    K  R  G  F  K  Q  D  E  K  Y  N  F  L  I  H  P  T  F  P  E  D  E  I  F  E  S  I  R  W  T  K  K  P  S
AAAGAGGGTTTAAGCAAGATGAGAAGTATAACTTTCTAATACATCCAACTTTCCCTGAAGATGAAATATTTGAATCCATCAGATGGACAAAGAAACCATC
     6810      6820      6830      6840      6850      6860      6870      6880      6890      6900

    Q  M  H  E  H  V  L  S  L  C  H  L  M  W  H  N  G  R  D  A  Y  K  K  F  V  E  K  I  R  S  V  S  A
ACAAATGCATGAACATGTGTTGTCTCTGTGTCACTTAATGTGGCACAATGGACGTGACGCATACAAAAAATTTGTGGAGAAGATACGCAGTGTAAGCGCT
     6910      6920      6930      6940      6950      6960      6970      6980      6990      7000

    G  R  A  L  Y  I  P  P  Y  D  L  L  L  H  E  W  Y  E  K  F
GGTCGTGCACTGTACATCCCTCCGTATGATTTGCTTTTGCATGAGTGGTATGAAAAATTTTAAAGATATAGAAATAGTAAACTGATAGTTTATTAGTTTT
     7010      7020      7030      7040      7050      7060      7070      7080      7090      7100


AT poly(A)
     7110      7120      7130      7140      7150      7160      7170      7180      7190      7200
```

Fig. 15

Fig. 16

HRV 89 - Genom

CATGGTGTGCATGGTTTCTGCATG
CACACGTACCAAAGAC

Nco I - Sph I Linker

Nukleotidnummer 2251 - 4703 (HRV89)

pKK 89/2A

H....Hind III
N....Nco I
P....Pvu II
S....Sph I

Fig. 17a

```
                              M V C M V S A C K D F C L R L A R
                        ATGGTGTGCATGGTTTCTGCATGCAAAGATTTTTGCTTAAGATTAGCCAG
                            2260      2270      2280      2290      2300


    D T N L H T Q E G V L T Q M P V E N Y I D S V L N E V L V V P M I
AGATACTAACCTACACACACAAGAAGGAGTACTCACACACAAACCCAGTTGAAAATTATATAGATAGTGTATTAAATGAAGTTCTTGTGGTGCCAAATATC
    2310      2320      2330      2340      2350      2360      2370      2380      2390      2400


    Q P S T S V S S H A A P A L D A A E T G H T S G V Q P E D M I E T
CAACCTAGCACATCTGTGTCAAGTCATGCAGCGCCTGCATTGGATGCTGCGGAAACCGGACACACCAGCTCTGTTCAACCTGAAGATATGATTGAAACTA
    2410      2420      2430      2440      2450      2460      2470      2480      2490      2500


    R Y V I T D Q T R D E T S I E S F L G R S G C I A M I E F N T S S D
GATATGTTATAACTGATCAAACAAGGGATGAAACAAGTATTGAGAGTTTCTTAGGTAGGTCAGGGTGTATCGCTATGATAGAATTTAATACAAGTAGTGA
    2510      2520      2530      2540      2550      2560      2570      2580      2590      2600


    K T E H D K I G K G F K T W K V S L Q E M A Q I R R K Y E L F T Y
TAAAACTGAACATGATAAAATTGGTAAAGGATTCAAAACATGGAAGGTTAGTCTTCAAGAAATGGCACAAATCAGAAGAAAATATGAATTATTCACATAT
    2610      2620      2630      2640      2650      2660      2670      2680      2690      2700


    T R F D S E I T I V T A A A A Q G N D S G H I V L Q F M Y V P P G
ACAAGATTTGATTCAGAGATAACAATAGTCACTGCAGCCGCAGCTCAAGGAAATGATAGTGGACATATAGTATTGCAATTTATGTATGTACCCCCAGGAG
    2710      2720      2730      2740      2750      2760      2770      2780      2790      2800


    A P V P E K R D D Y T W Q S G T N A S V F W Q E G G Q P Y P R F T I P
CACCTGTCCCCGAAAAACGTGATGATTACACATGGCAATCAGGAACAAATGCATCTGTGTTCTGGCAAGAAGGACAACCATACCCCAGATTCACAATCCC
    2810      2820      2830      2840      2850      2860      2870      2880      2890      2900


    F M S I A S A Y Y M F Y D G Y D G D S A A S K Y G S V V T N D M G
TTTTATGAGCATTGCATCAGCCTATTACATGTTTTATGATGGTTATGATGGTGATAGTGCAGCATCAAAATACGGTTCTGTAGTCACTAATGATATGGGA
    2910      2920      2930      2940      2950      2960      2970      2980      2990      3000


    T I C V R I V T S N Q K H D S N I V C R I Y H K A K H I K A W C P
ACCATATGTGTTAGAATAGTGACATCCAACCAAAAACACGATTCAAATATTGTGTGCCGCATTTACCACAAGGCCAAACATATAAAAGCATGGTGTCCTC
    3010      3020      3030      3040      3050      3060      3070      3080      3090      3100


    R P P R A V A Y Q H T H S T N Y I P S N G E A T T Q I K T R P D V F
GCCCACCAAGGGCTGTTGCCTATCAACACACACACTCAACCAATTACATACCATCCAATGGTGAGGCCACAACTCAGATTAAAACCAGACCTGATGTTTT
    3110      3120      3130      3140      3150      3160      3170      3180      3190      3200


    T V T N V G P S S M F V H V G N L I Y R N L H L F N S D L D D S I
TACCGTTACAAACGTCGGACCATCTAGTATGTTTGTACATGTGGGTAACTTAATCTATAGAAATCTTCATCTCTTTAATTCTGATCTTGATGATTCTATT
    3210      3220      3230      3240      3250      3260      3270      3280      3290      3300


    L V S Y S S D L I I Y R T N T E G N D V I P N C D C T E C T Y Y C
CTTGTATCATACTCCAGTGATCTAATCATATATCGAACAAACACTGAAGGTAATGATGTGATCCCTAATTGTGATTGCACTGAATGTACATATTACTGCC
    3310      3320      3330      3340      3350      3360      3370      3380      3390      3400


    H H K D R Y F P I R V T A H D W Y E I Q E S E Y Y P K H I Q Y N L L
ACCACAAAGATAGGTATTTTCCTATCAGAGTTACTGCACATGATTGGTATGAGATTCAAGAATCAGAATATTACCCAAAACATATCCAATATAATCTCCT
    3410      3420      3430      3440      3450      3460      3470      3480      3490      3500


    I G E G P C E P G D C G G K L L C K H G V I G M I T A G G E G H V
GATTGGAGAGGGTCCTTGTGAACCAGGAGATTGTGGAGGAAAACTATTGTGTAAACATGGTGTTATAGGTATGATTACAGCTGGAGGTGAAGGTCACGTT
    3510      3520      3530      3540      3550      3560      3570      3580      3590      3600


    A F I D L R K F Q C A E E Q G L S D Y V E H L G Q V F G V G F V D
GCTTTTATTGACCTGAGAAAATTCCAGTGTGCTGAGGAGCAAGGGTTATCTGATTATGTGGAACATCTTGGTCAAGTCTTTGGTGTAGGCTTCGTAGACA
    3610      3620      3630      3640      3650      3660      3670      3680      3690      3700
```

Fig. 17b

```
    S I K Q Q V N F I N P T S K I G S K V I K W L L R I V S A M I I M V
GCATCAAACAACAGGTAAACTTTATCAACCCCACTAGTAAAATTGGTTCAAAAGTGATTAAATGGTTGTTGAGGATAGTTTCAGCTATGATAATAATGGT
    3710      3720      3730      3740      3750      3760      3770      3780      3790      3800

    R N S S D P Q T V I A T L T L L G C S G S P W R F L K E K L C A W
AAGGAATAGTTCTGATCCACAAACTGTAATTGCCACTCTCACCCTTCTAGGTTGTTCAGGCTCACCATGGAGGTTTCTTAAAGAGAAACTCTGTGCGTGG
    3810      3820      3830      3840      3850      3860      3870      3880      3890      3900

                        ↓ P2-C
    L Q L S Y V H K Q ▽ S D S W L K K F T E A C N A A R G L E W I G Q K
CTCCAGCTTAGCTATGTACATAAGCAGTCTGATTCATGGCTCAAGAAATTTACTGAAGCGTGTAACGCAGCACGTGGGCTAGAGTGGATTGGACAAAAGA
    3910      3920      3930      3940      3950      3960      3970      3980      3990      4000

    I S K F I D W I K S M L P Q A Q L K I D Y L T K L K Q L N L L E K Q
TATCTAAATTTATAGATTGGATAAAGAGTATGTTACCACAGGCTCAATTGAAAATTGATTACCTAACCAAATTAAAACAACTTAATCTCTTAGAGAAACA
    4010      4020      4030      4040      4050      4060      4070      4080      4090      4100

    I E T I R L A P A S V Q E K I F I E I N T L H D L S L K F L P L Y
AATAGAAACAATTAGACTTGCACCTGCTAGTGTTCAGGAGAAAATTTTCATTGAAATAAACACCCTTCATGATTTATCCTTAAAATTCTTACCACTGTAT
    4110      4120      4130      4140      4150      4160      4170      4180      4190      4200

    A S E A R R I K N L Y I K C S N V I K G G K R N E P V A V L I H G
GCATCTGAAGCACGTAGAATTAAGAATTTATATATCAAATGCAGTAATGTTATTAAAGGGGGAAAGAGGAATGAACCAGTTGCAGTTCTAATACATGGTT
    4210      4220      4230      4240      4250      4260      4270      4280      4290      4300

  S P G T G K S L A T S V L A R M L T V E T D I Y S L P P D P K Y F D
CTCCTGGTACTGGAAAATCTCTTGCCACTTCTGTTCTTGCTCGAATGCTAACTGTTGAGACTGATATATATTCTTTGCCCCCAGATCCTAAATATTTTGA
    4310      4320      4330      4340      4350      4360      4370      4380      4390      4400

    G Y D Q Q S V V I M D D I M Q N P S G E D M T L F C Q M V S S V P
TGGGTATGATCAACAGAGTGTTGTTATCATGGATGATATCATGCAAATCCTAGTGGTGAAGACATGACTTTGTTTTGCCAAATGGTATCGAGTGTCCCT
    441C      4420      4430      4440      4450      4460      4470      4480      4490      4500

    F I P P M A D L P D K G K P F T S K F V L A S T N H T L L T P P T
TTCATACCTCCTATGGCAGATCTTCCAGATAAAGGAAAACCATTTACATCCAAGTTTGTACTTGCAAGCACTAATCACACTCTACTAACACCACCAACAG
    4510      4520      4530      4540      4550      4560      4570      4580      4590      4600

  V S S L P A M A R R F Y F D L D I Q V K K E Y L L D G K L D I A K S
TATCTTCATTACCAGCAATGGCAAGAAGGTTTTACTTTGATCTAGACATTCAAGTTAAGAAAGAGTATCTTTAGATGGCAAACTAGTATATAGCAAAAG
    4610      4620      4630      4640      4650      4660      4670      4680      4690      4700


    ..W..A..V..L..A..D..E..R..R..F..S..W.STOP

    CTTGGCTGTTTTGGCGGATGAGAGAAGATTTTCAGCCTGA
```

Fig. 18

HRV2

```
    P  P  P  P  A  I  T  D  L  L  Q  S  V  R  T  P  E  V  I  K  Y  C  E  G  N  R  W  I  I  P  A  E  C

CCACCACCACCTGCTATTACTGACTTGCTCCAGTCTGTTAGAACCCCTGAAGTTATTAAGTATTGTGAGGGTAATAGATGGATAATTCCAGCAGAATGCA
      4860      4870      4880      4890      4900      4910      4920      4930      4940      4950

    K  I  E  K  E  L  N  L  A  N  T  I  I  T  I  I  A  N  V  I  G  M  A  R  I  I  Y  V  I  Y  K  L  F  C

AGATAGAAAAGGAGTTGAACTTGGCTAACACAATCATAACAATCATTGCAAATGTTATTGGTATGGCGAGAATAATATATGTTATTTACAAACTTTTTTG
      4960      4970      4980      4990      5000      5010      5020      5030      5040      5050

                  ▽
    T  L  Q  G  P  Y  S  G  E  P  K  P  K

CACATTACAGGGACCATATTCAGGAGAACCAAAGCCCAA
      5060      5070      5080      5090
```

HRV89

```
    P  P  P  P  A  I  A  D  L  L  R  S  V  K  T  P  E  I  I  K  Y  C  Q  D  N  N  W  I  V  P  A  E  C

CCTCCACCTCCAGCCATAGCTGACCTCCTTAGGTCTGTGAAAACACCAGAGATCATTAAGTATTGCCAAGATAATAATTGGATTGTTCCAGCAGAGTGTT


    S  I  E  R  D  L  G  I  A  N  M  T  I  G  I  I  A  N  V  V  S  I  V  G  V  I  Y  I  I  Y  K  L  F  C

CTATTGAAAGAGATTTAGGGATAGCAAATATGACTATAGGTATAATAGCTAATGTGGTCTCTATAGTAGGTGTTATCTATATAATTTATAAATTGTTCTG

              ▽
    T  L  Q  G  P  Y  S  G  E  P  K  P  K

TACACTTCAGGGTCCATACTCAGGGGAACCTAAACCCAA
```

```
PPPPAITDLL QSVRTPEVIK YCEGNRWIIP AECKIEKELN LANTIITIIA NVIGMARIIY VIYKLFCTLQ GPYSGEPKPK    HRV2
PPPPAIADLL RSVKTPEIIK YCQDNNWIVP AECSIERDLG IANMTISIIA NVVSIVGVIY IIYKLFCTLQ GPYSGEPKPK    HRV89
```

Fig. 19

## 5' Untranslatierte Region

```
OBERE   SEQUENZ:  HRV89 DNA                    VON  ·  1 BIS  618
UNTERE  SEQUENZ:  HRV2 DNA                     VON     1 BIS  610
HOMOLOGIE:    79%
```

```
TTAAAACTGGGAGTGGGTTGTTCCCAC-T-CACTCCACCCATGCGGTGTTGTACTCTGTTATTACGGTAACTTTGTACGCCAGTTTT-TCCCACCCTTCC
**********   ***********  *  *  *  *  **  *  ** **  ***************************************  ** *  **  ****
TTAAAACTGGATCCAGGTTGTTCCCACCTGGATTTCCCACA-G-GGAGTGGTACTCTGTTATTACGGTAACTTTGTACGCCAGTTTTATCTC-CC-TTCC
        10        20        30        40        50        60        70        80        90       100


CCATAATGTAACTTAGAAGTTTGT-ACAATATGACCAATAGGTGACAATCATCCAGACT-GTCAAAGGTCAAGCACTTCTGTTTCCCCGGTCAATGAGGA
**  *  **************** *  ****  *  *********  *  *****  *****  *  *  **  ************************************  **
CC-CA-TGTAACTTAGAAGTTTTTCACAA-A-GACCAATAGCCGGTAATCAGCCAGATTACTGAA-GGTCAAGCACTTCTGTTTCCCCGGTCAATGTTGA
       110       120       130       140       150       160       170       180       190       200


TATGCTTTACCCAAGGCAAAAACCTTAGAGATCGTTATCCCCACACTGCCTACACAGAGCCCAGTACCATTTTTGATATAATTGGGTTGGTCGCTCCCTG
******   *  *  *  *********      *  ********  **  **  *  ******  **  ***   ****  ***  *****   **   **  **  ******  ***   *
TATGCTCCAAC-AGGGCAAAAACAACTGCGATCGTTAACCGCAAAGCGCCTACGCAAAGCTTAGTAGCATCTTTGAAATCGTTTGGCTGGTCGATCC--G
       210       220       230       240       250       260       270       280       290       300


CAAACCCAGCAG-TAGACCTGGCAGATGAGGCTGGACATTCCCCACTGGCGACAGTGGTCCAGCCTGCGTGGCTGCCTGCTCACCCTTCTTGGGTGAGAA
*  *    *  *  *  ****************************  **  **  ****************  **  *******************  ******  *  *****  ***
CCATTTCCCCTGGTAGACCTGGCAGATGAGGCTAGAAATACCCCACTGGCGACAGTGTTCTAGCCTGCGTGGCTGCCTGCACACCCT-AT-GGGTGTGAA
       310       320       330       340       350       360       370       380       390       400


GCCTAATTATTGACAAGGTGTGAAGAGCCGCGTGTGCTCAGTGTGCTTCCTCCGGCCCCTGAATGTGGCTAACCTTAACCCTGCAGCCGTTGCCCATAAT
***  **   **  ****************** *********   *  **   ***********************************************       **  *  ***
GCCAAACAATGGACAAGGTGTGAAGAGCCCCGTGTGCTCGCTTTGAGTCCTCCGGCCCCTGAATGTGGCTAACCTTAACCCTGCAGCTAGAGCACGTAAC
       410       420       430       440       450       460       470       480       490       500


CCAATGGGTTTGCGGTCGTAATGCGTAAGTGCGGGATGGGACCAACTACTTTGGGTGTCCGTGTTTC-CTGTTTTTCTTTTGATT-GCATTTTATGGTGA
******  **  *    *********  *  **  ***************************************  **  ****  ****   ***  **  **  *******
CCAATGTGTATCTAGTCGTAATGAGCAATTGCGGGATGGGACCAACTACTTTGGGTGTCCGTGTTTCACT-TTTTCCTTTATATTTGC-TT--ATGGTGA
       510       520       530       540       550       560       570       580       590       600


CAATTTATA--GTGTATAGATTGTCATC
****  ****   *  ****  ****  **  *
CAATATATACAATATATATATTGGCACC
       610       620
```

Fig. 20a

VP4

```
OBERE   SEQUENZ:   HRV89 PROTEIN                    VON     1 BIS     69
UNTERE SEQUENZ:   HRV2 PROTEIN                      VON     1 BIS     69
HOMOLOGIE:      94%
```

```
MGAQVSRQNVGTHSTQNSVSNGSSLNYFNINYFKDAASSGASRLDFSQDPSKFTDPVKDVLEKGIPTLQ
************************************** *** * * **********************
MGAQVSRQNVGTHSTQNSVSNGSSLNYFNINYFKDAASNGASKLEFTQDPSKFTDPVKDVLEKGIPTLQ
          10        20        30        40        50        60
```

VP2

```
OBERE   SEQUENZ:   HRV89 PROTEIN                    VON     70 BIS    336
UNTERE SEQUENZ:   HRV2 PROTEIN                      VON     70 BIS    330
HOMOLOGIE:      73%
```

```
SPTVEACGIESDRLIQITRGDSTITSQDTANAVVAYGVWPSYLTPDDATAIDKPTQPDTSSNRFYTLDSRSWTSASSGWWWKLPDALKNMGIFGENMFYHF
************ ************** *** ******* **   ** ***** ************ *   * * * ************ ***********
SPTVEACGIESDRIIQITRGDSTITSQDVANAIVAYGVWPHYLSSKDASAIDKPSQPDTSSNRFYTLRSVTWSSSSKGWWWKLPDALKDMGIFGENMFYHY
          10        20        30        40        50        60        70        80        90        100
```

```
LGRSGIETIHVQCNSSKFHQGLLIVAAIPEHQLASATSGNVSVGYNHTHPGEQGREV-VPSRTSSDNKRPSDDSWLNFDGTLLGNLPIYPHQYINLRTNNS
************** ****** **** ***** ***   *** **** ***** ****    *   *   *   ********** * *** **** ***
LGRSGIETIHVQCNASKFHQGTLIVALIPEHQIASALHGNVNVGYNYTHPGETGREVKAETRLNPD-LQPTEEYWLNFDGTLLGNITIFPHQFINLRSNNS
         110       120       130       140       150       160       170       180       190       200
```

```
ATLILPYVNAVPMDSMLRHNNWSLVIIPICPLQVQPGGTQSIPITVSISPMFSEFSGPRSKVVFSTTQ
** * ***********  ***************       **** ***** **** * *
ATIIAPYVNAVPMDSMRSHNNWSLVIIPICPL-ETSSAINTIPITISISPMCAEFSGARAKRQ
         210       220       230       240       250       260
```

0 261 403

Fig. 20b


## P3A

```
OBERE   SEQUENZ:  HRV2 PROTEIN                    VON  1410 BIS  1486
UNTERE SEQUENZ:  HRV89 PROTEIN                    VON  1425 BIS  1500
HOMOLOGIE:     65%
```

```
GPIDMKNPPPPAITDLLQSVRTPEVIKYCEGNRWIIPAECKIEKELNLAN-TIITIIANVIGMARIIYVIYKLFCTLQ
*  **    ******  ***  **  ***  ****   *  **  ****  **   *   **  **   *****      **  *********
G-IDLQSPPPPAIADLLRSVKTPEIIKYCQDNNWIVPAECSIERDLGIANMTI-GIIANVVSIVGVIYIIYKLFCTLQ
         10        20        30        40        50        60        70
```


## P3B  VPg

```
OBERE   SEQUENZ:  HRV89 PROTEIN                   VON  1501 BIS  1521
UNTERE SEQUENZ:  HRV2 PROTEIN                     VON  1487 BIS  1507
HOMOLOGIE:     85%
```

```
GPYSGEPKPKSRAPERRVVTQ
*********   *******
GPYSGEPKPKTKIPERRVVTQ
         10        20
```

0 261 403

Fig. 20c

P3C  Protease

OBERE  SEQUENZ:  HRV89 PROTEIN                    VON   1522 BIS   1704
UNTERE SEQUENZ:  HRV2 PROTEIN                     VON   1508 BIS   1690
HOMOLOGIE:      74%

```
GPEEEFGRSLLKHNCCVVTTDKGKFTGLGIYDQVMVLPTHSDPGSEILVDGVKVKVSDSYDLHNHEGVKLEITVVKLIRNEKFKDIRKYLPSREDDYPAC
******** ** *** ** **    ******* **    * *** *** ** ***    ** *****    * ****** ** ***** *** * *   ***** *
GPEEEFGMSLIKHNSCVITTENGKFTGLGVYDRFVVVPTHADPGKEIQVDGITTKVIDSYDLYSKNGIKLEITVLKLDRNEKFRDIRRYIPNNEDDYPNC
        10        20        30        40        50        60        70        80        90       100
```

```
NLALLANQDEPTIISVGDAVSYGNILLSGTNTARMIKYHYPTKAGIECGGVLYKVGSILGIHVGGNGRDGFSAMLLKSYFGETQ
*******  *****  ***  **********    ****  **  ****  ********  *    *****************  ***     *
NLALLANQPEPTIINVGDVVSYGNILLSGNQTARMLKYSYPTKSGIECGGVLYKIGQVLGIHVGGNGRDGFSAMLLRSYFTDVQ
       110       120       130       140       150       160       170       180
```

Fig. 20d

P3D Polymerase

```
OBERE  SEQUENZ:  HRV89 PROTEIN              VON  1705 BIS  2164
UNTERE SEQUENZ:  HRV2 PROTEIN              VON  1691 BIS  2150
HOMOLOGIE:    72%


GLITKELPVSVKNLPSVHVSSKTRLQPSVFHDVFPGTKEPAVLSSNDPRLETDFDSALFSKYKGNPACQVTPHMKIAVAHYAAQLSTLDINPQPLSLEES
X XX     X  XXXX X   XX XXXXXX XXXXX XXXXXXX X XX   XX  XXXXXXXXX  X     X  XX  XXXXX XXXX X X   XX X
GQITLSKKTSECNLPSIHTPCKTKLQPSVFYDVFPGSKEPAVLSEKDARLQVDFNEALFSKYKGNTDCSINDHIRIASSHYAAQLITLDIDPKPITLEDS
      10        20        30        40        50        60        70        80        90       100


VFGIEGLEALDLNTSAGFPYVSLGIKKKDLIDKKTKDITKLRKAIDEYGIDLPMVTFLKDELRKKEK-IKDGKTRVIEANSVNDTVLFRSVFGNLFSAFH
XXX XXXXXXXXXXXXXX   X XX XXX  XXXXX XX  XXX XX XXXXXXXXXXXXXX XX XX XXXXXXXX XXXXX XXX  XXXXXX XX
VFGTDGLEALDLNTSAGFPYIAMGVKKRDLINNKTKDISKLKEAIDKYGVDLPMVTFLKDELRKHEKVIK-GKTRVIEASSVNDTLLFRTTFGNLFSKFH
     110       120       130       140       150       160       170       180       190       200


KNPGIVTGSAVGCDPEVFWSTIPLMLDGECLMAFDYSNYDGSLHPVWFKCLSMLLEDIGFSSQLINQICNSKHIYKSKYYEVEGGMPSGCAGTSIFNTII
 XXXXXXXXXXXXXXXXXX XX XXX  X XXXXX XXXXX XX XX  X   X X  X   XX   X XXXX X  XXXXXXX XXXX XXXXXXX X
LNPGIVTGSAVGCDPEVFWSKIPAMLDDKCIMAFDYTNYDGSIHPIWFEALKQVLVDLSFNPTLIDRLCKSKHIFKNTYYEVEGGVPSGCSGTSIFNTMI
     210       220       230       240       250       260       270       280       290       300


NNIIIRTLVLDAYKNIDLDKLKILAYGDDVIFSYNFKLDMAVLAKEGEKYGLTITPADKSDVFQELTYKNVTFLKRGFRADERHSFLIHPTFPVAEIHDS
XXXXXXXXXXXXXXXXXXXXX XXXXXXXXX   XXX   X XX XXXXXXXXXXXX X  X X XXXXXXXXX  XX   XXXXXXXX XX  X
NNIIIRTLVLDAYKNIDLDKLKIIAYGDDVIFSYIHELDMEAIAIEGVKYGLTITPADKSNTFVKLDYSNVTFLKRGFKQDEKYNFLIHPTFPEDEIFES
     310       320       330       340       350       360       370       380       390       400


IRWTKNPSCMQEHVLSLCHLMWHNGRHAYQEFIKGIRSVSAGRALYIPAYEVLEHEWYEKF
XXXX XX X XXXXXXXXXXXXX XX  X    XXXXXXXXXXXXX X  X XXXXXX .
IRWTKKPSQMHEHVLSLCHLMWHNGRDAYKKFVEKIRSVSAGRALYIPPYDLLLHEWYEKI
     410       420       430       440       450       460
```

0 261 403

Fig. 20e

## P2A

```
OBERE   SEQUENZ:  HRV89 PROTEIN                VON    867 BIS  1008
UNTERE SEQUENZ:  HRV2 PROTEIN                  VON    851 BIS   992
HOMOLOGIE:    84%
```

```
GPSSMFVHVGNLIYRNLHLFNSDLDDSILVSYSSDLIIYRTNTEGNDVIPNCDCTECTYYCHHKDRYFPIRVTAHDWYEIQESEYYPKHIQYNLLIGEGP
*** * **************    *****************  *  *  **  ****    ****  **  *****  **  **************************
GPSDMYVHVGNLIYRNLHLFNSEMHESILVSYSSDLIIYRTNTVGDDYIPSCDCTQATYYCKHKNRYFPITVTSHDWYEIQESEYYPKHIQYNLLIGEGP
          10         20         30         40         50         60         70         80         90        100
```

```
CEPGDCGGKLLCKHGVIGMITAGGEGHVAFIDLRKFQCAEEQ
*****************  ****   *******  * *****
CEPGDCGGKLLCKHGVIGIVTAGGDNHVAFIDLRHFHCAEEQ
        110        120        130        140
```

## P2B

```
OBERE   SEQUENZ:  HRV89 PROTEIN                VON   1009 BIS  1103
UNTERE SEQUENZ:  HRV2 PROTEIN                  VON    993 BIS  1087
HOMOLOGIE:    64%
```

```
GLSDYVEHLGQVFGVGFVDSIKQQVNFINPTSKIGSKVI-KWLLRIVSAMIIMVRNSSDPQTVIATLTLLGCSGSPWRFLKEKLCAWLQLSYVHKQ
*  **    **   **  *****  *        ***     *  **  *  ** ***  ***  *    ********    *****  **************  *  *  **  *  **
GVTDYIHMLGEAFGNGFVDSVKEHIHAINPVGNI-SKKIIKWMLRIISAMVIIRNSSDPQTILATLTLIGCSGSPWRFLKEKFCKWTQLNYIHKE
          10         20         30         40         50         60         70         80         90
```

0 261 403

Fig. 20f

P2C

OBERE SEQUENZ: HRV2 PROTEIN          VON  1088 BIS  1409
UNTERE SEQUENZ: HRV89 PROTEIN        VON  1104 BIS  1424
HOMOLOGIE:    72%

```
SDSWLKKFTEACNAARGLEWIGNKISKFIEWMKSHLPQAQLKVKYLNELKKLNLYEKQVESLRVADMKTQEKIKMEIDTLHDLSRKFLPLYASEAKRIKT
**************************  ******  *  **********    **    **  ***  ***  *    *  *        ****  **  ******  **********  ***
SDSWLKKFTEACNAARGLEWIGQKISKFIDWIKSHLPQAQLKIDYLTKLKQLNLLEKQIETIRLAPASVQEKIFIEINTLHDLSLKFLPLYASEARRIKN
    10        20        30        40        50        60        70        80        90        100


LYIKCDNIIKQKKRCEPVAIVIHGPPGAGKSITTNFLAKMITNDSDIYSLPPDPKYFDGIEDQQSVVIMDDIMQNPAGDDMTLFCQMVSSVTFIPPMADLP
*****  *  **    **  ****    ***  **  ***    *    **  *  *            *****************************************  *  ***********  **********
LYIKCSNVIKGGKRNEPVAVLIHGSPGTGKSLATSVLARMLTVETDIYSLPPDPKYFDGIEDQQSVVIMDDIMQNPSGEDMTLFCQMVSSVPFIPPMADLP
    110        120        130        140        150        160        170        180        190        200


DKGKAFDSRFVLCSTNHSLLTPPTITSLPAMNRRFFLDLDIIVHDNFKDPQGKLNVAAAFRPCDVDNRIGNARCCPFVCGKAVSFKDRNSCNKYSLAQVY
****  *  *  ***  ****  ******    *****  ***    ****  *            ***  *  ******      ****  ****  *****  *******    **  *  *
DKGKPFTSKFVLASTNHTLLTPPTVSSLPAMARRFYFDLDIQV-KKEYLLDGKLDIAKSFRPCDVNIKIGNAKCCPFICGKAVEFKDRNSCTTLSLSQLY
    210        220        230        240        250        260        270        280        290        300


NIMIEEDRRRRQVVDVMTAIFQ
    ******        *  ****
SHIKEEDRRRSSAAQAMEAIFQ
        310        320
```

Fig. 20h

<u>VP1</u>

```
OBERE   SEQUENZ:   HRV89 PROTEIN                    VON    575 BIS·  866
UNTERE SEQUENZ:   HRV2 PROTEIN                      VON    568 BIS   850
HOMOLOGIE:     63%
```

```
NPVENYIDSVLNEVLVVPNIQPSTSVSSHAAPALQAAETGHTSSVQPEDMIETRYVITDQTRDETSIESFLGRSGCIAMIEFNTSSDKTEHDKIGKGFKT
******** **********  *    *  ********************* ****** * ***** * **********    *         *         *
NPVENYIDEVLNEVLVVPNINSSNPTTSNSAPALDAAETGHTSSVQPEDVIETRYVQTSQTRDEMSLESFLGRSGCI---HE--SKLEVTLANYNKENFTV
        10        20        30        40        50        60        70        80        90       100

HKVSLQEMAQIRRKYELFTYTRFDSEITIVTAAAAQGNDSGHIVLQFMYVPPGAPVPEKRDDYTWQSGTNASVFWQEGQPYPRFTIPFMSIASAYYMFYD
*  ********* *********** *    *   * *** * **********  **** *********** ** ****  ** * **********
HAINLQEMAQIRRKFELFTYTRFDSEITLVPCISALSQDIGHITMQYMYVPPGAPVPNSRDDYAWQSGTNASVFWQHGQAYPRFSLPFLSVASAYYMFYD
       110       120       130       140       150       160       170       180       190       200

GIEDGDSAASKYGSVVTNDMGTICVRIVTSNQKHDSNIVCRIYHKAKHIKAWCPRPPRAVAYQHTHSTN-YIPSNGEATTQIKTRPDVFTVTNV
***         **    ** **  * ****      *     *  **  ********** *********  *  * ** *      *  * *** *
GIED--EQDQNYGTANTNNMGSLCSRIVTEKHIHKVHIMTRIYHKAKHVKAWCPRPPRALEYTRAHRTNFKIEDRSIQT-AIVTRPIITTA
       210       220       230       240       250       260       270       280       290
```

0 261 403

Fig. 20g

VP3

OBERE  SEQUENZ:  HRV89 PROTEIN          VON    337 BIS    574
UNTERE SEQUENZ:  HRV2 PROTEIN           VON    331 BIS    567
HOMOLOGIE:    65%

```
GLPVMLTPGSGQFLTTDDTQSPSAFPYFHPTKEIFIPGQVRNLIEMCQVDTLIPVNNTQENVRSVNMYTVDLRTQVDLAKEVFSIPVDIASQPLATTLIG
****   ************ *** * *  ****** *** * ** * **** * * ***    * *** * *           *** * **********
GLPVFITPGSGQFLTTDDFQSPCALPWYHPTKEISIPGEVKNLVEICQVDSLVPINNTDTYINSENMYSVVLQSSINAPDKIFSIRTDVASQPLATTLIG
       10        20        30        40        50        60        70        80        90       100

ELASYYTHWTGSLRFSFMFCGSASSTLKLLIAYTPPGVGKPKSRREAMLGTHLVWDVGLQSTASLVVPWVSASHFRFTTPDTYSSAGIEITCWYQTNFVVP
*  ** ************** *  * *** ******   *  *     ****** ******** * **** **** * * *   *  *********  * *
EISSYFTHWTGSLRFSFMFCGTANTTVKLLLAYTPPGIAEPTTRKDAMLGTHVIWDVGLQSTISMVVPWISASHYRNTSP-GRSTSGIEITCWYQTRLVIP
      110       120       130       140       150       160       170     -  180       190       200

DSTPDNAKMVCMVSACKDFCLRLARDTNLHTQEGVLTQ
 ** *   * ** ******* ******* * *    *
PQTPPTARLLCFVSGCKDFCLRMARDTNLHLQSGAIAQ
      210       220       230
```

0 261 403